(19) **Europäisches Patentamt European Patent Office Office européen des brevets**

(11) **EP 1 929 998 A2**

(12) **EUROPEAN PATENT APPLICATION**

(43) Date of publication:
**11.06.2008 Bulletin 2008/24**

(51) Int Cl.:
*A61K 9/16* (2006.01)    *A61K 9/22* (2006.01)
*A61K 9/28* (2006.01)    *A61P 9/04* (2006.01)
*A61K 31/403* (2006.01)

(21) Application number: **07024778.8**

(22) Date of filing: **23.09.2002**

(84) Designated Contracting States:
**AT BE BG CH CY CZ DE DK EE ES FI FR GB GR IE IT LI LU MC NL PT SE SK TR**

(30) Priority: **21.09.2001 DK 200101375**
**31.10.2001 DK 200101611**
**03.07.2002 DK 200201044**

(62) Document number(s) of the earlier application(s) in accordance with Art. 76 EPC:
**02776907.4 / 1 429 734**

(71) Applicant: **Egalet A/S**
**3500 Vaerlose (DK)**

(72) Inventors:
• **Fischer, Gina**
**3500 Væløse (DK)**

• **Slot, Lillian**
**2830 Virum (DK)**
• **Bar-Shalom, Daniel**
**2980 Kokkedal (DK)**
• **Andersen, Christine**
**2950 Vedbæk (DK)**
• **Lademann, Anne-Marie**
**2920 Charlottenlund (DK)**

(74) Representative: **Johansen, Marianne**
**Albihns A/S**
**Havneholmen 29**
**Building 2, 3. floor**
**1561 Copenhagen V (DK)**

Remarks:
This application was filed on 20-12-2007 as a divisional application to the application mentioned under INID code 62.

(54) **Controlled release solid dispersions of carvedilol**

(57)    A controlled release pharmaceutical composition for oral use comprising a solid dispersion of
i) carvedilol, which at least partially is in an amorphous form,
ii) a pharmaceutically acceptable polymer that has plasticizing properties and which has a melting point or melting interval of a temperature of at the most 200 °C,
and the composition being designed to release carvedilol with a substantially zero order release,

The polymer is typically a polyethylene glycol and/or a polyethylene oxide having a molecular weight of at least about 20,000 in crystalline and/or amorphous form or a mixture such polymers, and the active substance is typically carvedilol.

**EP 1 929 998 A2**

## Description

### Field of the invention

The present invention relates to a novel pharmaceutical composition for controlled release of a carvedilol into an aqueous medium. The pharmaceutical composition is a coated matrix composition in which the matrix composition comprises a solid dispersion of i) at least one therapeutically, prophylactically and/or diagnostically active substance, which at least partially is in an amorphous form, ii) a pharmaceutically acceptable polymer that has plasticizing properties and which has a melting point or melting interval of a temperature of at the most 200 °C, and iii) optionally, a stabilizing agent, the at least one therapeutically, prophylactically and/or diagnostically active substance having a water solubility of at the most 3 mg/ml at 25 °C such as, e.g. at the most about 2 mg/ml, at the most about 1 mg/ml, and the composition being designed to release the active substance with a substantially zero order release. The composition is provided with a coating. The coating remains intact during the release phase and may thereafter crumble and/or erode. Furthermore, the coating covers the matrix composition in such a manner that only a specific surface area of the matrix composition is subject to erosion in an aqueous medium, i.e. the surface area from which the active substance is released is kept substantial constant during the time period.

The polymer is typically a polyethylene glycol and/or a polyethylene oxide having a molecular weight of at least about 20,000 in crystalline and/or amorphous form or a mixture such of polymers.

The design of the pharmaceutical composition is based on the finding that it is possible to control the release from such a composition by ensuring that the release predominantly takes place by erosion. Furthermore, the design is based on the finding that it is possible to obtain a composition comprising the active substance in a solid dispersion comprising the active substance at least partially in an amorphous form.

### Background of the invention

Many crystalline, therapeutically active substances have a very slight solubility in aqueous medium such as, e.g., body fluids. It is well known that changing a crystalline compound into its amorphous state will substantially increase the aqueous solubility of the compound. The amorphous state of an active substance may be obtained by melting the active substance, holding it in the molten state for a certain period of time and then cooling it to an amorphous solid. Such a method is limited to particular active substances that can produce stable amorphous solids and which are not degraded by the heating step.

Carvedilol is an active substance, which has very low water solubility. At pH values in the pharmaceutically relevant range of 1 to 8 the solubility of carvedilol in aqueous media is from about 0.01 mg/ml to about 1 mg/ml. The solubility depends on the pH value as carvedilol is a weak base, i.e. the solubility is larger in an acid medium than in a basic medium. Furthermore, carvedilol is subject to degradation under formation of various generally unwanted degradation products. Carvedilol is normally employed in pharmaceutical composition in the form of a racemic mixture. It is known that both the R(+) carvedilol and the S(-) carvedilol have a therapeutic effect (cf. below).

Carvedilol may exist in at least two different crystalline forms, normally denoted from I and form II. Form II has a melting point of about 114-115 °C, whereas form I has a melting point of about 123-124 °C (EP-A-0 893 440). Form I is described to be thermodynamically stable.

Thus, carvedilol is a substance with solubility and stability problems and, furthermore, such problems normally indicate that the bioavailability is low.

Accordingly, there is a need for novel compositions comprising carvedilol or other active substances having a low water solubility in which the solubility, stability and/or bioavailability is improved.

### Disclosure of the invention

In accordance with the present invention, compositions of a sparingly soluble, crystalline active substance, e.g. carvedilol, is converted to and stabilized in its amorphous form as a solid dispersion. The amorphous state and/or the solid dispersion is stabilized by a stabilizing agent contained in the composition, providing a suitable shelf life of the improved composition. The stabilized composition also provides an increased solubility of the active substance. The stabilizing agent prevents, inhibits or delays recrystallisation of the less soluble, crystalline form of the active substance.

The invention provides a carvedilol containing pharmaceutical composition for oral use, which provides zero order release, and which at least partially contains carvedilol in amorphous and/or crystalline form.

The invention also provides various combinations of immediate release carvedilol compositions and controlled release carvedilol compositions.

Carvedilol has emerged as one of the important and promising drug substances for cardiovascular diseases, especially due to the noticeable improvement of survival rates in patients with chronic cardiac insufficiency. To optimise the treatment the present inventors have developed a new controlled release composition of carvedilol.

Carvedilol is a therapeutically active compound, which is known to be useful in the treatment of mild to moderate hypertension. Carvedilol is a nonselective beta-adrenoreceptor antagonist and an alpha$_1$-adrenoreceptor antagonist and a vasodilator. It has no intrinsic sympathomimetic activity. The vasodilatory actions of carvedilol result primarily from alfa1-adrenoceptor blockade, whereas the alfa/beta-adrenoceptor blocking activity of carvedilol prevents reflex tachycardia when used in the treatment of hypertension. The multiple actions of carvedilol are responsible for the antihypertensive efficacy of the drug. It also has significant antioxidant properties. As a consequence of its antioxidant action in attenuating oxygen free radical initiated lipid peroxidation, it is also suggested useful in organ protection, in particular, cardioprotection. Carvedilol inhibits the generation of oxygen free radicals and prevents low-density lipoprotein (LDL) oxidation, which, in turn, reduces the uptake of LDL into the coronary vasculature. This antioxidant activity may contribute to carvedilol's cardioprotective effects. In fact, compared with captopril, carvedilol has demonstrated similarly favourable effects on the lipid profiles of hypertensive patients with dyslipidemia.

Like many other classes of medications, beta-blockers can be divided into three distinct groups. The first group consists of nonselective beta-blockers without auxiliary properties and includes such drugs as propranolol (Inderal) and timolol maleate (Blocadren).

The second group consists of selective blockers of beta receptor subtypes without ancillary properties. This group includes metoprolol (Lopressor) and atenolol (Tenormin). The third group consists of nonselective beta-blockers that have the ancillary property of vasodilation. Included in this group are labetalol (Normodyne), carvedilol and bucindolol. Bucindolol and carvedilol produce less "inverse agonism" than most other beta-blockers. Inverse agonism is the ability of a beta-blocker to inactivate active state receptors. The beta-blockers with the most inverse agonism, like propranolol, produce the greatest negative chronotropic and inotropic effects. Thus, bucindolol and carvedilol produce relatively fewer negative chronotropic and inotropic effects when compared with beta-blockers like propranolol.

The beta-blocking actions of carvedilol are generally evident in humans within one hour of administration, and the alpha-mediated vasodilatory effects, manifested by decreased peripheral resistance and decreased blood pressure, are evident within about 30 minutes of administration of an IR formulation. Carvedilol is rapidly absorbed following oral administration, achieving peak plasma concentrations within one to two hours. The apparent mean terminal elimination half-life of carvedilol generally ranges from seven to 10 hours. Carvedilol is metabolised by the liver and undergoes extensive first-pass metabolism. Three active metabolites of carvedilol have been identified, but none of these compounds appears to contribute to carvedilol's beta-blocking activity. Carvedilol is primarily metabolised by the liver, with less than 2 percent of a given dose excreted unchanged in the urine. Plasma concentrations of carvedilol are nevertheless increased in patients with renal failure. Carvedilol is highly bound to plasma.

Carvedilol is effective in the treatment of congestive heart failure. Carvedilol is registered for the following indications: hypertension, chronic cardiac insufficiency and angina pectoris. Carvedilol is currently marketed as an immediate release formulation only in 3.125 mg, 6.25 mg, 12.5 mg, 25 mg and 50 mg tablets.

There is a clinical rationale for long-term treatment of hypertension with carvedilol and, accordingly, it would be beneficial to provide a controlled release composition which enable a dosage frequency of at the most 4 times daily such as, e.g. 3 times daily, 2 times daily or 1 time daily. Furthermore, a controlled composition offers a reduced standard deviation of the concentration of carvedilol in the plasma after administration and thus, gives rise to a more predictable concentration in plasma. Furthermore, a dose regimen with a lower frequency of administration will potentially improve patient compliance.

In the compositions marketed today, carvedilol is present as a racemate having the formula (RS)-1-(9H-carbazol-4-yloxy)-3-[2-(2-methoxyphenoxy)-ethylaminopropan-2-ol. However, it is contemplated that one of the enantiomers is more potent than the other. Furthermore, the metabolite, desmethylcarvedilol, is contemplated to contribute to the therapeutic effect. Carvedilol is a weak base, i.e. it may form salts with organic as well as inorganic acids.

The biotransformation of carvedilol *in vivo* is complex and many metabolites have been identified. Carvedilol exhibits stereoselective first-pass metabolism. In addition, the S(-) carvedilol is metabolized faster than the R(+)-carvedilol and four major metabolites have been identified including 4'-hydroxy carvedilol (40HC), 5'-hydroxy carvedilol (50HC), 8-hydroxy carvedilol (80HC), and O-desmethyl carvedilol (ODMC/M2). The enatiomers exhibit similar alfa-1-blocking activity, but only the S(-) isomer posseses beta-blocking activity.

With respect to the effect of carvedilol, the carvedilol reduces the cardiac workload and improves the ventricular function. In contrast to selective alfa-1- blockers, reflex tachycardia is not produced with carvedilol treatment due to is beta-blocking activity. In addtion, the vasodilation effect of the drug and resulting afterload reduction may act to attenuate a worsening of hemodynamics, which is expected from the negative inotropic effect of acute beta-blokade.

Accordingly, altering the dosage form may result in unforeseen plasma profiles of the enatiomers and metabolites of the drug, inter alia as a result of the active ingredient being absorbed form different locations of the intestines where the pH and other conditions relevant for the absorption rate and ratio of the individual enatiomers may be unpredictable.

In the present context, the term "carvedilol" encompasses carvedilol as the racemate: (RS)-1-(9H-carbazol-4-yloxy)-3-[2-(2-methoxyphenoxy)-ethylaminopropan-2-ol as well as the two individual enantiomers: (S)-1-(9H-carbazol-4-yloxy)-3-[2-(2-methoxyphenoxy)-ethylaminopropan-2-ol and (S)-1-(9H-carbazol-4-yloxy)-3-[2-(2-methoxyphenoxy)-ethylaminopropan-2-ol, metabolites of carvedilol including desmethylcarvedilol, pharmaceutically acceptable salts, complexes, solvates or anhydrate thereof, and mixtures thereof.

The term "pharmaceutically acceptable salts" of carvedilol includes alkali metal salts such as, e.g., sodium or potassium salts, alkaline earth metal salts such as, e.g., calcium and magnesium salts, and salts with organic or inorganic acid like e.g. hydrochloric acid, hydrobromic acid, nitric acid, sulfuric acid, phosphoric acid, citric acid, formic acid, maleic acid, succinic acid, tartaric acid, methansulphonic acid, toluenesulphonic acid etc.

The term "solvates" includes hydrates or solvates wherein other solvates than water are involved such as, e.g., organic solvents like chloroform and the like.

Furthermore, carvedilol (or other active substances) may be in any of its crystalline, polymorphous or amorphous forms.

A pharmaceutical composition according to the present invention is a controlled release pharmaceutical composition for oral use comprising a solid dispersion of

i) at least one therapeutically, prophylactic ally and/or diagnostically active substance, which at least partially is in an amorphous form,
ii) a polyethylene glycol and/or a polyethylene oxide having a molecular weight of at least about 20,000 in crystalline and/or amorphous form or a mixture such polymers, and
iii) optionally, a stabilizing agent,

the at least one therapeutically, prophylactically and/or diagnostically active substance having a water solubility of at the most 3 mg/ml at 25 °C such as, e.g. at the most about 2 mg/ml, at the most about 1 mg/ml, and the composition being designed to release the active substance with a substantially zero order release.

In a preferred embodiment, the pharmaceutical composition according to the invention is coated with a coating having at least one opening exposing at the one surface of said matrix. The coating comprises

i) a first cellulose derivative which has thermoplastic properties and which is substantially insoluble in the aqueous medium in which the composition is to be used,
and at least one of
ii) a second cellulose derivative which is soluble or dispersible in water,
iii) a plasticizer, and
iv) a filler.

A pharmaceutical composition according to the invention is designed for controlled release of the active substance into an aqueous medium by erosion of at least one surface of the composition.

As mentioned above, a pharmaceutical composition according to the invention is especially suitable for the delivery of active substances, which normally are crystalline and have poor water solubility. In the following, such active substances are exemplified by carvedilol.

In a preferred embodiment of the invention, the active substance in the composition is selected from the group consisting of the racemate: (RS)-1-(9H-carbazol-4-yloxy)-3-[2-(2-methoxyphenoxy)-ethylaminopropan-2-ol, the two individual enantiomers: (S)-1-(9H-carbazol-4-yloxy)-3-[2-(2-methoxyphenoxy)-ethylaminopropan-2-ol and (S)-1-(9H-carbazol-4-yloxy)-3-[2-(2-methoxyphenoxy)-ethylaminopropan-2-ol, metabolites of carvedilol including desmethylcarvedilol, pharmaceutically acceptable salts, complexes, solvates and anhydrate thereof, and mixtures thereof.

The active substance is present in any of its crystalline, polymorphous or amorphous forms or mixtures thereof.

In the pharmaceutical technology (and in the present context), the term "solid dispersion" also embraces semi-solid dispersions. By the term is understood the finely dispersed distribution of one or more solids, e.g. an active substance like carvedilol, in an inert solid or semi-solid carrier. The active substance may be present in molecular dispersed form, i.e. as a solid solution, in fine crystalline dispersed form, in a glassy amorphous phase or dispersed as a fine amorphous powder. Eutectic mixtures, i.e. crystalline structures of active substances and carriers are also encompassed in the definition of "solid dispersions". Normally, the mean particle size is used to classify dispersed system. A colloidal dispersion is when the dispersed phase has a particle size between about 1 and about 1000 nm and a coarsely dispersion has a mean particle size of at least about 1000 nm and a molecular dispersion has a particle size below about 1 nm. Combinations between the various states are very likely and the most dominating character can be determined by X-ray diffraction spectra or differential thermoanalysis.

In a pharmaceutical composition according to the invention some of the active substance may be present in a molecular dispersion such as, e.g., in the form of a solid or semi-solid solution.

Typically, however, a pharmaceutical composition according to the invention contains the active substance on amorphous form in a colloidal dispersion.

Crystals or crystalline forms of the active substance may at the most partially be present in a composition of the invention. By storage of the composition it is contemplated that some crystallisation may occur - which is acceptable as long as it has no or only minor influence of the pharmaceutical properties of the composition (dissolution data and bioavailability of the composition).

In a preferred aspect of the invention, a composition comprises carvedilol that at least partially is present in amorphous form with a mean particle size of at least about 0.01 $\mu$m such as, e.g., from about 0.01 $\mu$m to about 500 $\mu$m, from about 0.05 $\mu$m to about 500 $\mu$m, from about 0.1 $\mu$m to about 500 $\mu$m, from about 0.5 $\mu$m to about 500 $\mu$m, about 1 $\mu$m to about 500 $\mu$m, typically from about 0.5 $\mu$m to about 300 $\mu$m, more typically from about 1 $\mu$m to about 200 $\mu$m, especially from about 1 $\mu$m to about 100 $\mu$m.

**Stability**

In the present context, the terms "stability" and "stabilizing agent" are employed to encompass one or more of the following:

Stability with respect to the final composition:

i) stability with respect to the physical stability of the composition (appearance, color, strength, etc
ii) stability with respect to *in vitro* dissolution behaviour of the active substance from the composition

Stability of the individual components:

iii) stability with respect to the chemical stability of the active substance (degradation of the active substance to other - normally - unwanted products)
iv) stability with respect to the form the active substance has in the composition; normally, the active substance is dissolved (molecularly dispersed) in the polymer as a solid dispersion. In such cases precipitation or otherwise formation of crystals of the active substance in the composition is an indication of a stability problem.
v) physical and chemical stability of the pharmaceutically acceptable polymer employed as component ii).

Normally, stability is considered under specific storage and test conditions. In the present context, a stable composition is a composition that does not change (with respect to a specific property) more than 20% within a time period of at least 2 weeks (when physical parameters are considered) or a period of at least 3 months (when chemical parameters are considers). Specific conditions appear from the patent claims herein.

An important feature of the invention is that the active substance is converted to and stabilized in its amorphous form as a solid dispersion. The amorphous state and/or the solid dispersion is stabilized either by a very careful choice of the concentration of the active substance in the composition and/or by addition of suitable stabilizing agents acting by stabilizing one or more of the conditions mentioned above under items i) to v).

**Stabilizing agent**

A pharmaceutical composition according to the invention contains a stabilizing agent. The stabilizing agent may serve more than one purpose, it may stabilize the amorphous state of the active substance in the composition in order to avoid, reduce or delay any recrystallization, it may stabilize the active substance or other ingredients towards proteolytic or oxidative degradation or it may have an anti-plasticizing effect.

A stabilizing agent may also contribute to an improved solubility of the active substance. Without being bound to any theory it may be assumed that the stabilizing agent together with the polyethylene glycol and/or the polyethylene oxide represent the dispersion medium wherein the solubility of the active substance may be higher than in the polyethylene glycol and/or polyethylene oxide. The same may apply with respect to the stability of the amorphous form of the active substance.

Accordingly, a composition according to the inventions may as a stabilizing agent contain a substance, which - together with the polyethylene glycol and/or polyethylene oxide - form a dispersion medium in which the active substance is contained.

In the following is given examples of various substances that may be employed as stabilizing agents. Although they are mentioned as having a specific function they may also have other stabilizing effects on the composition and therefore, they may be employed for other stabilizing purposes as well. An example is e.g. the use of an acidic substance that is believed to have stabilizing impact on both the stability of an amorphous state of the active substance as well as impact on the dissolution behaviour of the composition. The following classification of stabilizers should therefore not limit the use of the stabilizing substances to the specific function as it may as well serves other stabilizing functions as well.

With respect to carvedilol it present polymorphism in that several different crystalline forms exist. As the different forms of crystals may present different stability properties with respect to temperature, pressure, moisture, etc., and some forms may be metastable and the more stable forms tend to be less soluble, it is desirable to convert and preserve the crystallized bulk carvedilol into an amorphous state in the pharmaceutical composition. However, carvedilol has demonstrated to be highly difficult to formulate in a controlled delivery pharmaceutical composition, which releases carvedilol during a substantial period of time in order to decrease the need for dosing to only one daily dosage, and at the same time resulting in a satisfying absorption comparable to the absorption obtained with commercially available immediate release products such as Coreg® and Dilatrend®.

The present invention demonstrates that in order to obtain a composition capable of releasing the active substance with a zero order release pattern having the same release rate in both acidic and in basic environment so as to maintain a predictable release in the patient despite possible variations in retention times in the stomach, it is important that the pharmaceutically acceptable excipients included in the compositions are carefully selected in order to avoid an unpredicted behaviour of the composition when it comes to erosion of the composition and release of the active substance.

Especially with respect to carvedilol, the requirements of having the amorphous state of carvedilol in a composition and at the same time having a composition with suitable stability also with respect to the release of carvedilol from the composition can be obtained for an increased period of time by combining one or more of the following principles for the composition.

1. Avoid surface active agents which may directly or indirectly result in increased mobility of the system, which may again increase the possibility of crystal formation. In addition, non-ionic surface active agents surpringsinly have been observed to alter the desired zero order release of the polymer matrix.

2. Adjust pH in the polymer matrix to ensure conditions for having carvedilol present in dissolved form.

3. Add buffering agents in the polymer matrix in order to reduce the risk of precipitation of carvedilol (e.g. as crystals) when the composition is subjected to neutral/basic media like the intestinal fluids.

4. Select one or more polymers having a relative high molecular weight within the range possible in order to obtain an erosion time that is within the desired range for the composition.

5. Include at least one heating step in the process for the preparation of the composition when carvedilol and the polymer are in physical contact.

6. Increase Tg for the composition in order to have an enlarged difference between Tg and storage temperature. Suitable substances are e.g. mono, di-, oligo- or polysaccharides.

From the requirements mentioned above, it is seen that e.g. selection of specific acidic substances and buffering substances serve more than one purpose (e.g. stabilises the amorphous state of carvedilol and stabilises the release rate of carvedilol from the composition). Therefore, as mentioned earlier herein the following mentioned stabilisers may serve more than one function in a composition of the invention.

An important issue mentioned above is the necessity of avoiding certain surface active substances. To this end, the inventors have surprisingly found that it is possible to obtain a matrix composition that has the desired zero order release provided that diffusion of water into the composition balances dissolution of the matrix composition. This issue is discussed in details below and in the paragraph denoted "Controlled release".

**Stabilizing agents - improving conditions for the solubility of carvedilol**

As mentioned above, an important issue is to obtain conditions in the composition that favours the dissolution of carvedilol in the composition. Hereby it is believed that the balance between the crystalline and the amorphous state of carvedilol is favoured with respect to the amorphous state, i.e. an improved stability is obtained.

To this end, the present inventors have found that incorporation of an organic or inorganic acid favours the dissolution of carvedilol in the composition.

Suitable acids may be selected from the group consisting of inorganic acids, organic acids and pharmaceutically acceptable salts or complexes thereof. Mixtures thereof are also of relevance.

The acid may also be a mono-, di-, oligo, polycarboxylic acid or amino acids such as, e.g. acetic acid, succinic acid, citric acid, tartaric acid, acrylic acid, benzoic acid, malic acid, maleic acid, sorbic acid etc., aspartic acid, glutamic acid etc.

Examples of suitable organic acids include acetic acid/ ethanoic acid, adipic acid, angelic acid, ascorbic acid/ vitamin C, carbamic acid, cinnamic acid, citramalic acid, formic acid, fumaric acid, gallic acid, gentisic acid, glutaconic acid, glutaric acid, glyceric acid, glycolic acid, glyoxylic acid, lactic acid, levulinic acid, malonic acid, mandelic acid, oxalic acid, oxamic acid, pimelic acid, and pyruvic acid.

Examples of suitable inorganic acids include pyrophosphoric, glycerophosphoric, phosphoric such as ortho and meta phosphoric, boric acid, hydrochloric acid, and sulfuric acid.

In a specific aspect of the invention the acidic substance is meta and/or ortho phosphoric acid.

**Stabilizing measures - improving the solubility of carvedilol**

In accordance with the present invention, stable compositions of a sparingly soluble, crystalline active substance, e.g. carvedilol, is obtained. In one aspect of the invention, the ingredients and constitution of a composition of the invention are carefully selected in order to ensure that the concentration of the active substance in the composition or, alternatively, in the pharmaceutically acceptable polymer that has plasticizing properties is not greater than the saturation concentration of the active substance in the polymer.

Thus, in an aspect the invention relates to a stable controlled release pharmaceutical composition for oral use comprising a solid dispersion of component i) and ii)

i) at least one therapeutically, prophylactically and/or diagnostically active substance, which at least partially is in an amorphous form,
ii) a pharmaceutically acceptable polymer that has plasticizing properties and which has a melting point or melting interval of a temperature of at the most 200°C,

and, optionally, a stabilizing agent,
wherein the at least one therapeutically, prophylactically and/or diagnostically active substance has a water solubility of at the most 3 mg/ml at 25 °C such as, e.g. at the most about 2 mg/ml, at the most about 1 mg/ml, and the concentration of the active substance in the composition corresponds to a concentration of at the most the saturated concentration in component ii) at a temperature corresponding to the melting point or the lowest end point of the melting interval of component ii) optionally together with component iii).

The present inventors have found that it is of utmost importance in order to obtain a stable composition that the active ingredient is present in the solid dispersion in a suitable concentration that makes it possible to prevent formation of any unwanted precipitates during storage under normal conditions. As already discussed herein it is especially of interest to avoid formation of crystals of the active substance.

Normally supersaturated systems (i.e. systems wherein the concentration of a given substance in a medium is larger than the solubility in the medium) are instable systems that after a certain time period will lead to precipitation of the substance in the medium. In a saturated system, which is a stable system, an equilibrium between solid and dissolved substance will take place. In systems where the active substance is present in dissolved form and the concentration of the substance is well below the solubility normally no change with respect to formation of precipitates will take place (unless the substance is degraded to insoluble products etc.). A dissolved system may therefore be regarded as a stable system. However, in practice the situation is often much more complex and it is normally necessary to stabilize even dissolved system by use of different methods.

In a specific embodiment, the pharmaceutically acceptable polymer employed as component ii) is a polyethylene oxide having a molecular weight of at least about 20,000 in crystalline and/or amorphous form or a mixture such polymers. More details on suitable polymers are disclosed herein. The solubility of a particular active substance in PEO depends *inter alia* on the quality and the molecular weight of the PEO employed. Thus, in order to determine a suitable concentration of the active substance in a composition of the invention it is necessary to determine the solubility of the active substance in the PEO (or other polymers employed) in question. The solubility is normally determined at a temperature that corresponds to the melting or softening point of the PEO in question and the solubility determined is the saturation solubility. A person skilled in the art knows how to determine the solubility of a specific substance in a specific polymer.

When the solubility of carvedilol in a polymer composition is taken into account, the present inventors have found that a concentration of at the most about 23% w/w such as, e.g., at the most about 22% w/w, at the most about 21 % w/w or at the most about 20% w/w is suitable (% w/w based on the weight of the total composition.

In a specific embodiment, the invention relates to a composition containing carvedilol as active substance and PEO 200,000 as component ii) and wherein the concentration of carvedilol in PEO 200,000 is at the most about 22% w/w, at the most about 21 % w/w or at the most about 20% w/w.

**Stabilizing agents - zero order release**

*Difussion and dissolution adjusters*

As already discussed above, it is important that a composition according to the invention releases at least most of the active substance by a zero order release mechanism. One aspect of research about controlled-release delivery systems involves designing a system, which produces steady-state plasma drug levels. The release of active substance from such systems is also referred to as zero-order drug release kinetics. To meet this objective, numerous design variations have been attempted, and their major controlling mechanisms include diffusion/dissolution.

The release rate of a dissolved or dispersed active substance from a polymeric matrix composition introduced in a specific environment, strongly depends on the nature of the diffusion and sorption processes involving the polymer/environment system and the polymer/active substance system.

The active substance release data may be analysed using Eq. 1 and Eq. 2 where $M_t/M_{oo}$ is the fractional drug release, t is the release time, k is a kinetic constant characteristics of the drug/polymer system, $C_d$ is the tracer loading concentration and n is an exponent which characterisers the mechanism of release of the tracers.

$$\frac{M_t}{M_\infty} = k \cdot t^n \qquad \text{(Eq. 1)}$$

$$\frac{dM_t}{A \cdot dt} = n \cdot C_d \cdot k \cdot t^{n-1} \qquad \text{(Eq.2)}$$

Clearly, a desirable mechanism for many applications is that which leads to n = 1. This characterizes zero-order behaviour. The table below summarizes the general dependence of n on the diffusion mechanism.

| Diffusinal release solute release Exponent (n) | Overall Solute diffusion mechanism | time dependence of rate ($dM_t/d_t$) |
| --- | --- | --- |
| 0.5 | Fickian diffusion | $t^{-0.5}$ |
| 0.5 < n < 1.0 | Anomalous (non Fickian) diffusion | $t^{n-1}$ |
| 1.0 | Case II Transport | Zero-order (time independent) release |
| n > 1.0 | Super Case II transport | $t^{n-1}$ |

In the case of PEO matrices, the solubility of the polymer can alter the characteristics of the penetrated layer, leading to different behaviours in systems presenting different dissolution features. To control the release of the active agent, there should be a balance between diffusion of the active agent and solubilization of the polymer matrix. The diffusivity of the drug through the matrix, the swelling of the polymer, and its solubilization rate may be biased by changing the molecular weight of the polymer or blending polymer fractions with different molecular weights.

In the following is given examples on suitable excipients that may be added in order to adjust the balance between diffusion and dissolution so as to obtain zero order release rate. The pharmaceutically acceptable excipients suitable for establishing the above-mentioned desired balance, are in the present context also denoted DDAs (Diffusion and Dissolution Adjusters).

Thus, the matrix composition may also comprise one or more pharmaceutically acceptable excipients (DDAs). The function of the at least one pharmaceutically acceptable excipient is to establish the desired balance between on the one hand the diffusion rate of water into the matrix composition and on the other hand the dissolution rate of the matrix composition in an aqueous medium such as, e.g., water. As explained above, a zero order release rate is obtained if that the diffusion rate of the aqueous medium into the matrix composition corresponds to about 100% ± 30% such as, e.g. about 100% ± 25%, about 100% ± 20%, about 100% ± 15% or about 100% ± 10% or about 100% of the dissolution rate of the matrix composition. By the term "zero order release" is meant that the release takes place so as to obtain a zero order release of at least about 60% w/w such as, e.g. at least about 65% w/w, at least about 70% w/w, at least about 75% w/w, at least about 80% w/w, at least about 85% w/w, at least about 90% w/w, at least about 95% w/w or at least about 97or 98% w/w of the active substance from the pharmaceutical composition when subject to an in vitro dissolution test as described herein.

In general a test for diffusion of water into the matrix composition and a test for the dissolution of the matrix composition in an aqueous medium are performed using a matrix composition having the desired shape and being prepared analogous to the matrix composition in the final composition. This means that when the final composition is prepared by e.g. injection moulding then the matrix composition to be tested with respect to diffusion and dissolution behaviour is also prepared by injection moulding.

There may be cases where it is not necessary to adjust the matrix composition by adding a pharmaceutically acceptable excipient. Such cases are e.g. when the polymer employed in itself has the desired properties with respect to diffusion of water and dissolution of polymer.

In the experimental section herein examples are given showing that it has been possible to obtain the desired zero order release when a pharmaceutically acceptable excipients has been incorporated into the matrix composition.

Without being bound by any theory it is contemplated that in those cases where a slightly or insoluble active substance is employed then it may be necessary to circumvent the effect from the active substance (with respect to diffusion and/or dissolution of the matrix composition) by adding a very soluble pharmaceutically acceptable excipient. Accordingly, it is contemplated that when the at least one therapeutically, prophylactically and/or diagnostically active substance has a solubility of at the most about 3 mg/ ml such as, e.g. at the most about 1 mg/ml, at the most about 0.1 mg/ml, at the most about 0.05 mg/ml such as, e.g. at the most about 0.001 mg/ml in water at ambient temperature then the pharmaceutically acceptable excipient, if present, typically has a solubility of at least 1 mg/ml such as, e.g. at least about 3 mg/ml, at least about 5 mg/ml, at least about 10 mg/ml, at least about 25 mg/ml or at least about 50 mg/ml in water at ambient temperature.

There may situations, however, where it also may be suitable to incorporate water-soluble substances (and/or water-insoluble substances) as DDA's irrespective of the solubility of the active substance.

In the present case where the active substance employed has a low solubility in alkaline medium, it is contemplated that an inorganic or organic acid or substance having an acidic reaction in aqueous environment is employed as a DDA.

However, other factors than the solubility in water play a role in the erosion process and therefore there may be situations where such factors dominate the solubility factor and then the above-given combinations may be of minor importance.

Suitable pharmaceutically acceptable excipients (DDAs) may be selected from the group consisting of inorganic acids, inorganic bases, inorganic salts, organic acids or bases and pharmaceutically acceptable salts thereof, saccharides, oligosaccharides, polysaccharides, and cellulose and cellulose derivatives.

Alternatively or additionally, a suitable pharmaceutically acceptable excipient is a mono-, di-, oligo, polycarboxylic acid or amino acids such as, e.g. acetic acid, succinic acid, citric acid, tartaric acid, acrylic acid, benzoic acid, malic acid, maleic acid, sorbic acid etc., aspartic acid, glutamic acid etc.

Examples of suitable organic acids include acetic acid/ ethanoic acid, adipic acid, angelic acid, ascorbic acid/ vitamin C, carbamic acid, cinnamic acid, citramalic acid, formic acid, fumaric acid, gallic acid, gentisic acid, glutaconic acid, glutaric acid, glyceric acid, glycolic acid, glyoxylic acid, lactic acid, levulinic acid, malonic acid, mandelic acid, oxalic acid, oxamic acid, pimelic acid, and pyruvic acid.

Examples of suitable inorganic acids include pyrophosphoric, glycerophosphoric, phosphoric such as ortho and meta phosphoric, boric acid, hydrochloric acid, and sulfuric acid.

Examples of suitable inorganic compounds include aluminium.

Examples of organic bases are p-nitrophenol, succinimide, benzenesulfonamide, 2-hydroxy-2cyclohexenone, imidazole, pyrrole, diethanolamine, ethyleneamine,tris (hydroxymethyl) aminomethane, hydroxylamine and derivates of amines, sodium citrate, aniline, hydrazine.

Examples of inorganic bases include aluminium oxide such as, e.g., aluminium oxide trihydrate, alumina, sodium hydroxide, potassium hydroxide, calcium carbonate, ammonium carbonate, ammnonium hydroxide, KOH and the like.

Suitable pharmaceutically acceptable salts of an organic acid is e.g. an alkali metal salt or an alkaline earth metal salt such as, e.g. sodium phosphate, sodium dihydrogenphosphate, disodium hydrogenphosphate etc., potassium phosphate, potassium dihydrogenphosphate, potassium hydrogenphosphate etc., calcium phosphate, dicalcium phosphate etc., sodium sulfate, potassium sulfate, calcium sulfate, sodium carbonate, sodium hydrogencarbonate, potassium carbonate, potassium hydrogencarbonate, calcium carbonate, magnesium carbonate etc., sodium acetate, potassium acetate, calcium acetate, sodium succinate, potassium succinate, calcium succinate, sodium citrate, potassium citrate, calcium citrate, sodium tartrate, potassium tartrate, calcium tartrate etc.

A suitable inorganic salt for use in a matrix composition of the invention is sodium chloride, potassium chloride, calcium chloride, magnesium chloride etc.

Examples of such excipients are glucose and other monosaccharides, ribose, arabinose, xylose, lyxose, allose, altrose, inosito, glucose, sorbitol, mannose, gulose, idose, galactose, talose, mannitol, fructose, lactose, sucrose, and other disaccharides, dextrin, dextran or other polysaccharides, amylose, xylan, cellulose and cellulose derivatives such as, e.g. microcrystalline cellulose, methyl cellulose, ethyl cellulose, ethylhydroxyethyl cellulose, ethylmethylcellulose, hydroxyethylcellulose, hydroxyethylmethyl cellulose, carboxymethyl cellulose, hydroxypropyl cellulose, hydroxymethylpropyl cellulose, hydroxypropylmethyl cellulose, amylopectin, pectin, starch, sodium starch etc.,kaolin, bentonit, acacia, alginic acid, sodium alginate, calcium alginate, gelatin, dextrose, molasses, extract of Irish moss, panwar gum, ghatti gum, mucilage of isapol husk, veegum, glycollate, magnesium stearate, calcium stearate, stearic acid, talc, titanium dioxide, silicium dioxide, clays, croscarmellose, gums, agar etc.

**Buffering agents**

As mentioned under item 3 above, addition of a buffering agent in the composition may reduce the risk for precipitation of carvedilol crystals in the composition when subject to e.g. intestinal fluids. Such a precipitation will result in a decrease in the release of carvedilol from the composition.

The purpose of adding a buffering substance to the composition is to keep pH in the microenvironment around the carvedilol particle at a desirable level in order to favour dissolution of carvedilol.

In general, a buffering agent should have a pKa value close to the middle of the pH range required and the amount of buffer substance incorporated in the composition should be sufficient to maintain pH at the desired level when the composition is subject to intestinal fluids. Especially, it is the pH of the diffusion layer that should be kept constant. However, in those cases where it is not possible to incorporate a sufficient amount of buffer substance in the composition a buffering agent with a pKa slightly higher than the working pH (i.e. the pH of the diffusion layer) is preferred, and vice versa if the working pH of the composition is higher than the pH of the part or parts of the intestinal tract where the dissolution takes place. Accordingly, for carvedilol, the preferred buffer is one having a pKa value in the acidic area (i.e. from about 0.5 to about 5), especially when the concentration of the buffering substance is sufficient to maintain pH of the diffusion layer irrespective of the pH in the gastrointestinal tract. However, in those cases where this is not possible, the pKa value should be in the range of from about 0.5 to about 7 such as, e.g. from 2 to 7, from about 3 to about 7, from about 4 to about 6, such as about 5 at 37° C as an acidic micro-environment will favor a dissolution of the carvedilol in the intestinal tract when the intestinal fluids diffuse into the outer exposed layer of the controlled release formulation according to the present invention.

Examples of buffers

| Buffer | $KHC_4H_4O_6$ | $KHC_8H_4O_4$ | $KH_2PO_4 + Na_2HPO_4$ | $KH_2PO_4 + Na_2HPO_4$ | $Na_2B_4O_7 \, 10 \, H_2O$ |
|---|---|---|---|---|---|
| Density (g/ml) | 1.0036 | 1.0017 | 1.0028 | 1.0020 | 0.9996 |
| pH at 25°C | 3.557 | 4.008 | 6.865 | 7.413 | 9.180 |

Calcium citrate

Tris (hydroxymethyl aminomethane)

Suitable buffers are also phosphate, citrate, salicylate, glycine, acetate, etc.

In the paragraph above denoted "Diffusion-dissolution adjusters", a number of substances are mentioned, which are suitable for use as buffering agents. Below is a table with suitable buffering agents listed together with their pKa values.

List of possible DDA components listed with increasing pKa values

| | | |
|---|---|---|
| Pyrophosphoric | 177.98 | 0.85 ($pK_{a1}$) |
| Oxalic | 95.07 | 1.19 ($pK_{a1}$) |
| Glycerophosphoric | 172.08 | 1.47 ($pK_{a1}$) |
| Ethylenediamine tetraacetic acid (EDTA) | 292.24 | 1.70 ($pK_{a1}$) |
| Histidine | 155.16 | 1.82 ($pK_{a1}$) |
| Pyrophosphoric | 177.98 | 1.96 ($pK_{a2}$) |
| Maleic | 116.07 | 2.00 ($pK_{a1}$) |
| Benzenehexacarboxylic (mellitic) | 342.17 | 2.08 ($pK_{a1}$) |
| Phosphoric | 98.0 0 | 2.12 ($pK_{a1}$) |
| Brucine tetrahydrate | 466.53 | 2.30 ($pK_{a1}$) |

(continued)

| | | |
|---|---|---|
| Benzenepentacarboxylic | 296.18 | 2.34 (pK$_{a1}$) |
| Glycine | 75.07 | 2.34 (pK$_{a1}$) |
| Benzene-1,2,4,5-tetracarboxylic (pyromellitic) | 254.15 | 2.43 (pK$_{a1}$) |
| Benzenehexacarboxylic (mellitic) | 342.17 | 2.46 (pK$_{a2}$) |
| EDTA | 292.24 | 2.60 (pK$_{a2}$) |
| Malonic | 146.02 | 2.85 (pK$_{a1}$) |
| Phthalic | 116.13 | 2.90 |
| Benzenepentacarboxylic | 298.16 | 2.95 (pK$_{a2}$) |
| Salicylic | 138.12 | 2.98 |
| Benzene-1,2,3-tricarboxylic (hemimellitic) | 246.18 | 2.98 (pKa$_1$) |
| 1,4-Piperazinebis-(ethanesulfonic acid) "PIPES" | 302.37 | 3.00 (pK$_{a3}$) |
| Tartaric | 150.09 | 3.02 (pK$_{a1}$) |
| Fumaric | 116.07 | 3.03 (pK$_{a1}$) |
| Glycylglycine | 132.12 | 3.06 |
| Citric acid | 192.12 | 3.06 (pK$_{a1}$) |
| Cyclopentanetetra-1,2,3,4-carboxylic | 246.17 | 3.07 (pK$_{a1}$) |
| o-Phthalic | 166.13 | 3.10 (pK$_{a1}$) |
| Benzene-1,2,4,5-tetracarboxylic (pyromellitic) | 254.15 | 3.13 (pK$_{a2}$) |
| Benzene-1,3,5-tricarboxylic (trimesic) | 210.14 | 3.16 (pK$_{a1}$) |
| Benzenehexacarboxylic (mellitic) | 342.17 | 3.24 (pK$_{a3}$) |
| Dimethylmalonic | 132.12 | 3.29 (pK$_{a1}$) |
| Mandelic | 152.15 | 3.36 |
| Butane-1,2,3,4-tetracarboxylic | 234.12$_1$ | 3.36 (pK$_a$) |
| Malic | 134.09 | 3.40 (pK$_{a1}$) |
| 1,1-Cyclohexanediacetic | 200.18 | 3.52 (pK$_{a1}$) |
| 2-Methylpropane-1,2,3-triscarboxylic | 190.15 | 3.53 (pK$_{a1}$) |
| Hippuric | 179.18 | 3.64 |
| Propane-1,2,3-tricarboxylic (tricarballylic) | 176.12 | 3.67 (pK$_{a1}$) |
| Formic | 46.02 | 3.75 |
| 3,3-Dimethylglutaric | 160.17 | 3.79 (pK$_{a1}$) |
| I,I-Cyclopentanediacetic | 186.21 | 3.82 (pK$_{a1}$) |
| Itaconic | 130.1 | 3.84 (pK$_{a1}$) |
| Lactic | 90.08 | 3.86 |
| Benzenepentacarboxylic | 298.16 | 3.94 (pK$_{a3}$) |
| Benzene-1,3,5-tricarboxylic (trimesic) | 210.14 | 3.98 (pK$_{a2}$) |
| Barbituric | 128.09 | 3.98 |
| Ascorbic | 176.12 | 4.10 (pK$_{a1}$) |
| 2,2-Dimethflsuccinic | 146.14 | 4.11 (pK$_{a1}$) |
| Succinic | 118.09 | 4.19 (pK$_{a1}$) |
| Benzoic | 122.12 | 4.20 |
| Oxalic | 95 07 | 4.21 (pK$_{a2}$) |
| Benzene-1,2,3-tricarboxylic (hemimellitic) | 246.18 | 4.25 (pK$_{a2}$) |
| 3,6-Endomethylene-1,2,3,6-tetrahydrophthalic acid "EMTA" (pK$_{a1}$) | 183.62 | 4.30 |
| 2,2-Dimethylglutaric | 160.17 | 4.31 (pK$_{a1}$) |
| Butane-1,2,3,4-tetracarboxylic | 234.12 | 4.38 (pK$_{a2}$) |
| Benzenehexacarboxylic (mellitic) | 342.17 | 4.44 (pK$_{a4}$) |
| Benzene-1,2,4,5-tetracarboxylic (pyromellitic) | 254.15 | 4.44 (pK$_{a3}$) |
| Fumaric | 116.07 | 4.47 (pK$_{a2}$) |
| Cyclopentanetetra-1,2,3,4-carboxylic | 246.17 | 4.48 (pK$_{a2}$) |
| Tartaric | 150.09 | 4.54 (pK$_{a2}$) |
| Citric | 210.14 | 4.74 (pK$_{a2}$) |

(continued)

| | | |
|---|---|---|
| Acetic | 60.05 | 4.76 |
| n-Butyric | 88.10 | 4.82 |
| Propane-1,2,3-tricarboxylic (tricarballylic) | 176.12 | 4.84 ($pK_{a2}$) |
| Benzene-1,3,5-tricarboxylic (trimesic) | 210.14 | 4.85 ($pK_{a3}$) |
| Propionic | 74.08 | 4.87 |
| 2-Methylpropane-1,2,3-triscarboxylic | 190.15 | 5.02 ($pK_{a2}$) |
| Malic | 134.09 | 5.05 ($pK_{a2}$) |
| Benzenepentacarboxylic | 298.16 | 5.07 ($pK_{a4}$) |
| Pyridine | 79.1 | 5.23 |
| o-Phthalic | 116.13 | 5.27 ($pK_{a2}$) |
| Citric | 192.12 | 5.40 ($pK_{a3}$) |
| Butane-1,2,3,4-tetracarboxylic | 234.12 | 5.45 ($pK_{a3}$) |
| Benzenehexacarboxylic (mellitic) | 342.17 | 5.50 ($pK_{a5}$) |
| 2,2-Dimethylglutaric | 160.17 | 5.51 ($pK_{a2}$) |
| Itaconic | 130.1 | 5.55 ($pK_{a2}$) |
| Cyclopentanetetra-1,2,3,4-carboxylic | 246.17 | 5.57 ($pK_{a3}$) |
| Succinic | 118.09 | 5.57 ($pK_{a2}$) |
| Benzene-1,2,4,5-tetracarboxylic (pyromellitic) | 254.15 | 5.61 ($pK_{a4}$) |
| Benzene-1,2,3-tricarboxylic (hemimellitic) | 246.18 | 5.87 ($pK_{a3}$) |
| Dimethylmalonic | 132.12 | 5.98 ($pK_{a2}$) |
| Histidine | 156.16 | 6.00 ($pK_{a2}$) |
| Hydroxylamine | 34.0 | 6.03 |
| Carbonic ($H_2CO_3$+$CO_2$) | 62($CO_2$) | 6.10 ($pK_{a1}$) |
| Malonic | 104.06 | 6.10 ($pK_{a2}$) |
| 2-(N-Morpholino)-ethane sulfonic acid "MES" | 195.2 | 6.15 ($pK_{a2}$) |
| Glycerophosphoric | 172.08 | 6.19 ($pK_{a2}$) |
| Propane-1,2,3-tricarboxylic (tricarballylic) | 176.12 | 6.20($pK_{a3}$) |
| Benzenepentacarboxylic | 298.16 | 6.25 ($pK_{a5}$) |
| Maleic | 116.07 | 6.26 ($pK_{a2}$) |
| 2,2-Dimethylsuccinic | 146.14 | 6.29 ($pK_{a2}$) |
| EDTA | 292.24 | 6.30 ($pK_{a3}$) |
| 3,3-Dimethylglutaric | 160.17 | 6.31 ($pK_{a2}$) |
| Bis(2-hydroxyethyl)imino-tris(hydroxymethyl) methane "BIS-TRIS" | 209.24 | 6.46 |
| Benzenehexacarboxylic (mellitic) | 342.17 | 6.59 ($pK_{a6}$) |
| N-(2-Acetamido)imino-diacetic acid "ADA" | 190.17 | 6.60 ($pK_{a3}$) |
| Butane-1,2,3,4-tetracarboxylic | 234.12 | 6.63 ($pK_{a4}$) |
| Pyrophosphoric | 177.98 | 6.68 ($pK_{a3}$) |
| 1,1-Cyclopentanediacetic (3,3 tetramethylene glutaric acid) ($pK_{a2}$) | 186.21 | 6.70 |
| 1,4-Piperazinebis-(ethanesulfonic acid) "PIPES" | 302.37 | 6.8 ($pK_{a4}$) |
| N-(2-Acetamido)-2-aminoethanesulfonic acid "ACES" | 182.20 | 6.9 ($pK_{a2}$) |
| 1,1-Cyclohexanediacetic | 200.18 | 6.94 ($pK_{a2}$) |
| 3,6-Endomethylene-1,2,3,6-tetrahydrophthalicacid "EMTA" (pK2) | 183.62 | 7.00 |
| Imidazole | 68.08 | 7.00 |
| 2-(Aminoethyl)trimethylammonium chloride "CHOLAMINE" | 156.69 | 7.10 |
| N,N-Bis(2-hydroxyethyl)-2-aminoethanesulfonic acid "BES" ($pK_{a2}$) | 213.25 | 7.15 |
| 2-Methylpropane-1,2,3-triscarboxylic | 190.15 | 7.20 ($pK_{a3}$) |
| 2-(N-Morpholino)propane-sulfonic acid "MOPS" | 209.27 | 7.20 ($pK_{a2}$) |
| Phosphoric | 98.0 | 7.21 ($pK_{a2}$) |
| N-Tris(hydroxymethyl)methyl-2-aminoethane sulfonic acid "TES" ($pK_{a2}$) | 229.28 | 7.50 |
| N-2-Hydroxyethylpiperazine-N'-2-ethanesulfonic acid "HEPES" ($pK_{a2}$) | 238.31 | 7.55 |
| 2-Hydroxyethylimino-tris(hydroxymethyl)methane "MONO-TRIS" | 165.18 | 7.83 |

(continued)

| | | |
|---|---|---|
| Brucine tetrahydrate | 466.53 | 7.95 (pK$_{a2}$) |
| 4-(2-Hydroxyethyl)- I -piperazinepropane sulfonic acid "EPPS" | 252.23 | 8.00 |
| Tris(hydroxymethyl)aminomethane"TRIS" | 121.14 | 8.10 |
| N-Tris(hydroxymethyl)methylglycine "TRICINE" | 180.18 | 8.15 |
| Glycinamide | 74.04 | 8.20 |
| N,N-Bis(2-hydroxyethyl)glycine"BICINE" | 163.18 | 8.35 |
| N-Tris(hydroxymethyl)methyl-2-aminopropane sulfonic acid "TAPS" (pK$_{a2}$) | 243.3 | 8.40 |
| N-Glycyl-glycine | 132.12 | 8.40 |
| Histidine | 155.16 | 9.17 (pK$_{a3}$) |
| Boric | 43.82 | 9.24 |
| Pyrophosphoric | 177.98 | 9.39 (pK$_{a4}$) |
| Ethanolamine | 61.08 | 9.44 |
| Glycine | 75.07 | 9.60 (pK$_{a2}$) |
| Trimethylamine | 59.11 | 9.74 |
| Cyclopentanetetra-1,2,3,4-carboxylic | 246.17 | 10.06 (pK$_{a4}$) |
| Carbonic (H$_2$CO$_3$ + CO$_2$) | 62(CO$_2$) | 10.25 (pK$_{a2}$) |
| 3-Cyclohexylamino-l-propanesulfonic acid "CAPS" | 221.32 | 10.40 (pK$_{a2}$) |
| EDTA | 292.24 | 10.60 (pK$_{a4}$) |
| Methylamine | 31.06 | 10.64 |
| Dimethylamine | 45.09 | 10.72 |
| Ethylamine | 45.09 | 10.75 |
| Triethylamine | 101.19 | 10.76 |
| Diethylamine | 73.14 | 10.98 |
| Ascorbic | 176.12 | 11.79 (pK$_{a2}$) |
| Phosphoric | 98.00 | 12.32 (pK$_{a3}$) |

**Stabilizing measures - phycical contact between the polymer and the active substance**

It should also be mentioned that a composition of the invention is especially suitable when it is desired to have an amorphous form of the active substance in the composition, because the most convenient process for the preparation of a composition of the invention involves heating of the polymer together with the active substance and the conversion from the crystalline state to the amorphous state requires addition of energy (heating).

Normally, when preparing a composition according to the invention heating is employed for an injection moulding process. During heating it has been observed that PEO in various qualities forms free radicals that results in the formation of *inter alia* formaldehyde and formic acid. These products may often lead to further degradation e.g. of the active substance present in the composition and it is therefore necessary to take the necessary precautions in this respect. Oxidative free radicals degradation by hydroperoxides can be catalysed by certain transition metal ions, especially those of copper, cobalt and manganese. Thus, employment of PEO qualities devoid of or only containing a very small amount of such transition metal ions may improve stability. Another possibility is to use component ii) in a quality that ensures that free radicals formed, if any, do not significantly increase the degradation of the active substance in the composition. Such a quality could e.g. be a quality containing an antioxidant that functions by preventing the formation of free radical during heating or by scavenging any free radicals formed. Another possibility is to add such antioxidant to the formulation before any heating takes place.

Suitable qualities include PEO 200,000 NF or LF from Dow Chemicals.

A composition according to the invention may therefore further comprise one or more antioxidants that inhibits the formation of peroxides and/or inactivates any peroxides present.

Suitable antioxidants for use includes beta-caroten (a vitamin A precursor), ascorbic acid, ascorbyl palmitate, butylated hydroxyanisole, butylated hydroxytoluene, hypophosphorous acid, monothioglycerol, potassium metabisulfite, sodium metabisulfite, propyl gallate, sodium formaldehylde sulfoxylate, sodium thiosulfate, sulfur dioxide, tocopherol, tocopherol acetate, tocopherol hemisuccinate, TPGS or other tocopherol derivatives, sulfides, phosphine etc. Other suitable antioxidants are described herein.

It is believed that the amorphous state of the carvedilol is furthermore favoured by the processing procedures of the preparation of the product according to the present invention, which in a preferred embodiment involves injection moulding of the pharmaceutical units.

The injection moulding technique have the advantage of simultaneous mixing and heating the components during increased pressure in a one step procedure without exposure to air and moisture because the injection moulding is performed in a single closed compartment from the time the blend has entered the machine to the final pharmaceutical units are ejected ready for packaging. The preferred packaging for the final pharmaceutical product according to the present invention is a light and moisture tight package.

## Stabilizing measures - other aspects

It is currently believed that a relatively low content of water in the composition is desirable in order to avoid recrystallization or crystal growth of the active substance. Accordingly, a low water content of any of the ingredients in the composition is important. Accordingly, the water content of the DDA such as, e.g., citric acid is at the most about 15% w/w such as, e.g., at the most about 12% w/w, such as e.g., at the most about 10% w/w, such as e.g., at the most about 8% w/w, such as e.g., at the most about 5% w/w, such as e.g., at the most about 3% w/w, such as e.g., at the most about 2% w/w, such as at the most about 1 % w/w or at the most about 0.5% w/w.

## Controlled release

During the last decades many different systems for modifying the release of an active drug substance from a pharmaceutical composition have been developed. Most of them aim at obtaining a zero or a first order release rate of the active substance from the composition. Zero order release rate (i.e. constant release of the active substance with time) seems to be very difficult to obtain from a pharmaceutical composition. The present invention is based on a polymeric matrix composition, which is construed to deliver the active substance in a zero order release manner. The present invention is a further development based on the Applicant's previously described drug delivery systems, see e.g. EP-B-0 406 315, EP-B-0 493 513, EP-B-0 740 310 and WO 99/51208 the disclosure of which is hereby incorporated by reference.

In particular, it has surprisingly been found that it is possible to obtain zero order release from a polymeric matrix composition without any content of a water dispersible or water soluble surface active agent or a mixture of such surface active agents which has at least one domain which is compatible with the polymer in the polymer matrix composition and at least one other domain which is substantially lipophilic and which has a melting point that is lower than the polymer used in the polymeric matrix composition. The presence of such a substance (e.g. like PEG 400 monostearate or PEG 2000 monostearate) has been contemplated to function as a so-called repair medium. Such a repair medium has a substantially hydrophilic domain, which gives it affinity to the (crystalline) polymeric phase, thereby filling in domains between grains and cracks in the polymer matrix and reducing the water affinity of these domains and in the polymer matrix itself. Water diffusion in the interface between the polymer crystals is thereby substantially eliminated, thus substantially limiting diffusion of water into the composition to the surface layer of the matrix, so that erosion of the composition is predominantly effected by the dissolving action of the aqueous phase on a surface or surfaces of the composition exposed to the aqueous medium. In other words a repair medium seems to prevent the diffusion of water in the polymer matrix composition.

However, in certain cases, the present inventors have observed that inclusion of a water soluble surface active agent has a negative impact on the mobility and/or stability of a composition.

The present inventors have found that it is possible to obtain a zero order release from a polymer matrix composition although water may be able to diffuse into the matrix. When water diffuse into the polymer matrix composition a resulting boundary layer (or swelling layer) can be formed at the surface of the matrix composition, which is exposed to the aqueous medium. In general the diffusion of an active substance through such a boundary layer is important for the release of an active substance and, accordingly, the thickness of the boundary layer is important for the release rate. However, the present inventors have found that it is possible to eliminate or substantially eliminate the impact of the boundary layer on the release rate of the active substance from a polymer matrix composition by ensuring that the thickness of the boundary layer is relatively small and/or that the release of the active substance from a polymer matrix composition is governed by erosion of the composition and the diffusion of the active substance through the boundary layer, if any, has no or only a small impact on the overall release rate.

The present inventors have found that when water is allowed to diffuse into a polymer matrix composition zero order release is obtained when the release rate is governed or controlled by erosion of a constant surface area per time unit. In order to ensure that the erosion of the polymer matrix composition is the predominant release mechanism, the inventors have found that it is necessary to provide a polymer matrix composition which has properties that ensures that the diffusion rate of water into the polymer matrix composition substantially corresponds to the dissolution rate of the polymer matrix composition into the aqueous medium. Thus, by adjusting the nature and amount of constituents contained in the polymer matrix composition along this line the present inventors have obtained polymer matrix compositions, which release the active substance by a zero order release mechanism. The compositions employed are coated in such a manner that at least one surface is exposed to the aqueous medium and this surface has a substantially constant or controlled surface area during erosion. In the present context controlled surface area relates to a predetermined surface area typically predicted from the shape of the coat of the unit dosage system. It may have a simple uniform cylindrical shape or the cylindrical form can have one or more tapered ends in order to decrease (or increase) the initial release period.

A suitable method for controlling the release of at least one therapeutically, prophylactically and/or diagnostically active substance into an aqueous medium by erosion of at least one surface of a pharmaceutical composition comprising

i) a matrix composition comprising a) polymer or a mixture of polymers, b) an active substance and, optionally, c) one or more pharmaceutically acceptable excipients, and

ii) a coating having at least one opening exposing at the one surface of said matrix, the coating comprising

a) a first cellulose derivative which has thermoplastic properties and which is substantially insoluble in the aqueous medium in which the composition is to be used,
and at least one of
b) a second cellulose derivative which is soluble or dispersible in water,
c) a plasticizer, and
d) a filler,

the method comprising adjusting the concentration and/or the nature of the ingredients making up the matrix composition in such a manner that the diffusion rate of the aqueous medium into the matrix composition corresponds to about 100% $\pm$ 30% such as, e.g. about 100% $\pm$ 25%, about 100% $\pm$ 20%, about 100% $\pm$ 15% or about 100% $\pm$ 10% or about 100% of the dissolution rate of the matrix composition so as to obtain a zero order release of at least about 60% w/w such as, e.g. at least about 65% w/w at least about 70% w/w, at least about 75% w/w, at least about 80% w/w, at least about 85% w/w, at least about 90% w/w, at least about 95% w/w or at least about 97 or 98% w/w of the active substance from the pharmaceutical composition when subject to an in vitro dissolution test as described herein.

In a specific embodiment of the invention, the polymer a) is a substantially water soluble or crystalline polymer or a mixture of substantially water soluble and/or crystalline polymers.

By use of such a method it is possible already during the developmental work to test various polymer matrix compositions with respect to diffusion rate of water into the composition and to dissolution rate of the polymer matrix composition in an aqueous medium. Based on such results adjustment of e.g. the concentration and/or nature of the individual constituents in the composition may be performed until the diffusion rate balance the dissolution rate. In such a manner, a relatively simple instrument has been provided in order to ensure a zero order release rate from the final composition.

In another aspect, the invention relates to a pharmaceutical composition for controlled release of at least one therapeutically, prophylactically and/or diagnostically active substance into an aqueous medium by erosion of at least one surface of the composition, the composition comprising

i) a matrix composition comprising a) a polymer or polymers, b) an active substance, which is present in the polymer (s) at least partly in amorphous form and, optionally, c) one or more pharmaceutically acceptable excipients, and
ii) a coating having at least one opening exposing at the one surface of said matrix, the coating comprising

a) a first cellulose derivative which has thermoplastic properties and which is substantially insoluble in the aqueous medium in which the composition is to be used,

and at least one of
b) a second cellulose derivative which is soluble or dispersible in water,
c) a plasticizer, and
d) a filler, and the concentration and/or the nature of the ingredients making up the matrix composition has been adjusted in such a manner that the diffusion rate of the aqueous medium into the matrix composition corresponds to about 100% $\pm$ 30% such as, e.g. about 100% $\pm$ 25%, about 100% $\pm$ 20%, about 100% $\pm$ 15% or about 100% $\pm$ 10% or 100% of the dissolution rate of the matrix composition so as to obtain a zero order release of at least about 60% w/w such as, e.g. at least about 65% w/w at least about 70% w/w, at least about 75% w/w, at least about 80% w/w, at least about 85% w/w, at least about 90% w/w, at least about 95% w/w or at least about 97 or 98% w/w of the active substance from the pharmaceutical composition when subject to an in vitro dissolution test as described herein.

As mentioned before, in a specific embodiment, the polymer a) is a substantially water soluble or crystalline polymer or a mixture of substantially water soluble and/or crystalline polymers.

**Matrix composition**

The pharmaceutical composition according to the invention comprises a matrix composition in the form of a solid dispersion comprising

a) a polymer or a mixture of polymers,
b) an active substance and, optionally,
c) one or more pharmaceutically acceptable excipients.

In a specific embodiment, the polymer is a substantially water soluble or crystalline polymer or a mixture of substantially water soluble and/or crystalline polymers.

**Polymers**

Suitable polymers for use according to the invention typically comprises a polyglycol, e.g. in the form of a homopolymer and/or a copolymer. In a specific embodiment the polymer is substantially water soluble or crystalline polymer or a mixture of substantially water soluble and/or crystalline polymers. Suitable polymers for use in a composition according to the invention are polyethylene oxides and/or block copolymers of ethylene oxide and propylene oxide. Polyethylene oxides which are suitable for use in the matrix composition are those having a molecular weight of from about 20,000 daltons, such as, e.g., from about 20,000 to about 700,000 daltons, from about 20,000 to about 600,000 daltons, from about 35,000 to about 500,000 daltons, from about 35,000 to about 400,000 daltons, from about 35,000 to about 300,000 daltons, from about 50,000 to about 300,000 daltons, such as, e.g. about 35,000 daltons, about 50,000 daltons, about 75,000 daltons, about 100,000 daltons, about 150,000 daltons, about 200,000 daltons, about 250,000 daltons, about 300,000 daltons or about 400,000 daltons.

A particular suitable polyethylene oxide is one, which in itself has a suitable balance between the diffusion rate of water into the polymer and a dissolution rate of the polymer. Suitable examples are polyethylene oxides having a molecular weight of about 35,000 daltons, about 50,000 daltons, about 100,000 daltons, about 200,000 daltons, about 300,000 daltons and about 400,000 daltons.

Typical block copolymers of ethylene oxide and propylene oxide may comprise up to about 30% w/w of the propylene oxide based block, and has a molecular weight of about 5,000 daltons, typically about 5,000 to about 30,000 daltons such as, e.g. from about 8,000 to about 15,000 daltons.

Polyethylene glycols (which when the molecular weight is above about 20,000 is denoted polyethylene oxides) are mixtures of condensation polymers of ethylene glycol.

The average molecular weight (MW) can be calculated from the following equation

$$MW = (56,110 \times 2)/ \text{hydroxyl number}$$

Where the hydroxyl number is defined as the number indicating the amount in mg of potassium hydroxide, which is equivalent to the acetic acid, which, by acetylation, is bound by 1 g of a substance.

Mixtures of PEO with different average molecular weights can be used in order to obtain a PEO with a desirable average molecular weight. It is important to note that in such cases it is necessary to use the PEO, which have MW closest to the desired molecular weight. The individual amount of the two PEO necessary to obtain a PEO with a desired MW can be calculated from the hydroxyl number and the equation given above.

The polymer may have a melting point, which is above the body temperature of the human or animal in which the composition is to be used. Thus, the polymer(s) employed in the matrix composition will suitably have a melting point of about 20-120°C such as, e.g. from about 30 to about 100°C or from about 40 to about 80°C.

Alternatively to a polymer of a polyglycol type as described above other polymers may be suitable for use in the matrix composition a). Thus, in other embodiments of the invention, the polymer is selected from one or more of the following polymers: water soluble natural polymers such as glucomannan, galactan, glucan, polygalacturonic acid, polyxylane, polygalactomannans, rhanogalacturonan, polyxyloglycan, arabinogalactan, and starch; water soluble polymers such as PVA, PVB, methocel, Eudragit L methyl ester and PHPV; biodegradable polymers such as PHA, and PLA; hydrogels, such as olyacrylic amid, and dextran; copolymers such as polylactic acid with polyglycolic acid; and others such as alginate and pectins including low methylated or methoxylated pectins.

**Active substances**

A composition according to the invention and the concept of obtaining a stable composition comprising a solid dispersion of the active substance can also be applied to other active substances than e.g. carvedilol. A pharmaceutical composition according to the invention comprises one or more active substances, i.e. substances, which are therapeutically, prophylactically, diagnostically and/or biologically active substance. The term "active substance" as used herein broadly includes any compound, or mixture thereof, that can be delivered from the composition to produce a beneficial result.

As discussed above, a composition of the present invention is especially suitable for incorporation of crystalline active substances that are convertible into an amorphous form by gentle heating and at the same time have limited water solubility. However, there may be situations where it is desirable to employ other active substance such as, e.g. more water soluble active substance. The following lists encompass both water soluble and less water soluble active substances.

The active and beneficial agents include pesticides, herbicides, germicides, biocides, algicides, rodenticides, fungicides, insecticides, antioxidants, plant hormone promoters, plant growth inhibitors, preservatives, disinfectants, sterilization agents, catalysts, chemical reactants, fermentation agents, food supplements, nutrients, cosmetics, therapeutically active substances (drugs), vitamins, sex sterilants, fertility inhibitors, fertility promoters, air purifiers, microorganism attenuators, ecological agents and other agents that benefit the environment in which they are used.

In the present context the term "drug substance" includes any physiologically or pharmacologically active substance that produces a localized or systemic effect in animals, in particular in mammals, including humans and primates. Other animals include domestic household, sport or farm animals such as sheep, goats, cattle, horses and pigs, laboratory animals such as mice, rats and guinea pigs, fishes, avians, reptiles and zoo animals. The term "therapeutically, prophylactically and/or diagnostically active substance" includes the term drug substance within its meaning.

In the present context, the term "ecological agent" denotes a non-therapeutic substance that has a biological effect on plants or animals in the environment. An ecological agent may be a pesticide, such as an insecticides or herbicide, a fertilizer a pheromone, a plant growth hormone or the like.

The active substance or substances included in a pharmaceutical composition of the invention may be selected from many therapeutic categories, in particular from substances which may advantageously be administered orally, rectally, vaginally, or administered to a body cavity (e.g. the urinary bladder, kidney pelvis, the gall bladder, the uterus, a central nervous system cavity, infectious/malignant/post-operative cavities, etc.).

Examples of such substances are hypnotics, sedatives, tranquilizers, anti-convulsants, muscle relaxants, analgesics, anti-inflammatory, anaesthetics, anti-spasmodics, antiulcer-agents, anti-parasitics, anti-microbials, anti-fungal, cardiovascular agents, diuretics, cytostatics, anti-neoplastic agents, anti-viral agents, anti-glaucoma agents, antidepressants, sympathomimetics, hypoglycaemics, diagnostic agents, anti-cough, physic energizers, anti-parkinson agents, local anesthetics, muscle contractants, anti-malarials, hormonal agents, contraceptives, anorexic, anti-arthritic, anti-diabetic, anti-hypertensive, anti-pyretic, anti-cholingergic, bronchodilator, central nervous system, inotropic, vasodilator, vasoconstrictor, decongestant, hematine, iron salts and complexes, electrolyte supplement, germicidal, parasympathetolytic, parasympathethomimetic, antiemetic, psychostimulant, vitamin, beta-blockers, H-2 blocker, beta-2 agonist, counterirritants, coagulating modifying agents, stimulants, anti-hormones, drug-antagonists, lipid-regulating agents, uricosurics, cardiac glycosides, ergots and derivatives thereof, expectorants, muscle-relaxants, anti-histamines, purgatives, contrastmaterials, radiopharmaceuticals, imaging agents, anti-allergic agents.

Examples of specific active substances suitable for use in a composition of the invention are:

Carvedilol, morphine, diclofenac, nifedipine, calcitonin, rivastigmine, methylphenidate, fluoroxetine, rosiglitazone, prednison, prednisolone, codeine, ethylmorphine, dextromethorphan, noscapine, pentoxiverine, acetylcysteine, bromhexine, epinephrine, isoprenaline, orciprenaline, ephedrine, fenoterol, rimiterol, ipratropium, cholinetheophyllinate" proxiphylline, bechlomethasone, budesonide, deslanoside, digoxine, digitoxin, disopyramide, proscillaridin, chinidine, procainamide, mexiletin, flecainide, alprenolol, proproanolol, nadolol, pindolol, oxprenolol, labetalol, timolol, atenolol, pentaeritrityltetranitrate, isosorbiddinitrate, isosorbidmononitrate, niphedipin, phenylamine, verapamil, diltiazem, cyclandelar, nicotinylalcholhol, inositolnicotinate, alprostatdil, etilephrine, prenalterol, dobutamine, dopamine, dihydroergotamine, guanetidine, betanidine, methyldopa, reserpine, guanfacine, trimethaphan, hydralazine, dihydralazine, prazosine, diazoxid, captopril, nifedipine, enalapril, nitroprusside, bendroflumethiazide, hydrochlorthiazide, metychlothiazide, polythiazide, chlorthalidon, cinetazon, clopamide, mefruside, metholazone, bumetanide, ethacrynacide, spironolactone, amiloride, chlofibrate, nicotinic acid, nicheritrol, brompheniramine, cinnarizine, dexchlorpheniramine, clemastine, antazoline, cyproheptadine, proethazine, cimetidine, ranitidine, sucralfat, papaverine, moxaverine, atropin, butylscopolamin, emepron, glucopyrron, hyoscyamine, mepensolar, methylscopolamine, oxiphencyclimine, probanteline, terodilin, sennaglycosides, sagradaextract, dantron, bisachodyl, sodiumpicosulfat, etulos, diphenolxylate, loperamide, salazosulfapyridine, pyrvin, mebendazol, dimeticon, ferrofumarate, ferrosuccinate, ferritetrasemisodium, cyanochobalamine, folid acid heparin, heparin co-factor, diculmarole, warfarin, streptokinase, urokinase, factor VIII, factor IX, vitamin K, thiopeta, busulfan, chlorambucil, cyclophosphamid, melfalan, carmustin, mercatopurin, thioguanin, azathioprin, cytarabin, vinblastin, vinchristin, vindesin, procarbazine, dacarbazine, lomustin, estramustin, teniposide, etoposide, cisplatin, amsachrin, aminogluthetimid, phosphestrol, medroxiprogresterone, hydroxiprogesterone, megesterol, noretisteron, tamoxiphen, ciclosporin, sulfosomidine, bensylpenicillin, phenoxymethylpenicillin, dicloxacillin, cloxacillin, flucoxacillin, ampicillin, amoxicillin, pivampicillin, bacampicillin, piperacillin, mezlocillin, mecillinam, pivmecillinam, cephalotin, cephalexin, cephradin, cephadroxil, cephaclor, cefuroxim, cefotaxim, ceftazidim, cefoxitin, aztreonam, imipenem, cilastatin, tetracycline, lymecydine, demeclocycline, metacycline, oxitetracycline, doxycycline, chloramphenicol, spiramycin, fusidic acid, lincomycin, clindamycin, spectinomycin, rifampicin, amphotericin B, griseofulvin, nystatin, vancomycin, metronidazole, tinidazole, trimethoprim, norfloxacin, salazosulfapyridin, aminosalyl, isoniazid, etambutol, nitrofurantoin, nalidixic acid, metanamine, chloroquin, hydroxichloroquin, tinidazol, ketokonazol, acyclovir, interferon idoxuridin, retinal, tiamin, dexpantenol, pyridoxin, folic acid, ascorbic acid, tokoferol, phytominadion, phenfluramin, corticotropin, tetracosactid, tyrotropin, somatotoprin, somatrem, vasopressin, lypressin, desmopressin, oxytocin, chloriongonadotropin, cortison, hydrocortisone, fluodrocortison, prednison, prednisolon, fluoximesteron, mesterolon, nandrolon, stanozolol, oximetolon, cyproteron, levotyroxin, liotyronin, propylthiouracil, carbimazol, tiamazol, dihydrotachysterol, alfacalcidol, calcitirol, insulin, tolbutamid, chlorpropamid, tolazamid, glipizid, glibenclamid, phenobarbital, methyprylon, pyrityldion, meprobamat, chlordiazepoxid, diazepam, nitrazepam, oxazepam, dikaliumclorazepat, lorazepam, flunitrazepam, alprazolam, midazolam, hydroxizin, chlometiazol, propionmazine, alimemazine, chlorpromazine, levomepromazine, acetophenazine, fluphenazine, perphenazine, prochlorperazine, trifluoperazine, dixyrazine, thiodirazine, periciazin, chloprothixene, zuclopentizol, flupentizol, thithixen, haloperidol, trimipramin, opipramol, chlomipramin, desipramin, lofepramin, amitriptylin, nortriptylin, protriptylin, maptrotilin, caffeine, cinnarizine, cyclizine, dimenhydinate, meclozine, prometazine, thiethylperazine, metoclopramide, scopolamine, phenobarbital, phenytoine, ethosuximide, primidone, carbamazepine, chlonazepam, orphenadrine, atropine, bensatropine, biperiden, metixene, procylidine, levodopa, bromocriptin, amantadine, ambenon, pyridostigmine, synstigmine, disulfiram, morphine, codeine, pentazocine, buprenorphine, pethidine, phenoperidine, phentanyl, methadone, piritramide, dextropropoxyphene, ketobemidone, acetylsalicylic acid, phenazone, phenylbutazone, azapropazone, piroxicam, ergotamine, dihydroergotamine, cyproheptadine, pizitifen, flumedroxon, allopurinol, probenecid, sodiummaurothiomalate auronofin, penicillamine, estradiol, estradiolvalerianate, estriol, ethinylestradiol, dihydrogesteron, lynestrenol, medroxiprogresterone, noretisterone, cyclophenile, clomiphene, levonorgestrel, mestranol, ornidazol, tinidazol, ekonazol, chlotrimazol, natamycine, miconazole, sulbentin, methylergotamine, dinoprost, dinoproston, gemeprost, bromocriptine, phenylpropanolamine, sodiumchromoglicate, azetasolamide, dichlophenamide, betacarotene, naloxone, calciumfolinate, in particular clonidine, thephylline, dipyradamol, hydrochlothiazade, scopolamine, indomethacine, furosemide, potassium chloride, morphine, ibuprofen, salbutamol, terbutalin, sulfonylurea, metformin, insulin, calcitonin, glucagons-like peptide-1.

The active substance can be in various forms, such as uncharged molecules, molecular complexes, crystalline forms, amorphous forms, polymorphous form, solvates, anhydrates, and pharmaceutically acceptable salts such as a hydrochloride, hydrobromide, sulfate, laurylate, palmitate, phosphate, nitrite, nitrate, borate, acetate, maleate, tartrate, oleate, and salicylate. For acidic active substance, salts of metals, amines amino acids or organic cations, quaternary ammoniums, can be used. Derivatives of active substances such as esters, ethers and amides which have solubility characteristics suitable for use herein can be used alone or mixed with other drugs. After release of the derivative from the composition it may be converted by enzymes, hydrolysed by body pH or other metabolic processes to the parent drug or to another biologically active form.

A pharmaceutical composition of the invention may in addition be suitable for the delivery of polypeptides, for example hormones, enzymes such as lipases, proteases, carbohydrates, amylases, lactoferrin, lactoperoxidases, lysozymes, nanoparticles, etc., and antibodies. The composition may also be employed for the delivery of microorganisms, either living, attenuated or dead, for example bacteria, e.g. gastrointestinal bacteria such as streptococci, e.g. *S. faecium, Bacillus* spp. such as *B. subtilis* and *B. licheniformis,* lactobacteria, *Aspergillus* spp., bifidogenic factors, or viruses such as indigenous vira, enterovira, bacteriophages, e.g. as vaccines, and fungi such as baker's yeast, *Saccharomyces cerevisiae* and fungi imperfecti. A pharmaceutical composition of the invention may also be used for the delivery of active agents in specialized carriers such as liposomes, cyclodextrines, nanoparticles, micelles and fats.

A further use for which a composition of the invention is suited is the delivery of active substances to animals. Examples of such active substances for veterinary use are antiparasitics, corticosteroids, antibiotics, antiimflammatory agents, growth promoters and permittants, antifungals and antihelmintics.

A pharmaceutical composition of the invention is designed to release the active substance in a controlled manner such as by a zero order release mechanism. Accordingly, the composition is especially suitable for a controlled release of an active substance. In the present context the tern "controlled release" is used to designate a release a desired rate during a predetermined release period. Terms like "modified", "delayed", "sustained", "prolonged", "extended" etc. release are in the present context synonyms to the term "controlled release".

In an embodiment of the invention, the active substance is a pharmaceutically active powder. The powder typically has a particle size of from about 0.01 $\mu$m to about 500 $\mu$m, 0.1 $\mu$m to about 500 $\mu$m, typically from about 0.5

μm to about 300 μm, more typically from about 1 μm to about 200 μm, especially from about 5 μm to about 100 μm.

A pharmaceutical composition according to the invention is - due to the possibility of designing the composition in such a manner that i) a zero order release is obtained and ii) a controlled release during a predetermined time period is obtained - suitable for use for water soluble as well as slightly soluble or insoluble active substances. However, it is contemplated that a composition is especially suitable for use when the at least one therapeutically, prophylactically and/or diagnostically active substance has a solubility of at the most about 3 mg/ ml such as, e.g. at the most about 1 mg/ml, at the most about 0.1 mg/ml, at the most about 0.05 mg/ml such as, e.g. at the most about 0.001 mg/ml in water at ambient temperature and/or a prolonged release of the active substance is desired in order to obtain i) a prolonged residence time within the body after administration, ii) a reduced peak plasma concentration in order to avoid peak related side effects, iii) reduced frequency of administration in order e.g. to obtain a better patient compliance, etc.

To this end it seems that substantially hydrophobic active substances tend to result in a decrease in the erosion rate of the matrix composition. Substantially hydrophilic or water-soluble active substances seem to have the opposite effect, i.e. they tend to result in a faster erosion of the matrix.

The at least one therapeutically, prophylactically and/or diagnostically active substance will suitably be present in an amount of up to about 60%, typically up to about 50%, by weight of the matrix composition. An active substance content of about 60% is contemplated to be the maximum content, which still allows for a sufficient content of the polymer and, when relevant, the pharmaceutically acceptable excipient in the composition. The active substance may, on the other hand, be present in the composition in much smaller amounts, depending on the nature and potency of the active substance in question.

**Pharmaceutically acceptable excipients**

In general, the stabilizing agents mentioned herein before may also be employed as pharmaceutically acceptable excipients.

*Other ingredients in the matrix composition*

The matrix composition may also contain other excipients as well, e.g. in order to improve the technical properties of the matrix composition so that it may be easier to produce or in order to improve the stability of the composition.

A suitable pharmaceutically acceptable excipient for use in a matrix composition of the invention may be selected from the group consisting of fillers, diluents, disintegrants, glidants, pH-adjusting agents, viscosity adjusting agents, solubility increasing or decreasing agents, osmotically active agents and solvents.

Suitable excipients include conventional tablet or capsule excipients. These excipients may be, for example, diluents such as dicalcium phosphate, calcium sulfate, lactose or sucrose or other disaccharides, cellulose, cellulose derivatives, kaolin, mannitol, dry starch, glucose or other monosaccharides, dextrin or other polysaccharides, sorbitol, inositol or mixtures thereof; binders such as acacia, sodium alginate, starch, gelatin, saccharides (including glucose, sucrose, dextrose and lactose), molasses, extract of Irish moss, panwar gum, ghatti gum, mucilage of isapol husk, carboxymethylcellulose, methylcellulose, veegum, larch arabolactan, polyethylene glycols, ethylcellulose, water, alcohols, waxes, polyvinylpyrrolidone such as, e.g., PVP K90 (may be used to improve mixing of the polymer with the other ingredients) or mixtures thereof; lubricants such as talc, magnesium stearate, calcium stearate, staeric acid, hydrogenated vegetable oils, sodium benzoate, sodium chloride, leucine, carbowax 4000, magnesium lauryl sulfate, colloidal silicon dioxide and mixtures thereof, disintegrants such as starches, clays, cellulose derivatives including crosscarmellose, gums, aligns, various combinations of hydrogencarbonates with weak acids (e.g. sodium hydrogencarbonate/tartaric acid or citric acid) crosprovidone, sodium starch glycolate, agar, cation exchange resins, citrus pulp, veegum HV, natural sponge, bentonite or mixtures thereof; volatile solvents such as alcohols, including aqueous alcohols, petroleum benzine, acetone, ether or mixtures thereof; plasticizers such as sorbitol and glycerine; and others such as cocoa butter, polyethylene glycols or polyethylene oxides, e.g. with a molecular weight of about 1,000-500,000 daltons, typically about 1,000-100,000 daltons, more typically 1,000-50,000 daltons, especially about 1,000-10,000 daltons, in particular about 1,500-5,000 daltons, and mixtures thereof, hydrogenated vegetable oils, glycerinated gelatin or mixtures thereof.

The matrix composition may in addition include a cellulose derivative, e.g. a cellulose derivative selected from the group consisting of methylcellulose, carboxymethylcellulose and salts thereof, microcrystalline cellulose, ethylhydroxyethylcellulose, ethylmethylcellulose, hydroxyethylcellulose, hydroxyethylmethylcellulose, hydroxypropylcellulose, hydroxypropylmethylcellulose, hydroxymethylcellulose and hydroxymethylpropylcellulose. Of these cellulose derivatives, hydroxypropylmethylcellulose and methylcellulose are preferred for incorporation in the matrix composition.

Furthermore, the matrix composition may comprise one or more agents selected from the group consisting of sweetening agents, flavouring agents and colouring agents, in order to provide an elegant and palatable preparation. Examples of colouring agents are water soluble FD&C dyes and mixtures thereof with corresponding lakes and direct compression sugars such as Di-Pac from Amstar. In addtion, coloured dye migration inhibitors such as tragacanth, acacia or attapulgite talc may be added. Specific examples include Calcium carbonate, Chromium-cobalt-aluminium oxide, ferric ferrocyanide, Ferric oxide, Iron ammonium citrate, Iron (III) oxide hydrated, Iron oxides, Magnesium carbonate, Titanium dioxide.

Examples of suitable fillers are also dextrin, sucralfate, calcium hydroxyl-apatite, calcium phosphates and fatty acid salts such as magnesium stearate.

The filler may be added in an amount so that the combination of the filler and the active substance comprises up to about 60%, typically up to about 50%, by weight of the first composition.

In order to soften the carrier system, a plasticziser may be incorporated in the composition. A suitable plasticizer is selected from the group consisting of phosphate esters; phthalate esters; amides; mineral oils; fatty acids and esters; fatty alcohols, vegetable oils and hydrogenated vegetable oils including acetylated hydrogenated cottonseed glyceride and acetylated hydrogenated soybean oil glycerides; acetyl tributyl citrate, acetyl triethyl citrate, Castor oil, diacetylated monoglycerides, dipropylene glycol salicylate glycerin, glyceryl cocoate, mono- and di-acetylated monoglycerides, nitrobenzene, carbon disulfide, β-naphtyl salicylate, phthalyl glycolate, diocyl phthalate; sorbitol, sorbitol glyceryl tricitrate; sucrose octaacetate; a-tocopheryl polyethylene glycol succinate, phosphate esters; phthalate esters; amides; mineral oils; fatty acids and esters; fatty alcohols; and vegetable oils, fatty alcohols including cetostearyl alcohol, cetyl alcohol, stearyl alcohol, oleyl alcohol and myristyl alcohol; methyl abietate, acetyl tributyl citrate, acetyl triethyl citrate, diisooctyl adipate, amyl oleate, butyl ricinoleate, benzyl benzoate, butyl and glycol esters of fatty acids, butyl diglycol carbonate. butyl oleate, butyl stearate. di(beta-methoxyethyl) adipate, dibutyl sebacate, dibutyl tartrate, diisobutyl adipate, dihexyl adipate, triethylene glycol di(beta-ethyl butyrate), polyethylene glycol di(2-ethyl hexoate), diethylene glycol monolaurate, monomeric polyethylene ester, hydrogenated methyl ester of rosin, methoxyethyl oleate, butoxyethyl stearate, butyl phthalyl butyl glycolate, glycerol tributyrate, triethylene glycol dipelargonate, beta-(p-tert-amyl phenoxy)ethanol, beta(p-tert-butytphenoxy)ethanol, beta-(p-teft-butytphenoxyethyl)acetate, bis(beta-p-tert-buthylphenoxydiethyl)ether. camphor, Cumar W-1, Cumar MH-1, Cumar V-1, diamyl phthalate, (diamylphenoxy) ethanol, diphenyl oxide, technical hydroabietyl alcohol, beckolin, benzene hexahydrochlonde, Clorafin 40, Piccolastic A-5, Piccalastic A-25" Flexol B-400, Glycerol alfa-methyl alfa-phenyl ether, chlorinated naphthalene, HB-40, monoamylphthalate. Nevillac 10 o-nitrodiphenyl and Paracril 26.

Preferred anti-oxidative agents include TPGS due to surfactant properties, BHA, BHT, t-butyl hydroquinone, calcium ascorbate, gallic acid, hydroquinone, maltol, octyl gallate, sodium bisulfite, sodium metabisulfite,tocopherol and derivates thereof, citric acid, tartaric acid, and ascorbic acid. Other antioxidants include trivalent phosphorous like e.g phosphite, phenolic antioxidants, hydroxylamines, lactones such as substituted benzofuranones. Hindered phenols, thiosynergists and/or hindered amines are useful for the long-term stability for polymers, whereas the following antioxidants are suitable for use also in situation where the active substance is subject to oxidation: acids (ascorbic acid, erythorbic acid, etidronic acid, gallic acid, hypophosphorous acid, nordihydroguairetic acid, propionic acid etc.), phenols (e.g. BHA, BHT, t-butyl hydroquinone, dodecyl gallate, octyl gallate, 1,3,5-trihydroxybenzene), organic and inorganic salts (calcium ascorbate, sodium ascorbate, sodium bisulphite, sodium metabisulfite, sodium sulfite, potassium bisulphite, potassium metabisulphite), esteres (calcium ascorbate, dilauryl thiodipropionate, dimyristyl thiodipropionate, distearyl thiodipropionate), pyranon (maltol), and vitamin E (tocopherol, D-α-tocopherol, DL-α-tocopherol, tocopheryl acetate, d-α-tocopheryl acetate, dl-α-tocopheryl acetate. However, other anti- oxidative agents known in the art may be used according to the present invention.

**pH dependant release**

In some situations it may be convenient that the composition releases the active substance in a pH dependant manner. As described in e.g. WO 99/51208 a pH dependant release can be obtained by inclusion of a so-called release rate modifier. The release rate modifier is preferably selected from materials conventionally used in the pharmaceutical industry to produce enteric coatings. A number of different types of compounds suitable for use as enteric coatings are known in the art; see e.g. *Remington's Pharmaceutical Sciences,* 18th Edition, 1990. Release modifiers may in particular be selected from one of three general classes, namely cellulose derivatives, methacrylic acid polymers and modified gelatine compounds. Preferred release modifiers include cellulose acetate phthalate, polyvinyl acetate phthalate, hydroxypropyl methylcellulose phthalate and hydroxypropyl methylcellulose acetate succinate, as well as methacrylic acid copolymers. Modified gelatine compounds include gelatine treated with e.g. formaldehyde or glutaraldehyde.

Examples of commercially available polymers suitable as release modifiers are EUDRAGIT® L and EUDRAGIT® S, available from Röhm GmbH, Germany, and enteric coating agents available from Shin-Etsu Chemical Co., Japan. The release modifier will typically be present in the composition in an amount of about 0.1-10%, based on the weight of the matrix, preferably about 0.5-4%, e.g. about 1-3%, such as about 1.5-2.0%. If desired, a suitable mixture of more than one release modifier may be used in order to obtain a desired release profile in any given composition.

The release modifier enables a difference in release of the active substance/erosion of the matrix dependant on pH.

**Shape**

The geometric form of the composition is important for the obtainment of the above-mentioned controlled zero order. Thus, in a preferred version of the invention, the pharmaceutical composition of the invention has a geometric shape, which enables a substantially constant surface area to become exposed during erosion of the matrix.

Specific examples of compositions with different shapes and sizes are:

| Batch | Length [mm] | Diameter [mm] | Vol [mm$^3$] |
|---|---|---|---|
| 01-0034-042 | 7,5 | 5,05 | 150 |
| 01-0035-042 | 6,0 | 5,64 | 150 |
| 01-0043-042 | 9,0 | 4,6 | 150 |

The following table describes formulations having a cylindrical form and oval openings in both ends

| Batch | Length [mm] | Vol [mm$^3$] | Longest/shortest diameter [mm] | |
|---|---|---|---|---|
| 01-0075-042 | 6.0 | 150 | 8.74 | 3.64 |
| 01-0076-042 | 7.5 | 150 | 7.82 | 3.21 |

The coated compositions obtained were open at two opposite ends.

The area for an open end is calculates as the volume/length of the cylindrical formulations.

In a further embodiment of the invention it is possible to prepare different strength based on only one specific matrix composition.

The different strengths of the pharmaceutical composition are then prepared based on a desired specific formulation, which has shown the desired release duration. The release period is then secured by keeping the same length in each strength formulation. Simply by decreasing or increasing the exposed area with the same fold as the desired increase or decrease, respectively, in the desired strength compared to the strength of the basis formulation different. In other words, the ratio between the amount of active substance and surface area of the original basis formulation is constant in each individual strength formulation.

However, minor corrections in the calculated area for the additional strength formulations may be necessary in case the erosion rate (length of the eroded matrix/time unit) is dependent on the size of the area indicating non-linearity. However such non-linearity may be tested by measuring the erosion rate individually with two different exposed areas of the same matrix composition. In case the formulations show different dissolution rates, the ratio between the areas and the rates may be calculated.

For instance, the present according to the present invention, the results from Examples 1 to 4 demonstrates that

| Round | 7.5mm | 5.05 mm diameter | 8 hours | 0.94 mm/h |
|---|---|---|---|---|
| Round | 9mm | 4.6 mm diameter | 9 hours | 1.00 mm/h |
| Oval | 6mm | 8,74/3.64 mm diam. | 5.33 hours | 1.12mm/h |
| Oval | 7.5mm | 7,82/3.21 mm diam. | 6.49 hours | 1.15mm/h |

Accordingly the release rate of the present matrix formulation is increased with decreased area. The ratio between the two rates is 0.94:1 and not 1:1

The ratio between the areas is 1. 1: for the round formulations.

These factors can be used to adjust the area and/or the length of the specific desired new strength when exactly the same matrix is preferred in different pharmaceutical strengths.

Such increase in dissolution rate with decreasing exposed area may be an advantage as it is expected that smaller areas in vivo may result in relative slower release.

In vitro, it is believed that when the area is decreased, the physical factors of the dissolution parameters, (paddle rotation speed) might have a decreased erosion effect on the surface area bearing in mind the present shape of the formulation is a tube where the coat or wall of the tube remains intact during the erosion process.

The observation from the results mentioned above may relate to the low solubility of carvedilol at high pH values. With smaller diameters, more shell wall is present per mm$^2$ surface to protect de formulation from diffusion of the buffer solution.

In a still further embodiment, a formulation as disclosed in Batch 084 (12% load, 6 mm oval, 150 mm$^3$ corresponding to 25 mg) having a erosion time of 5.6 hours and a length of 6 mm resulting in a dissolution rate of approximately 1 mm/h (1.06 mm/h calculated) may be used for the preparation of dosages of 12.5 mg and 6,25 mg simply decreasing the area of batch 084 by a factor 2 and 4 respectively. Furthermore, a 50 mg may be prepared by increasing the area with a factor 2 and in case the size of the formulation is being bigger than desired, the load may be increased. Consequently, if the load is increased to 18% from 12%, the area is increased 1.5 in order to provide a 50 mg formulation.

In a further embodiment of the invention the design of a formulation may be made based on the dissolution of a different formulation. If the desired rate is corresponding to the 6 mm oval formulation as used in the clinical study disclosed herein having a dissolution rate of 1 mm/h and the basis formulation has a dissolution rate of 1.08. The calculated length would be 5.55 mm and the exposed area may be adjusted accordingly to have the desired content.

Accordingly, preferred designs of a formulation wherein the dissolution rate is 1.08 in a oval 6 mm shape with a surface area of 25 mm$^2$ and a load of Carvedilol and wherein the desired dissolution duration is 5.5 hours is a formulation having a length of 5.5 mm

The surface areas may be adjusted to the desired content of active substance as illustrated above

Other designs according to the present invention for a 25 mg Carvedilol having a volume of 159 mm$^3$ includes:

Surface area (one end) of 27.17 mm$^2$ and 5.85 mm length
Surface area (one end) of 25.00 mm$^2$ and 6.40 mm length

Designs for a 50 mg Carvedilol having a volume of 318 mm$^3$ includes:

Surface area (one end) of 45 mm$^2$ and 7 mm length
Surface area (one end) of 50 mm$^2$ and 6.4 mm length
Surface area (one end) of 55 mm$^2$ and 5.6 mm length

Designs for a 12,5 mg Carvedilol having a having a volume of 79.5 mm$^3$ includes
Surface area (one end) of 13.6 mm$^2$ and 5.85 mm length
Designs for a 6.25 mg Carvedilol having a volume of 39.75 mm$^3$ includes:

Surface area (one end) of 6.8 mm$^2$ and 5.85 mm length
Such small formulations may be prepared with a thicker shell for patient compliance reasons. The final size of all the formulations may be adjusted simply with adjusting the thickness of the shell for example by selecting the oaverall size of the 12.5 mg formulation.

## Coating

The pharmaceutical composition may thus have the shape of a cylindrical rod, which is provided with a coating, which is substantially insoluble in and impermeable to fluids such as body fluids during the intended release period, the coating having an opening at one or both ends. Polymers useful as coatings are preferably those, which are possible to process by extrusion, solution or in the form of a dispersion. Most preferred are those, which are available in a food grade or a pharmaceutical grade quality. Examples of such polymers are cellulose acetate, polyamide, polyethylene, polyethylene terephthalate, polypropylenem polyurethane, polyvinyl acetate, polyvinyl chloride, silicone rubber, latex, polyhydroxybutyrate, polyhydroxyvalerate, teflon, polylactic acid or polyglycolic acid and copolymers thereof, copolymers such as ethylene vinyl acetate (EVA), styrene-butadienestyrene (SBS) and styrene-isoprene-styrene (SIS).

The coating may also be a coating, which is substantially soluble in and permeable to fluids such as body fluids during the intended release period provided that the coating dissolves so much slower than the matrix composition that the coating remains intact until the matrix has eroded and released the active substance. Examples of suitable polymers include polyols as described herein.

The coating may further comprise any of the above-mentioned matrix materials in a form, which erodes at a substantially slower rate than the rest of the matrix. The coating may thus comprise a matrix of one or more substantially water soluble crystalline polymers and, optionally, a non-ionic emulsifier, the coating being one which is eroded in the aqueous phase at a substantially slower rate than the matrix composition comprising the active substance, whereby a substantially constant area of the matrix composition comprising the active substance is exposed during erosion of the matrix composition, and whereby the coating is substantially eroded upon erosion of the matrix composition comprising the active substance. Such a coating will be designed so that its longitudinal erosion rate is substantially the same as the longitudinal erosion rate of the matrix, whereby the matrix and the coating will erode longitudinally towards the centre of the composition at substantially the same rate. Thus, when the matrix composition has been completely eroded by the aqueous medium, the coating will also be substantially completely eroded. A matrix composition having such a coating has the obvious advantage of being completely biodegraded upon release of the active substance. Such a coating will typically be a combination of a polyethylene glycol and a mixture of, for example, polyethylene glycol 400 monostearate or another non-ionic emulsifier, and may also include a filler. The content of the mixture of non-ionic emulsifiers and the filler in the coating will be determined in each particular case according to the characteristics, e.g. erosion rate and size, of the matrix comprising the active substance.

In an embodiment of the invention, the coating is one, which disintegrates or crumbles after erosion of the matrix. A coating of this type would remain intact as long as it was supported by the matrix containing the active substance, but it would lack the ability to remain intact after erosion of the matrix, whereby it would then disintegrate or crumble, so that it would not remain in e.g. a human or animal for any significant amount of time after the complete erosion of the matrix and the release of the active substance.

The coating may also be an enteric coating employing methacrylates, a co-polymer of methacrylate-galactomannan etc.

In an interesting embodiment, the controlled release composition of the invention further comprises a coating having at least one opening exposing at least one surface of the matrix, the coating being one which crumbles and/or erodes upon exposure to the aqueous medium at a rate which is equal to or slower than the rate at which the matrix erodes in the aqueous medium, allowing exposure of said surface of the matrix to the aqueous medium to be controlled. Coatings of this type are described in WO 95/22962, to which reference is made and which is incorporated herein by reference. These coatings comprise:

(a) a first cellulose derivative which has thermoplastic properties and which is substantially insoluble in the aqueous medium in which the composition is to be used, e.g. an ethylcellulose such as ethylcellulose having an ethoxyl content in the range of 44.5-52.5%, or cellulose acetate, cellulose propionate or cellulose nitrate;
and at least one of:
(b) a second cellulose derivative which is soluble or dispersible in water, e.g. a cellulose derivative selected from the group consisting of methylcellulose, carboxymethylcellulose and salts thereof, cellulose acetate phthalate, microcrystalline cellulose, ethylhydroxyethylcellulose, ethylmethylcellulose, hydroxyethylcellulose, hydroxyethylmethylcellulose, hydroxypropylcellulose, hydroxypropylmethylcellulose, hydroxymethylcellulose and hydroxymethylpropylcellulose;
(c) a plasticizer, e.g. selected from the group consisting of phosphate esters; phthalate esters; amides; mineral oils; fatty acids and esters thereof with polyethylene glycol, glycerin or sugars; fatty alcohols and ethers thereof with polyethylene glycol, glycerin or sugars; and vegetable oils; or a non-ionic surfactant; and
(d) a filler, e.g. selected from conventional tablet or capsule excipients such as diluents, binders, lubricants and disintegrants.

The use of a plasticizer will often be desirable in order to improve the processibility of the ethylcellulose or the first cellulose derivative. The plasticizer may also be a non-ionic surfactant, e.g. a non-ionic surfactant selected from the group consisting of diacetylated monoglycerides, diethylene glycol monostearate, ethylene glycol monostearate, glyceryl monooleate, glyceryl monostearate, propylene glycol monostearate, macrogol esters, macrogol stearate 400, macrogol stearate 2000, polyoxyethylene 50 stearate, macrogol ethers, cetomacrogol 1000, lauromacrogols, nonoxinols, octocinols, tyloxapol, poloxamers, polyvinyl alcohols, polysorbate 20, polysorbate 40, polysorbate 60, polysorbate 65, polysorbate 80, polysorbate 85, sorbitan monolaurate, sorbitan monooleate, sorbitan monopalmitate, sorbitan monostearate, sorbitan sesquioleate, sorbitan trioleate, sorbitan tristearate and sucrose esters; nitrobenzene, carbon disulfide, β-naphtyl salicylate, phthalyl glycolate, dioctyl phthalate.

Other suitable plasticizers appear from EP-B-0 746 310 to which reference is made.

A coating of this type may in addition further comprise a release modifier of the type described above, so that the coating is provided with an erosion profile similar to that of the matrix composition in terms of the relative rate of erosion in the stomach and the intestines, respectively. In this case, it may be advantageous to incorporate a somewhat higher concentration of the release modifier in the coating than the concentration of release modifier in the matrix, so as to ensure that the coating does not erode in the stomach at a faster rate than the matrix.

**Pharmaceutical composition**

As mentioned above a pharmaceutical composition according to the invention is a coated matrix composition from which the active substance is released in by a zero order release mechanism.

A composition according to the invention containing a drug substance is typically for oral administration and may be in the form of a tablet or a capsule or in the form of a multiple unit dosage form. Due to the possibility of controlling the release rate of the active substance the composition may be adapted for oral administration 1-6 times a day, normally 1-4 times daily such as 1-3 times daily. The technology may also provide compositions for administration only once or twice daily. In the present context the term "once daily" is intended to mean that it is only necessary to administer the pharmaceutical composition once a day in order to obtain a suitable therapeutic and/or prophylactic response; however, any administration may comprise co-administration of more than one dosage unit, such as, e.g. 2-4 dosage units if the amount of active substance required may not be formulated in only one composition or if a composition of a smaller size is preferred.

A composition of the invention is especially suitable for administration at bed time so that it is effective during night and at early morning hours.

The dosage of the active substance depends on the particular substance, the age, weight condition etc. of the human or animal that will be treated with the composition etc. All such factors are well known to a person skilled in the art.

The controlled release of the active substance is caused by erosion at a substantially constant rate of a surface or surfaces of the first composition

The rate at which the active substance is released from the matrix is a predetermined rate, i.e. a rate, which is controllable over a certain period of time. The release rate required in each particular instance may *inter alia* depend on the amount of active substance to be released for it to exert the desired effect, as well as on the overall dosage of the active substance contained in the matrix. The substance of which the matrix is composed and the distribution of the active substance in the matrix may therefore be selected according to one or more of these criteria to ensure the desired level of release of the active substance.

Due to the controlled release of the active substance obtainable from the pharmaceutical composition of the invention, it is possible to obtain a substantially constant rate of release of the active substance over a specific period of time, corresponding to the dosage necessary for the treatment in question, so that adherence to a strict dosage regimen, e.g. requiring administration of a drug at set intervals up to several times a day, may be dispensed with.

Furthermore, it is possible to include two or more different active substances in the pharmaceutical composition of the invention, and the two or more different active substances may be adapted to be released at different concentrations and/or intervals, thus making it easier for patients to follow a prescribed regimen.

An additional advantage of a pharmaceutical composition of the invention, compared to other known controlled release compositions, is that it may be produced by relatively simple and inexpensive methods.

Furthermore, a pharmaceutical composition according to the invention allows for the incorporation of high concentrations of the active substance relative to the size of the delivery system. This is obviously a great advantage, notably when the composition is to be used for the delivery of a therapeutically, prophylactically and/or diagnostically active substance, since it allows for the delivery of the required amount of the active substance without the size of the composition being unnecessarily large. In addition, sparingly soluble or non-soluble active substances may be readily incorporated into a composition of the invention. A composition of the invention may thus be used for the delivery of, for example, sparingly soluble or non-soluble pharmaceutical powders which can otherwise be difficult to administer.

As mentioned above, the release of the active substance from the pharmaceutical composition corresponds to a substantially zero order release determined by *in vitro* dissolution test according to USP. The substantially zero order release is obtained in a time period of at least 1 hours such as, e.g. at least 2 hours, at least 3 hours, at least 4 hours or at least 5 hours, or in a time period of at least 5 hours such as, e.g. at least 6 hours, at least 7 hours, at least 8 hours, at least 9 hours or at least 10 hours.

**Carvedilol compositions**

In the experimental section herein examples are given on suitable carvedilol containing compositions, which are based on the concept described herein.

Especially suitable polymers are those of the polyol type described herein such as, e.g. polyethylene glycol, a polyethylene oxide and/or a block copolymer of ethylene oxide and propylene oxide. The polymers have a molecular weight of from about 20,000 daltons, such as, e.g., from about 20,000 to about 700,000 daltons, from about 20,000 to about 600,000 daltons, from about 35,000 to about 500,000 daltons, from about 35,000 to about 400,000 daltons, from about 35,000 to about 300,000 daltons, from about 50,000 to about 300,000 daltons, such as, e.g. about 35,000 daltons, about 50,000 daltons, about 75,000 daltons, about 100,000 daltons, about 150,000 daltons, about 200,000 daltons, about 250,000 daltons, about 300,000 daltons or about 400,000 daltons.

From the examples it is seem that employment of PEO 200,000 leads to a suitable composition.

Suitable pharmaceutically acceptable excipients are also described herein such as, e.g. inorganic acids, inorganic bases, inorganic salts, organic acids or bases and pharmaceutically acceptable salts thereof, saccharides, oligosaccharides, polysaccharides, and cellulose and cellulose derivatives. The organic acid may be a mono-, di-, oligo or polycarboxylic acid such as, e.g. acetic acid, succinic acid, citric acid, tartaric acid, acrylic acid, benzoic acid, malic acid, maleic acid, sorbic acid etc.

From the examples it is seem that employment of an organic acid like citric acid leads to a suitable composition.

Accordingly, in a specific embodiment the invention relates to a pharmaceutical composition in which the matrix composition comprises carvedilol, PEO 200,000 and citric acid.

**Multiple units composition**

The pharmaceutical composition according to the invention may furthermore be used in the preparation of a multiple units pharmaceutical composition, e.g. in the form of a capsule or tablet. A multiple units pharmaceutical composition is a composition, which comprises a multiplicity of individual units in such a form that the individual units will be made available upon disintegration of the composition, typically a capsule or tablet, in the stomach of humans or animals ingesting said composition. Thus, in this case, at least some of the individual units in said multiple units pharmaceutical composition will consist of the composition of the invention, the individual units being of a size, which allows them to be incorporated into such a composition.

**Preparation**

The delivery system as well as the first composition of the invention may be produced by various methods which are either known per se in the pharmaceutical industry or which, for example, are used in the production of polymer-based materials, depending upon the desired embodiment and the materials employed in the composition in question. As mentioned above, one advantage of the composition according to the invention is that it may be produced by methods, which are relatively simple and inexpensive.

A pharmaceutical composition may be produced by, for example, co-extrusion of the coating with the matrix composition and the active substance, extrusion and dip coating, injection moulding and dip coating, or by extrusion or injection moulding and solvent coating by spraying or dipping.

For further details reference is made to the experimental section herein.

**Other aspects of the invention**

It is a further advantage of the present invention that one formulation matrix can be used as the base for different strength of the pharmaceutical formulation because release pattern as well as erosion time can be maintained despite different load of the carvedilol in reverse to the amount of polymer as has been demonstrated herein (see batch EC-042-047, EC-042-77 and EC-042-78 in the Examples herein). This is highly desirable due to the considerable validation and running control of processes and analyses in the regulatory approval procedure and is very unusual in the field of controlled delivery technology where sophisticated techniques are very often used.

Furthermore, formulations having different strength together with an equal size is thereby easily obtained and different production equipment for each strength is avoided.

In case it is not desired even to adjust the load of a preferred specific formulation, different strength may still easily be obtained by simply alter the thickness of the shell. In the preferred embodiment of the invention where the product is injected moulded, the smaller strength can simply be produced with such thicker shells only by decreasing the diameter of the pin leaving room for the matrix when the shell material is injected into the moulding part of the tool. Thickening of the shell will not alter the release profile substantially as long as the exposed area is above a certain size depending on the erosion properties of the matrix and the design.

It should be noted that for active ingredients like carvedilol having linear pharmacokinetics, a zero order release from different formulations will result in dose linearity as long as the release duration is the same between the formulations. Accordingly, different strength having dose linearity may easily be obtained even with complete different formulations as long as each of the formulations are released with a zero order pattern and with the same release period.

The active ingredient carvedilol is at present marketed for different indications; hypertension, angina and congestive heart failure where the latter indication requires much lower strength than the former ones. Accordingly, carvedilol is administered in dosages within the range of 3.125 mg up to 100 mg per day. The initial dosage for congestive heart failure is 3.25 mg b.i.d. and patients with hypertension are treated with up to 50 mg per day administered in one or two dosages. High daily dosages of 100 mg are administered in several divided dosages during the day for treatment of angina.

A suitable selection of once daily formulation strengths to cover the need as outlined above would involve at least a span in strength of 8 fold (for example comprising a 6.25mg, 12.5mg, 25 mg and 50 mg formulation). However, due to the avoidance of peak plasma concentrations it is believed that daily dosages of 100 mg may not be necessary with the formulation according to the present invention. In addition, a once daily 37,5 mg formulation may be used instead of administration of 50 mg per day administered in one or two dosages of the immediate release product.

Accordingly, a further embodiment of the invention relates to series of pharmaceutical formulation products for the same or different medical indication wherein the individual formulations strength are prepared from the same pharmaceutical excipients.

In a more preferred embodiment, only the load of the active ingredient in reverse of one other excipient, such as the polymer is different between the different strength of the pharmaceutical series.

In an alternative embodiment, the ratio between one or more of the excipients, the polymer, or of the active ingredient is kept constant in the different strength of the pharmaceutical series.

In a still further embodiment, the span in strength from the lowest to the highest dosage of the series is at last 2 fold, such as at least 4 fold, such as 6 fold, more preferred 8 fold and still more preferred 16 fold.

It should be noted, that different colors may be added for patient compliance or marketing reasons to the different strengths of the pharmaceutical series. In such cases, the color component is not necessary to be regarded a pharmaceutical excipients.

**Method for controlling the release**

As mentioned above, the invention also relates to a method for controlling the release of a therapeutically, prophylactically and/or diagnostically active substance from a pharmaceutical composition. To this end all details and particulars described above under the composition aspect applies *mutatis mutandi* to the method aspect and other aspects of the invention.

The invention is further illustrated in the following figures and non-limiting examples.

Fig. 1       is a plug holder suitable for use when determining diffusion and dissolution rate. A stopper on the right seals the plug holder, and the swelling layer is formed on the left side on the plug.

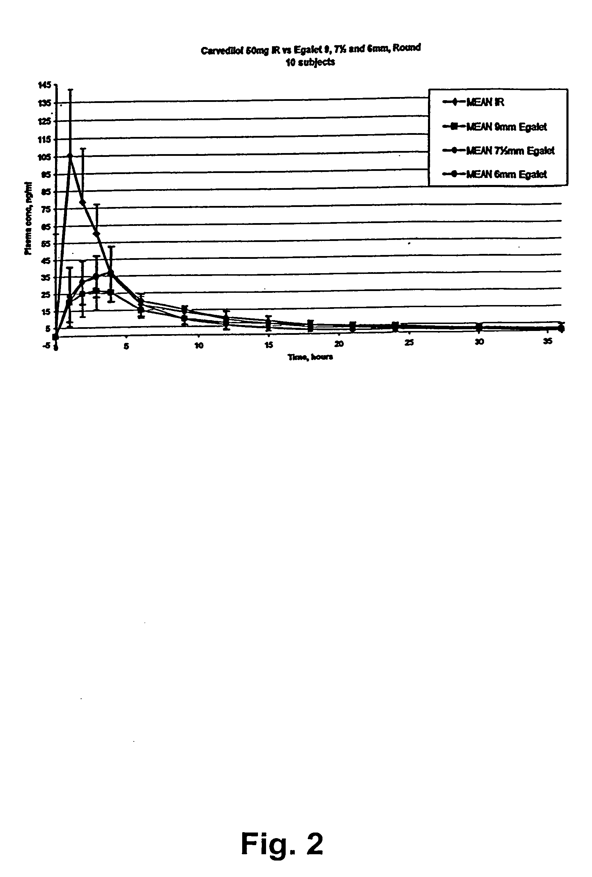

Fig. 2       is a dissolution curve from Example 6

Fig. 3       shows the DSC of carvedilol as starting material and a peak is observed corresponding to that carvedilol is employed in crystalline form.

Fig. 4-5     are the DSCs of PEO 200,000 and citric acid, respectively, and show that the substances are employed as crystals.

Fig. 6       shows that PEO + citric acid only has one peak indicating that citric acid is present on amorphous or dissolved form or possible in a different crystalline form.
            Carvedilol when admixed with PEO and citric acid maintain at least some of its crystallinity (no heating has taken place).

Fig. 7       shows DSC's of compositions according to the invention. No peak is present for carvedilol indicating the carvedilol is present in amorphous form. Storage of the compositions as mentioned above for about 1 month did not show any substantial difference in the DSC pattern.

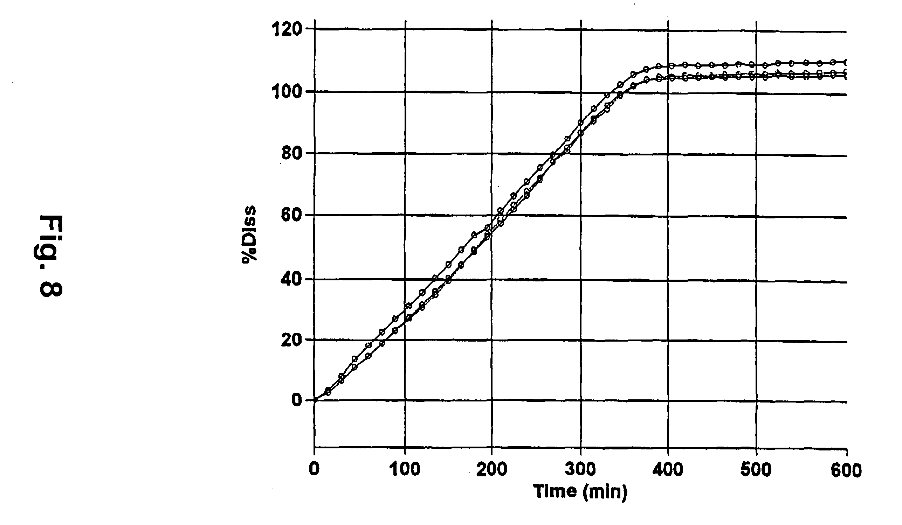

Fig. 8       shows the dissolution profile relating to a composition of Example 7 denoted 0069; the dissolution has been determined in FaSSIF medium (cf. Dressmann et al. J. Pharm. Sci. **11 Suppl. 2** (2000) pp S73-S80.

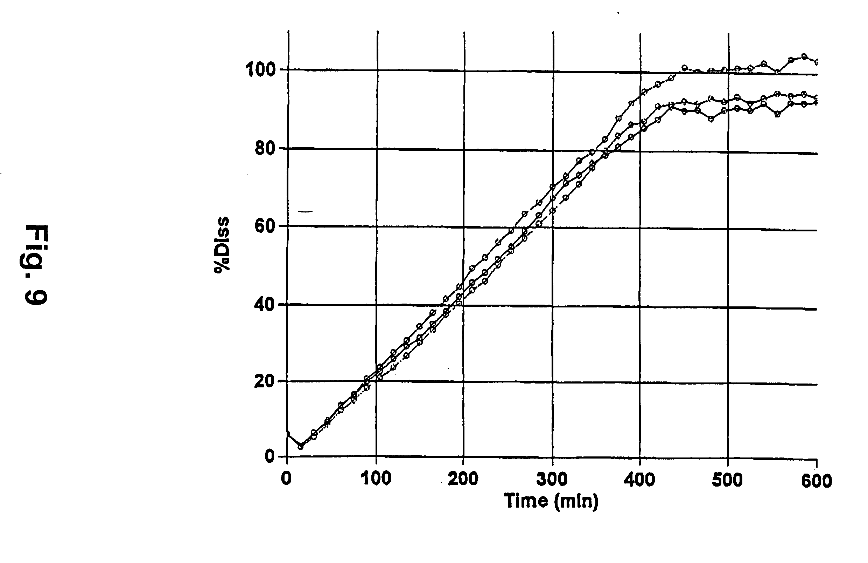

Fig. 9       shows the dissolution profile relating to a composition of Example 7 denoted 0069; the dissolution has been determined in FeSSIF medium (cf. Dressmann et al. J. Pharm. Sci. **11 Suppl. 2** (2000) pp S73-S80.

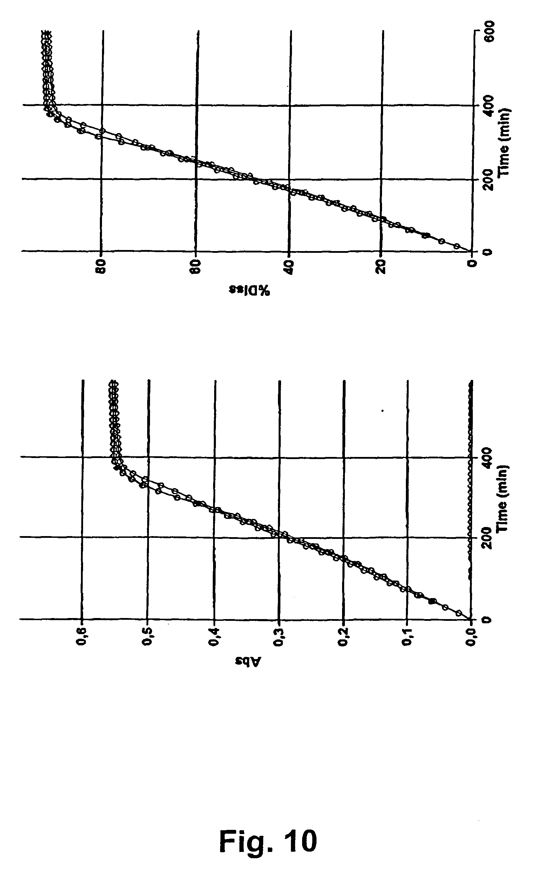

Fig. 10      shows the dissolution profile relating to a composition of Example 7 denoted 0069; the dissolution has been determined after 26 days storage at 30 °C and 60% RH.

**Methods**

**Diffusion/ Dissolution studies**

*Method for determination of dissolution rate of the matrix*

A composition according to the invention has properties that ensure that the diffusion rate of water into the polymer matrix substantially corresponds to the dissolution rate of the polymer matrix composition into the aqueous medium. In the following is given a simple method to test these conditions.

The polymers that are suitable for use according to the present invention and which are sufficiently hydrophilic are water-soluble. When contacted with water, a sharp advancing waterfront divides the intact and not penetrated matrix from a swollen front. Under stationary conditions, a constant thickness surface layer is formed by the swollen polymer and by a high concentration of polymer in solution.

In fact, once the hydrodynamic external conditions are defined, a stationary state is reached where the rate of penetration of the moving boundary equals the rate of removal of the polymer at the external surface.

The time lapse until the quasi-stationary state is reached is called swelling time. At steady state, the dissolution rate is constant and can be defined equally by either the velocity of the retracting front of the polymer or the velocity of the front separating the pure penetrate and the liquid dissolving sublayer. Thus, both fronts are synchronized.

When the dissolution rate equals the penetration rate (i.e. the diffusion rate) a constant thickness surface layer should be observed. The dissolving layer evolution during water conditioning should reflect the different dissolution characteristics of the materials employed. The surface layer thickness is measured as a function of time.

In order to measure the diffusion rates of water, samples may be prepared in the form of plugs fitting to the sample holder (e.g. 2 mm, 4 mm, 6 mm, 7.5 mm and 12 mm long and preferable with the same shape and volume as the desired dosage unit). The sample holder is prepared by translucent glass in a tubular shape and with noticeable marks indicated with a specific distance.

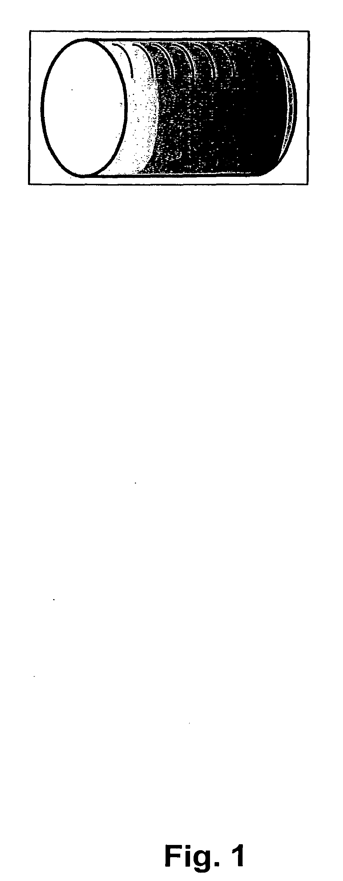

The test proceeds as follows: Place 1 plug incorporated into the glass tube in a vessel - optionally with a water soluble dye (e.g. $Cu^{2+}$) - and the plug/glass tube is placed in a dissolution apparatus e.g. according to monograph: USP 24, page1941-1950, which is hereby incorporated by reference (see Fig. 1). By employment of the USP method it is possible to determine the diffusion rate as well as the dissolution rate in the same experiment. The copper ions are blue-coloured so they are visually detectable and due to the metric scale on the tube, the diffusion rate can be calculated (unit is length/time). The dissolution rate is determined by determining the amount of substance (e.g. active substance) released and at the same time determining the length of the matrix composition that has been eroded. Thus, the dissolution rate is also in length/time units. As the dissolution profile easily can be obtained from the data measured, a simple means for the determination of whether the release follows zero order is to investigate the dissolution profile and see whether linearity is present.

Agitation is provided, and the length of the front of matrix is measured at desired time intervals as a function of time. The measurement may be a simple visual identification of the marks on the glass tube.

When the dissolution rate equals the penetration rate a constant thickness surface layer is observed. The different dissolving layers in different matrices obtained during the water contact, reflect the different dissolution characteristics of the matrix. The thickness of the surface layer as a function of time is then compared. The specific aqueous medium may be selected individually.

Dissolution test

Dissolution tests were performed in accordance with the USP 24, NF 19, (711) Dissolution, Apparatus 2 equipped with a paddle. The dissolution medium was 0.1 N hydrochloric acid during the first 120 min, which was then substituted with a buffer solution pH 6.8. The volume of the dissolution medium was 1000 ml and the rotation speed of the paddle was 120 rpm during the first 120 min and then 50 rpm. Samples were withdrawn at suitable time intervals and analysed for content of carvedilol by means of UV spectrometry at a wavelength of 284 nm.

**Examples**

**A general method for the preparation of a controlled release composition is described below.**

Preparation of the matrix composition

An accurate amount of the polymer (i.e. in the examples below: the polyethylene oxide) is loaded into a MTI mixer followed by an accurate amount of the active substance and of the pharmaceutically acceptable excipients(s), if any. The mixing is performed at 2050/1450 rpm and at a time period of 10 min + 4 min + short final spinning. At the start of the mixing the temperature is about 19 °C (the first time period) and the final temperature of the mixture is about 52 °C (the second and third time period). The mixture is then allowed to cool to room temperature and is ready to be fed into an injection moulding machine.

**Preparation of the coating composition**

The coating composition was prepared by first adding the hydroxymethylcellulose then cetostearyl alcohol, and finally the titanium dioxide to an MTI-Mixer at a temperature about 21 °C. After mixing for nearly 9 min at 1000 rpm (I: 0.9 A) the mixer was stopped (temperature about 46°C) and the adhered material manually incorporated into the mixture. The mixture was left to cool for about 10 minutes. The mixing is then finalized with a short high-speed mix in order to minimize lumps formation. The mixture was then allowed to cool to room temperature, after which it had a suitable consistency for being fed into an injection moulding machine.

Example of coat composition

Batch: 58-014-01-013

| % | Batch | Material | amount (g) | Weight (g) | step |
|---|---|---|---|---|---|
| 79 | 991207-A | ***Ethocel*** | 632 | 632 | 1 |
| 20 | 990426-B | Cetylstearyl Alkohol | 160 | 160.1 | 2 |
| 1 | 97051301 | $TiO_2$ | 8 | 8.0 | 3 |
| 100 | | total | 800 | 800.1 | |

The final dosage units may be prepared according to two different methods.
In one method, the coat and the matrix moulded individually followed by a manually incorporation of the moulded matrix plug into the moulded coat. The moulding machine used is an Arburg Allrounder 220 S 250/60.
In the second method, the coat and matrix are moulded in one process where the coat is moulded in a first step and the matrix is moulded directly into the coat in a second step. The moulding machine used is Arburg Allrounder 420 V 800-60/35.
The following table describes formulations having a cylindrical form and circular openings in both ends.

| Batch | Length [mm] | Diameter [mm] | Vol [mm$^3$] |
|---|---|---|---|
| 01-0034-042 | 7,5 | 5,05 | 150 |
| 01-0035-042 | 6,0 | 5,64 | 150 |
| 01-0043-042 | 9,0 | 4,6 | 150 |

The following table describes formulations having a cylindrical form and oval openings in both ends

| Batch | Length [mm] | Vol [mm$^3$] | Longest/shortest diameter [mm] | |
|---|---|---|---|---|
| 01-0075-042 | 6.0 | 150 | 8.74 | 3.64 |
| 1-0076-042 | 7.5 | 150 | 7.82 | 3.21 |

The coated compositions obtained were open at two opposite ends.
The area for an open end is calculates as the volume/length of the cylindrical formulations.

**Example 1**

**Preparation of a pharmaceutical composition comprising carvedilol as an active substance**

A composition (plug batch No. 01-0045-042), formulation batch No. 01-0034 042 according to the invention was prepared from the following ingredients:

| No | Raw materials | Reference: |
|---|---|---|
| 1 | PEO 200,000 | S-Ega40200; USP24-NF19 2000 p. 2497 |
| 2 | Carvedilol | Ph.Eur. 3rd Ed. 2000 p.359 |
| 3 | Citric Acid | Ph.Eur. 3rd Ed. 1997 p.645 |

| Matrix | % w/w |
|---|---|
| Polyethylene oxide | 64.6 |
| Carvedilol (Cipla) | 30 |

(continued)

| Matrix | % w/w |
|---|---|
| Citric acid | 5.4 |

The coating and the matrix were prepared as described above. One dosage form contains 50 mg carvedilol. The composition was 7.5 mm long.

The composition was subjected to the dissolution test described above. The following results were obtained:

| Time (h) | dissolved carvedilol (% w/w of the coated composition) |
|---|---|
| 0 | 0 |
| 1 | 14.1 |
| 2 | 27.1 |
| 3 | 39.3 |
| 4 | 49.9 |
| 5 | 60.7 |
| 6 | 72.5 |
| 7 | 85.0 |
| 8 | 99.7 |

The dissolution profile corresponds to a zero order release of carvedilol from the composition.

## Example 2

### Preparation of an oval shaped pharmaceutical composition comprising carvedilol as an active substance

A composition (batch No. 01-0076-042) according to the invention was prepared from the following ingredients:

| Matrix | % w/w |
|---|---|
| Polyethylene oxide | 64.6 |
| Carvedilol (Cipla) | 30 |
| Citric acid | 5.4 |

The coating and the matrix were prepared as described above. One dosis form contains 50 mg carvedilol. The composition was 7.5 mm long and had an oval cross sectional shape.

The composition was subjected to the dissolution test described above. The following results were obtained:

| Time (h) | dissolved carvedilol (% w/w of the coated composition) |
|---|---|
| 0 | 0 |
| 1 | 15.9 |
| 2 | 30.1 |
| 3 | 46.2 |
| 4 | 62.2 |
| 5 | 77.61 |
| 6 | 92.4 |

The dissolution profile corresponds to a zero order release of carvedilol from the composition.

## Example 3

### Preparation of a pharmaceutical composition comprising carvedilol as an active substance

A composition (plug batch No. 01-0044-042, dosage unit batch No. 01-0043 042) according to the invention was prepared from the following ingredients:

| Matrix | % w/w |
|---|---|
| Polyethylene oxide | 64.6 |
| Carvedilol (Cipla) | 30 |
| Citric acid | 5.4 |

The coating and the matrix were prepared as described above. One dosage form contains 50 mg carvedilol. The composition was 9 mm long.

The composition was subjected to the dissolution test described above. The following results were obtained:

| Time (h) | dissolved carvedilol (% w/w of the coated composition) |
|---|---|
| 0 | 0 |
| 1 | 13.2 |
| 2 | 22.5 |
| 3 | 33.2 |
| 4 | 44.7 |
| 5 | 56.2 |
| 6 | 67.0 |
| 7 | 77.2 |
| 8 | 88.1 |
| 9 | 98.6 |

The dissolution profile corresponds to a zero order release of carvedilol from the composition.

**Example 4**

**Preparation of a pharmaceutical composition comprising carvedilol as an active substance**

A composition (batch No. 01-0075-042) according to the invention was prepared from the following ingredients:

| Matrix | % w/w |
|---|---|
| Polyethylene oxide | 64.6 |
| Carvedilol (Cipla) | 30 |
| Citric acid | 5.4 |

The coating and the matrix were prepared as described above. One dosage unit form contains 50 mg carvedilol. The composition was 6 mm long and had an oval shape.

The composition was subjected to the dissolution test described above. The following results were obtained:

| Time (h) | dissolved carvedilol (% w/w of the coated composition) |
|---|---|
| 0 | 0 |
| 1 | 17.0 |
| 2 | 35.1 |
| 3 | 55.1 |
| 4 | 74.7 |
| 5 | 93.8 |

The dissolution profile corresponds to a zero order release of carvedilol from the composition.

**Example 4 A**

**Preparation of a pharmaceutical composition comprising carvedilol as an active substance**

A composition (batch No. EC-042-211) according to the invention was prepared from the following ingredients:

| Matrix | % w/w |
|---|---|
| Polyethylene oxide | 86 |
| Carvedilol (Cipla) | 14 |

The coating and the matrix were prepared as described above. One dosage unit form contains 25 mg carvedilol. The composition was 12 mm long and had circular end surfaces.

The composition was subjected to the dissolution test described above. The following results were obtained:

Dissolved carvedilol (% w/w of the coated composition) from hour 1 to 15 hours

| Time (h) | dissolved carvedilol (% w/w of the coated composition) |
|---|---|
| 1 | 12.4 |
| 2 | 21.6 |
| 3 | 29.2 |
| 4 | 35.4 |
| 5 | 40.0 |
| 6 | 44.5 |
| 7 | 49.4 |
| 8 | 54.3 |
| 9 | 59.4 |
| 10 | 64.6 |
| 11 | 70.6 |
| 12 | 75.5 |
| 13 | 79.8 |
| 14 | 84.1 |
| 15 | 88.7 |
| 16 | 92.6 |
| 17 | 94.6 |

The dissolution profile corresponds to a zero order release of carvedilol from the composition.

**Example 5**

**Pilot phase I studies in health volunteers employing carvedilol compositions according to the invention**

Carvedilol has emerged as one of the important and promising drugs for cardiovascular diseases including hypertension and congestive heart failure, and results in a noticeable improvement of survival rates in patients with chronic cardiac insufficiency. To further optimize the treatment, Carvedilol Egalet® has been developed as a once daily composition.

Carvedilol is currently marketed as an immediate release formulation only in 3,125mg, 6,25mg, 12,5mg, 25mg and 50mg tablets. Only the 6,25mg and 25mg application form is available throughout the EU whilst of the other strengths some are missing in certain member states. A 25mg immediate release application form may be used as a reference.

Carvedilol is registered for the following indications:

Hypertension

Chronic cardiac insufficiency

Angina pectoris

Carvedilol Egalet® is developed for long-term treatment of hypertension and is therefore developed for a maintenance dosage. However, the present invention encompasses other dosages where a controlled delivery is desired.
The expected advantages offered by the Carvedilol Egalet® compared to the immediate release formulation include:

i) Reduced standard deviation and thus, a more predictable concentration in plasma.

ii) A dose regimen with lower frequency of administration and thereby potentially improvement of patient compliance.

For patients with cardiac insufficiency, it is recommended to take Carvedilol with a meal to delay absorption and thereby avoid adverse reactions. Carvedilol Egalet® offers the advantage of reduced $C_{max}$, even if taken fasting. (Latest studies CL-EG-pilot-1 and CL-EG-pilot-02 shows that $C_{max}$ is only slightly as high as for 25 mg Carvedilol IR).
Patients with hypertension have a well-described low compliance, presumably because there are no recognizable symptoms connected with the condition. Compliance with a once-daily regimen is higher and therefore offers a therapeutic advantage. Recommendations for the use of Carvedilol vary between countries.
An evaluation of Carvedilol in "Drugs" from 1997 lists in the summary under Dosage and Administration "A dosage of Carvedilol 12.5 mg once daily for 2 days, increased to 25 mg daily thereafter and increased to 50 mg once daily after 2 weeks if necessary, is recommended for patients with mild to moderate hypertension".
According to the American Physician's Desk Reference (PDR) 2000, Carvedilol should be prescribed twice daily for all indications.
According to the German Drug Listing (Rote Liste 2001 for Dilatrend®), Carvedilol should be prescribed twice daily for cardiac insufficiency and angina pectoris, and once to twice daily for hypertension with a maximum dose of 2 x 25 mg.
According to "Drugs, Fact and Comparison", Carvedilol is prescribed twice daily for hypertension. In all countries, the maximum daily dose is 25 mg b.i.d., and it is against this dose and frequency that Carvedilol Egalet is tested herein.

*Composition of Carvedilol Egalet*

In the development work on Carvedilol Egalet®, different compositions of matrix have been tested, i.e. the load of drug has been varied.
In Table 1 below is given the final composition of the coated composition used in the pilot studies. The individual composition employed in Pilot tests III, Iv and V corresponds to the compositions of Examples 1-4.

Table 1. Composition of Carvedilol Egalet®

| Ingredients | Percentage | Function | Reference to standards |
|---|---|---|---|
| **Active substance** | | | |
| Carvedilol | 32% | Active compound | Cipla |
| **Excipients** | | | |
| Citric Acid | 5% | Matrix | Ph. Eur. |
| Polyethylene Oxide (PEO 200000) | 63% | Matrix | USP |
| Ethylcellulose | 79% | Shell | Ph. Eur |
| Ceto-stearyl alcohol | 19,8% | Shell | Ph. Eur |
| Titanium dioxide | 1% | Shell | Ph. Eur |
| Ferro Oxide | 0,2% | Colouring | USP |

**Pharmacodynamics**

There are several pharmacodynamics issues to be described for the Carvedilol Egalet[®]. The following is a list of issues and the considerations regarding their testing.

01. Bioavailability

a) Rate and extent of absorption

b) Fluctuations in drug concentration

c) Variability arising from the formulation

d) Dose proportionality

e) Risk of unexpected release characteristics

2. Factors influencing the performance of a modified drug formulation

f) GI function

g) Diurnal rhythm

3. Stereoisomers

Ad a-c Absorption, fluctuations and variability:

These characteristics are described by the pharmacokinetic studies already conducted and will be further confirmed in studies planned.

a. Absorption

There is no literature on slow release formulations of Carvedilol. One study has been identified on in-vivo absorption of Carvedilol formulated in timed-release capsules. This study by Nolte et al found absorption throughout the GI tract, correlating with the absolute absorption areas of the different parts of the intestinal tract. They found a relatively high absorption of Carvedilol in the large intestine, amounting to approximately 10% of the total absorption.
This supports the findings from the pilot studies performed on the Carvedilol Egalet[®], where the plasma curves show that Carvedilol is being absorbed throughout the GI tract, including the colon, and that the absorption in the colon is present, but considerably reduced compared to earlier in the GI tract. b.

Fluctuations

To evaluate fluctuations in plasma concentration, comparison should be made between plasma profiles from the same concentration given. Data from pilot study IV and V on Carvedilol IR 50mg compared to Carvedilol Egalet[®] 50mg shows that Cmax for the Carvedilol Egalet[®] is reduced approximately 50%, whereas the C24h, which will correspond to Cmin in a once daily dosing, is 2,5 times as high.
In the studies, the Carvedilol IR has been given in a single dose. Patients will be taking Carvedilol IR b.i.d., wherefore peak/trough ratio should be measured for this dose regimen. This will be done in the steady state studies.

c. Variability

In published literature the variability of Carvedilol is very high, with standard deviations of >50%. The study with the highest number of subjects, i.e. 44 shows a SD of 70%.
There are no indications that the variability will be higher than for the immediate release formulation.

d. Dose proportionality:

At this point it is only planned to market one dose of Carvedilol Egalet[®], and no investigations into dose proportionality are planned. Literature describes dose linearity for Carvedilol in the range of 6,25-50mg.

e. Dose dumping:

Carvedilol is being released from the Egalet tablet by the erosion of the matrix from the exposed surfaces only as the coat prevents contact to the aqueous medium of the intestines. Accordingly, release of all of the Carvedilol at one time is not possible.
A further advantage of the injection moulding of shell and matrix in one process step is that the shell and the matrix reach a high degree of adherence.
An uncoated and thus unprotected matrix has been investigated through dissolution tests, which show that release time in-vitro for a freely exposed Carvedilol matrix from a 9 mm Egalet® is about two hours. Accordingly, the coating actively prevents release due to the limited exposed area.
In addition, the in-vivo-in-vitro correlation of the Egalet® has been described to some extend through scintigraphy. 2 hours in vitro release in the stomach will correspond to at least 3 hours in vivo, and will not be below 2 hours. Thus any dose dumping would be of less severity than seen after intake of 50 mg conventional immediate release tablet.
The immediate release tablet has been investigated as 50 mg o.d. in several clinical studies, but is associated to an increased number of adverse events, compared to lower doses, due to the increased trough-peak ratio.

f. GI function:

GI transit time may influence the release rate. A very fast transit time where the tablet is excreted before the content is fully released, will result in a decreased AUC. This is a well-described issue for slow release products.
For the Carvedilol Egalet® the effect of GI transit time can be clearly demonstrated because the non-degradable shell can be collected. When all Carvedilol is released with normal transit times, remaining Carvedilol can be found in the shells with decreased transit time. This has been documented in findings from pilot study III.
As release rate is constant for any given formulation, release time is depending on the length of the Egalet® tablet. For the matrix formulation described in Table 1, which releases at the rate of 1 mm/hour in *in-vitro* dissolution, tablets of 9mm have shown complete release with normal transit time.
The absorption of Carvedilol Egalet® in patients with Morbus Crohn and Collitis ulcerosa has not been investigated. Until that is performed, the use of the product in the patient population is contraindicated.
The effect of food will be evaluated in a traditional PK study. Preliminary information on food effect will be obtained in a pilot study on 10 volunteers.

g. Diurnal rhythms

Carvedilol has been shown to preserve the diurnal rhythm of blood pressure; there are no reasons to believe that a slow release formulation will influence this rhythm differently than the IR formulation. This will be explored in the phase II study, where ambulatory BP will be measured for 24 hours.

3. Stereoisomers

Carvedilol is a racemic mixture of R(+) and S(-) -enantiomers: S(-), which is a potent $\beta1$ and $\beta2$ antagonist and $\alpha$-adrenoceptor antagonist and R(+), which has 1/100 of the beta effect and the same $\alpha$ effect as the S(-). The pharmacokinetics of these is described both in healthy volunteers and in patients with cardiac insufficiency.
Theoretically, the plasma profiles of the enantiomers seen after intake of the Carvedilol Egalet® could be different from the one seen after Carvedilol immediate release, given that the t½ of the two are different (9.6 h for R(+) and 22.1 h for S(-)). In steady state, however, the plasma profiles are similar to that of Carvedilol, and it is not expected that the blood pressure lowering effect will be different for the Carvedilol Egalet® than for the Carvedilol IR.

**Pharmacokinetics**

The development of Carvedilol Egalet® has involved investigational pilot studies on healthy volunteers. No full-scale studies have been performed up to now.
Different formulations of Carvedilol Egalet® have been tested and through this work the final formulation has been identified, and a strategy was planned for the clinical testing programme.

**Pilot Phase I studies**

**Completed pharmacokinetic studies**

The pharmacokinetic studies on Carvedilol Egalet® listed in Table 1 are part of the development work to obtain preliminary information on the pharmacokinetics.

**Table 1 Completed pilot pharmacokinetic studies, listed chronologically**

| Study No. | Design | Treatment | Doses (mg)[a] | No. of subjects |
|---|---|---|---|---|
| Pilot test I | *Single-dose PK:* Open-label, 2-armed, parallel group (Carvedilol Egalet® vs. Carvedilol IR) | Single-dose | C Egalet: 25mg  C IR: 25mg | 6 |
| Pilot test II | *Single-dose PK:* Open-label, 2-armed, parallel group (Carvedilol Egalet® vs. Carvedilol IR) | Single-dose | C Egalet: 50mg  C IR: 50mg | 6 |
| Pre-Pilot I | *Single-dose PK:* Open-label, Carvedilol Egalet® | Single-dose | C Egalet: 50mg | 2 |
| Pre-Pilot II | *Single-dose PK:* Open-label, Carvedilol Egalet® | Single-dose | C Egalet: 50mg | 2 |
| Pre-Pilot III | *Single-dose PK:* Open-label, Carvedilol Egalet® | Single-dose | C Egalet: 37,5mg | 2 |
| Pre-Pilot IV | *Single-dose:* Collection of excreted shells | Single-dose | C Egalet: 50mg | 2 |
| Pilot test III | *Single-dose PK:* Open-label, 4-way cross-over study (3 doses Carvedilol Egalet® vs. Carvedilol IR) | Single-dose | C Egalet: 25mg/37,5mg / 50mg o.d. C IR: 37,5mg b.i.d. | 6 |
| Pilot Test IV | *Single-dose PK:* Open-label, 4-way cross-over (3 different shapes of Carvedilol Egalet® vs. Carvedilol IR) | Single-dose | C Egalet: 50mg o.d. C IR: 50mg o.d. | 10 |
| Pilot Test V | *Single-dose PK:* Open-label, 4-way cross-over (2 different shapes of Carvedilol Egalet® vs. Carvedilol IR) As a final fixed sequence arm; the chosen shape of Carvedilol Egalet® in fed subjects | Single-dose | C Egalet: 50mg o.d. C IR: 50mg o.d. | 10 |

In all studies, the investigational products were administered orally as tablets.
The formulations tested in these studies showed a prolonged release of Carvedilol with reduced $C_{max}$ and measurable plasma concentrations over 36 hours.

**Results and Discussion- Pilot pharmacokinetic Studies**

The pilot phase I studies completed up to now clearly indicates that it is possible to produce a slow release Carvedilol Egalet® with a PK profile required of a once daily formulation.

In pilot test III, the influence of the length of the Egalet® tablet on the release characteristics was described. In pilot test IV, Egalet® tablets with 3 different diameters and lengths has been tested. In vitro dissolution tests indicated that an increased diameter would not influence the speed of erosion and pilot IV and V has confirmed this. $C_{max}$ is increasing proportionally to the increasing surface area exposed of the Egalet. Tmax does not differ between the formulations. The mean of the plasma concentrations measured for the 6mm Carvedilol Egalet® 50mg is reduced due to an unexpected high number of subjects having a fast transit time in that treatment group; 6 of 10 subjects excreted the Egalet before hour 24.

In pilot study V, two of the same lengths of Carvedilol Egalet® as in pilot test IV were tested, but in a different oval shape, and compared to Carvedilol immediate release. Preliminary data assessment supports the conclusion from pilot study IV that Cmax increases with increasing diameter of the Egalet®. When comparing data for the round Egalet in pilot IV to the "easy-to-swallow" oval shaped Egalet in pilot V, for the 6 mm and the 7½ mm lengths respectively, and the exposed matrix area being constant, there are no observed difference by the change of shape. To obtain preliminary information on the effect of food on Carvedilol Egalet®, the 6 mm Egalet formulation was tested after a standard, high-fat meal, according to guidelines. The first 3 arms of the study were randomised and the last, the fed, was a fixed sequence arm. The data results from the last sequence have not been received yet and full data analysis for pilot study V has thus not been completed.

The composition, the Carvedilol Egalet® 6 mm, is a composition, for which we aim at showing an AUC equivalent to the marketed twice-daily formulation. Preliminary data assessment from pilot study V shows for the 6mm oval Egalet an AUC of 97,7% of the Carvedilol IR in fasting subjects.

| Formulation | n | AUC (0-36h) h*ng/ml | rel. AUC % | C max ng/ml | C (12h) ng/ml | C (24h) ng/ml | t max hours |
|---|---|---|---|---|---|---|---|
| **CL-EG-01 (round egalet)** | | | | | | | |
| **9mm** | 10 | 285 | 70 | 26,7 | 7,1 | 4,2 | 3 |
| **7½mm** | 10 | 355 | 88 | 37,8 | 10,3 | 3,8 | 4 |
| **6mm** | 10 | 336 | 76 | 37,4 | 9,1 | 2,9 | 4 |
| **IR** | 10 | 433 | 100 | 105,5 | 5,8 | 1,9 | 1 |
| **CL-EG-02 (oval egalet)** | | | | | | | |
| **IR** | 10 | 444 | 100 | 95,8 | 6,6 | 2,4 | 1 |
| **7½mm** | 10 | 344 | 76 | 33,2 | 9,0 | 5,0 | 4 |
| **6mm** | 10 | 421 | 97 | 41,7 | 11,8 | 4,8 | 4 |
| **6mm + food** | 10 | 362 | 80 | 39,0 | 10,9 | 3,9 | 3 |

In the table is given relevant pharmacokinetic parameters from the pilot studies (see Fig 1).

**Example 6**

**Preparation of a composition of carvedilol - DSC measurements**

A composition according to the invention was made from the following:

| | |
|---|---|
| PEO 200,000 | 67% w/w |
| Carvedilol | 28% w/w |
| Citric acid | 5% w/w |

The composition was made according to the general process described herein.

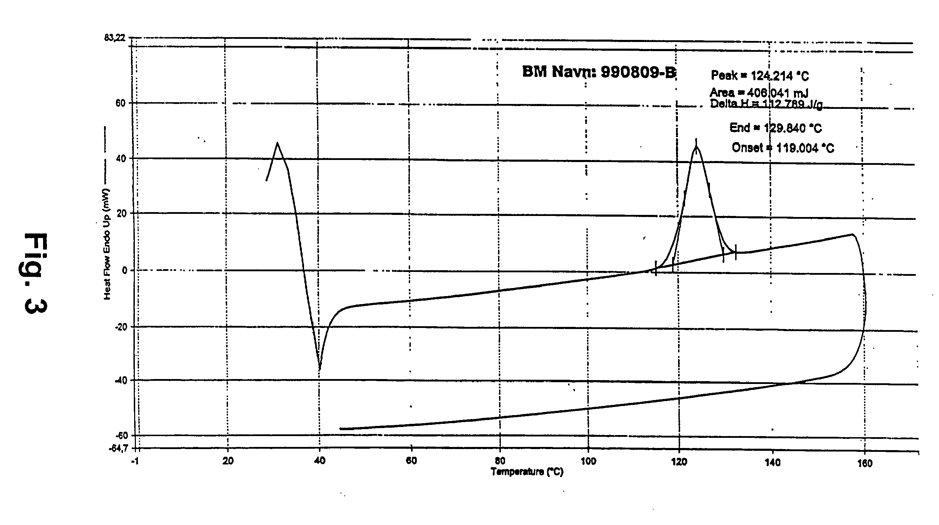

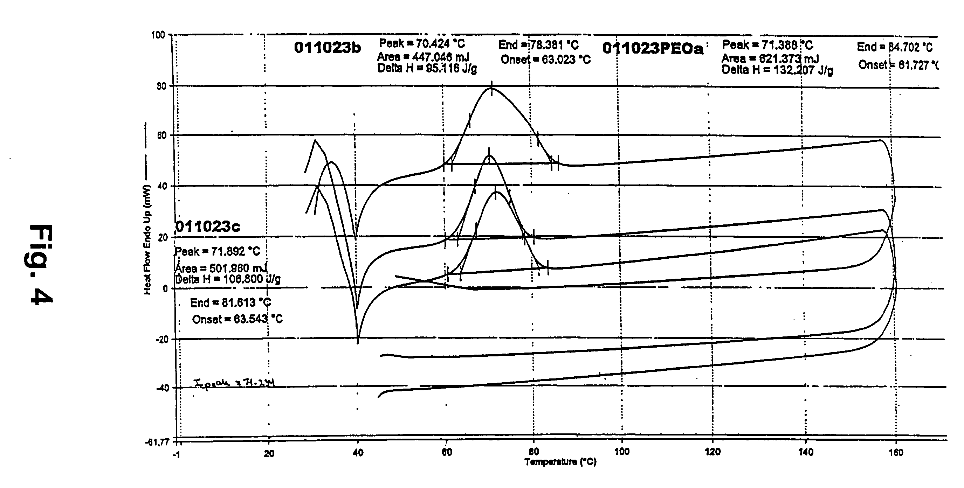

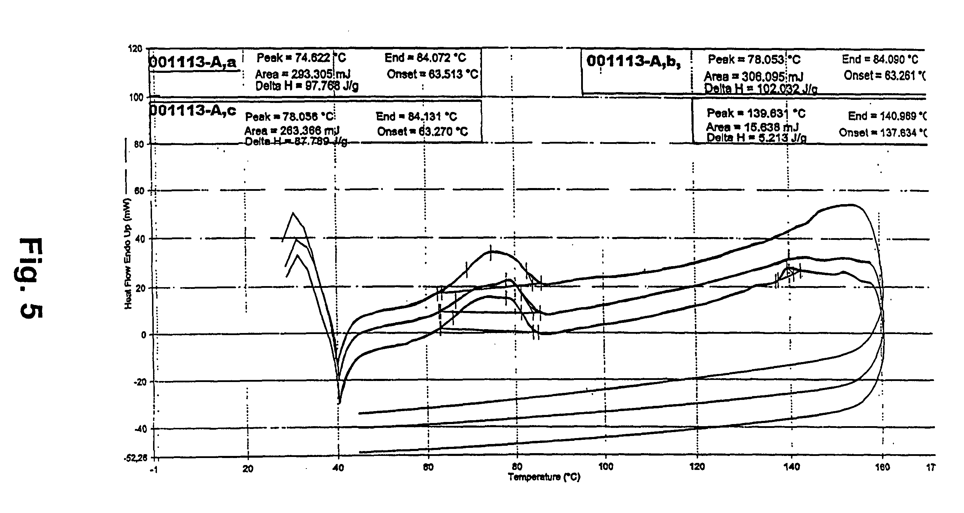

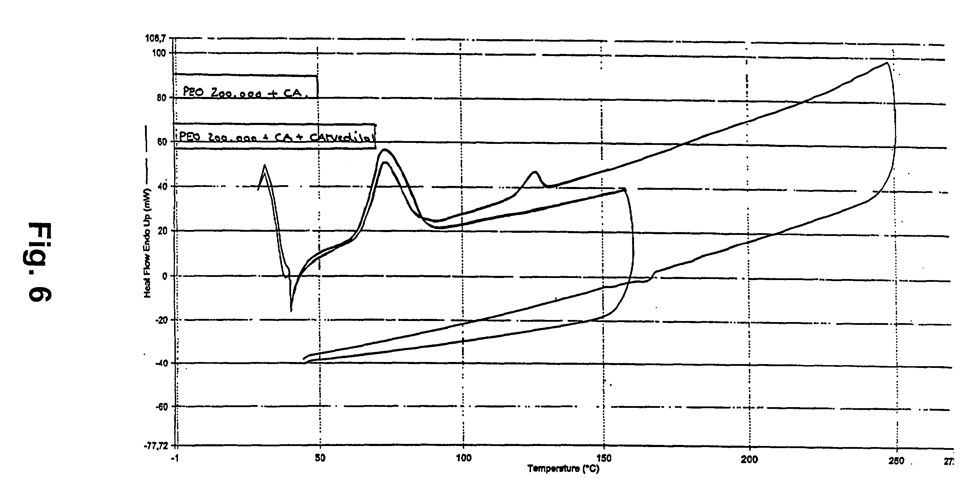

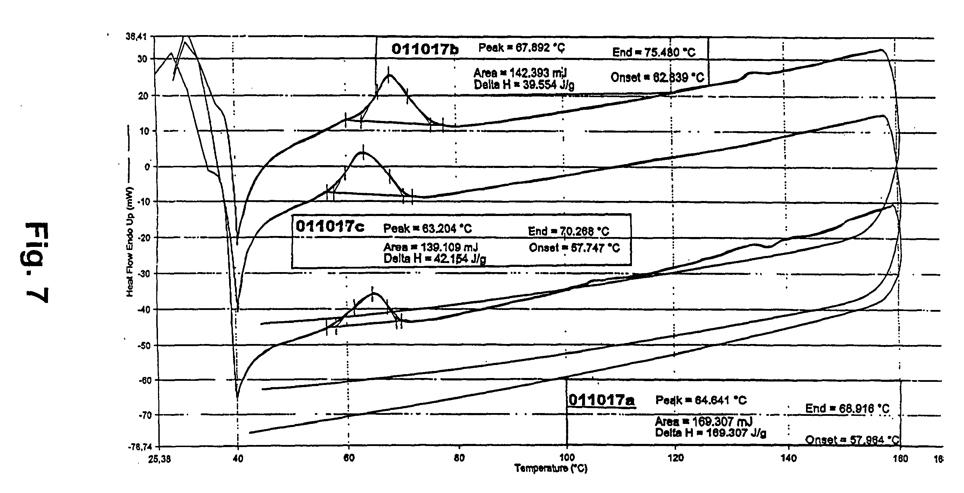

All starting materials as well as a mixture of PEO 200,000 and citric acid was subject to differential scanning caliometry measurements (thermal measurement). The final composition was also investigated at time 0 and 1 month after storage at 25 °C/60% RH and 40 °C/70% RH. The results are shown in figs. 3-7.

**Example 7**

**Compositions according to the invention**

This example illustrates the invention and gives a number of different compositions according to the invention. In the right hand column is given comments to the individual compositions with respect to the impact on the composition of the ingredients employed and with respect to the dissolution profile obtained.

Abbreviations:

| PEG: | polyethylene glycol |
|---|---|
| PEG ms: | polyethylene glycol monostearat |
| HPMCP HP 50: | hydroxypropyl methylcellulose pthalate (HP 50 is grade) |
| TPGS: | $\alpha$-tocopheryl polyethylene glycol succinate |

Polymer system

| | Matrix | | Desired Release time 12 hours in 12 mm long tubular tablet. Result |
|---|---|---|---|
| | Ingredient | % w/w | |
| EC-042-011 25 mg Carvedilol | PEO 200 000 Carvedilol | 86 14 | No zero-order release in acid medium, release time after 17 h. |
| EC-042-013 25 mg Carvedilol | PEO 200 000 Carvedilol Lactose Klucel PEG 2000 ms | 50 14 24 5 7 | No zero-order release, release time after 14h. |
| EC-042-014 25 mg Carvedilol | PEO 200 000 Carvedilol PEG 2000 ms | 81 14 5 | Released after 14 h. After 1 month (18-22°C), no release in buffer. |
| EC-042-015 25 mg Carvedilol | PEO 200 000 Carvedilol HPMCP HP 50 | 81 14 5 | Release time after more than 20 h. Matrix left in the shell. |
| EC-042-016 25 mg Carvedilol | PEO 200 000 Carvedilol PEG 2000 ms HPMCP HP 50 | 76 14 5 5 | Release time after 16 h. Matrix left in the shell. |
| EC-042-020 25 mg Carvedilol | PEO 200 000 Carvedilol PEG 2000 ms | 81 14 5 | Released after 12 h. Almost zero-order. |
| EC-042-024 25 mg Carvedilol | PEO 200 000 Carvedilol PEG 2000 ms | 70 14 16 | Released after 11h. After 2-3 month storage (18°C-22 °C), no release in buffer. |

(continued)

| | Matrix | | Desired Release time 12 hours in 12 mm long tubular tablet. Result |
|---|---|---|---|
| | Ingredient | % w/w | |
| EC-042-025 25 mg Carvedilol | PEO 200 000 Carvedilol PEG 2000 ms Hydroxyethyl cellulose (Natrosol) HPMCP HP 50 | 65 14 14 4 3 | Release time increased but HPMCP did not lower the release rate in acid medium. Release time after 15 h. No zero-order release. |
| EC-042-030 50 mg Carvedilol | PEO 200 000 Carvedilol PEG 2000 ms | 52 32 16 | No zero order release. |
| EC-042-031 50 mg Carvedilol | PEO 600 000 Carvedilol PEG 2000 ms | 52 32 16 | No zero order release. |
| EC-042-034 25 mg Carvedilol | PEO 45.000 PEG 2000 ms Carvedilol | 70 14 16 | No zero-order release, the matrix swell. |
| EC-042-037 50 mg Carvedilol | PEO 200.000 Carvedilol PEG 2000 ms | 52 32 16 | No zero-order release. Release time 14-16h. Carvedilol precipitated in buffer medium. |
| EC-042-039 50 mg Carvedilol | PEO 200.000 Carvedilol PEG 2000 ms Starch | 52 32 6 10 | No zero order release, release time > 25 h. Starch increased the release time |
| EC-042-042 50 mg Carvedilol | PEO 200.000 Carvedilol PEG 2000 ms Carbomer 974 | 43 32 10 15 | Too low release rate, Carbomer 974 dereased the release time |
| EC-042-043 50 mg Carvedilol | PEO 200.000 Carvedilol PEG 2000 ms Carbomer 974 | 47 32 16 5 | Too low release rate, Carbomer 974 dereased the release time |
| EC-042-044 50 mg Carvedilol | PEO 200.000 Carvedilol PEG 2000 ms Carbomer 974 | 55 32 10 3 | Too low release. Carbomer decreased the release. |
| EC-042-048 50 mg Carvedilol | PEO 200.000 Carvedilol | 68 32 | No release in buffer medium. Precipitation of carvedilol |
| EC-042-051 50 mg Carvedilol | PEO 200.000 Carvedilol | 68 32 | No release in buffer medium. Precipitation of carvedilol |
| EC-042-052 25 mg Carvedilol | PEO 200.000 Carvedilol | 84 16 | Almost zero-order release. Release time 15h. Formulation was unstable. |

Acidic stabilizicers

| Batch No. | Matrix | | Desired Release time 12 hours in 12 mm long tubular tablet. Result |
|---|---|---|---|
| | Ingredient | % w/w | |
| EC-042-045 50 mg Carvedilol | PEO 200.000 PEG 2000 ms Carvedilol Citric Acid | 47 16 32 5 | Release time was too short. Zero-order release, release time 11 h Faster release in acid than in buffer |
| EC-042-050 50 mg Carvedilol | PEO 200.000 PEG 2000 ms Carvedilol Succinic Acid | 47 16 32 5 | Zero-order, release time too short. Faster release in acid than in buffer |
| EC-042-066 50 mg Carvedilol | PEO 200.000 PEG 2000 ms Carvedilol Citric Acid | 58 5 32 5 | Zero-order, release time 12 h. Faster release in acid than in buffer |
| EC-042-069 50 mg Carvedilol | PEO 200.000 Carvedilol PEG 2000 ms Pectin Citric Acid | 40 32 3 20 5 | No zero-order release. Release in acid medium faster than in buffer. Some matrix was still left in the matrix. Pectin delayed release |
| EC-042-070 50 mg Carvedilol | PEO 200.000 Carvedilol PEG 2000 ms Starch (corn) Citric Acid | 40 32 3 20 5 | No release in buffer medium. |
| EC-042-081 50 mg Carvedilol | PEG 35.000 PEO 600.000 Carvedilol Citric Acid | 42 21 32 5 | Zero-order release, release time 16 h. Matrix was left in the shell. PEO 600.000 delayed the release |
| EC-042-082 50 mg Carvedilol | PEG 35.000 PEO 600.000 Carvedilol PEG 2000 ms Citric Acid | 39 19 32 5 5 | No zero-order release. Release time > 18h. |
| EC-042-083 50 mg Carvedilol | PEO 600.000 PEO 200.000 Carvedilol Citric Acid | 10 69 16 5 | Zero-order release, Release time > 18h. Matrix left in the shell. PEO 600.000 delayed the release. |

| Batch No. | Matrix | | Conclusion |
|---|---|---|---|
| | Ingredient | % w/w | |
| EC-042-047 50 mg Carvedilol | PEO 200.000 Carvedilol Citric Acid | 63 32 5 | Zero-order, release time 14 h. |
| EC-042-049 50 mg Carvedilol | PEO 200.000 Carvedilol Succinic Acid | 63 32 5 | Succinic acid could be used instead of citric acid as release time and release pattern were the same. |

(continued)

| Batch No. | Matrix | | Conclusion |
|---|---|---|---|
| | Ingredient | % w/w | |
| EC-042-053 50 mg Carvedilol | PEO 200.000 Carvedilol Citric Acid | 65,5 32 2,5 | Amount of citric acid too small. Same slope in acid and buffer was not observed. |
| EC-042-054 50 mg Carvedilol | PEO 200.000 Carvedilol Citric Acid | 58 32 10 | Amount of citric acid too high. Same slope in acid and buffer was not observed. |
| EC-042-073 50 mg Carvedilol | PEO 200.000 Carvedilol Citric Acid Tristearin | 61 32 5 2 | Almost zero-order release. Release in acid faster than in buffer. Matrix left in the shell. |
| EC-042-077 25 mg Carvedilol | PEO 200.000 Carvedilol Citric Acid | 81,5 16 2,5 | Zero-order release, release time 14 h. Comparable with 50 mg carvedilol EC-042-047 |
| EC-042-078 12.5 mg Carvedilol | PEO 200.000 Carvedilol Citric Acid | 90,75 8 1,25 | Zero-order release, release time 14 h. Comparable with 50 mg and 25 mg EC-042-047 and EC-042-077 |
| EC-042-079 25 mg Carvedilol | PEO 200.000 Carvedilol Citric Acid | 79 16 5 | Release profile in acid medium was increased when Citric Acid/ Carv increased |
| EC-042-080 50 mg Carvedilol | PEG 35.000 Carvedilol Citric Acid | 63 32 5 | PEG 35000 increased the release in acid medium. Undesired |
| EC-042-084 50 mg Carvedilol | PEO 200.000 Carvedilol Citric Acid Aluminum lactate | 74 16 5 5 | Almost zero-order, release time 13 h. Aluminium lactate reduced the release in acid medium. |

Addition of Zink Sulphate

| Batch No. | Matrix | | Conclusion |
|---|---|---|---|
| | Ingredient | % w/w | |
| EC-042-085 25 mg Carvedilol | PEO 200.000 Carvedilol Citric Acid Zink Sulphate | 77,5 16 5 1,5 | Zero-order release, release time 14 h. Zink Sulphate decreased release in acid compared to buffer. |
| EC-042-086 25 mg Carvedilol | PEO 200.000 Carvedilol Citric Acid Zinc Sulphate | 74,5 16 5 4,5 | Zero-order release, release time 14 h. Zink Sulphate decreased release in acid compared to buffer |

(continued)

| Batch No. | Matrix | | Conclusion |
|---|---|---|---|
| | Ingredient | % w/w | |
| EC-042-087 25 mg Carvedilol | PEO 200.000 Carvedilol Citric Acid Zinc Sulphate | 79,5 16 2,5 2 | Zero-order release, release time 13h. Zink Sulphate decreased release in acid |

Polymer with inorganic ingredients

| Batch No. | Matrix | | Conclusion |
|---|---|---|---|
| | Ingredient | % w/w | |
| EC-042-040 50 mg Carvedilol | PEO 200.000 PEG 2000 ms Carvedilol SiO2 | 52 6 32 10 | No release in buffer medium. See also EC-042-037, not comprising SiO2 |

**Polymer**

| *Batch No.* | | *Matrix* | | *Conclusion* |
|---|---|---|---|---|
| | *Formulation* | *Ingredient* | *% w/w* | |
| 042-130 | 50 mg Carvedilol | PEO 100.000 Carvedilol Potassium Sulfite Sucrose BHT | 74,8 24 0,2 0,5 0,5 | The dissolution profile shows zero-order release. |
| 042-149 | 50 mg Carvedilol | PEO 200.000 LF Carvedilol | 76 24 | The dissolution profile shows zero-order release, however different slope between acid and buffer. |

**Organic antioxidants**

| *Batch No.* | | *Matrix* | | *Conclusion* |
|---|---|---|---|---|
| | *Formulation* | *Ingredient* | *% w/w* | |
| 042-115 | 50 mg Carvedilol | PEO 200.000 Carvedilol Ascorbic acid | 75,9 24 0,1 | Diminishes degradation of PEO 200.000 and Carvedilol. The dissolution profile shows zero-order release. |
| 042-116 | 50 mg Carvedilol | PEO 100.000 Carvedilol Ascorbic acid | 75,9 24 0,1 | Diminishes degradation of PEO 100.000 and Carvedilol. The dissolution profile shows zero-order release. |

(continued)

| Batch No. | Formulation | Matrix Ingredient | % w/w | Conclusion |
|---|---|---|---|---|
| 042-133 | 50 mg Carvedilol | PEO 200.000<br>Carvedilol<br>Potassium Sulfite<br>Salicylic acid<br>BHT | 72,3<br>24<br>0,2<br>3<br>0,5 | Diminishes degradation of PEO 200.000 and Carvedilol. The dissolution profile shows zero-order release. |
| 042-135 | 50 mg Carvedilol | PEO 200.000<br>Carvedilol<br>Potassium Metabisulfite<br>BHT<br>Gentisic acid | 74,7<br>24<br><br>0,2<br>1<br>0,1 | The increased dosage of BHT did not produce any significant change in the level of impurities caused by PEO 200.000 compred to 0.5% BHT The dissolution profile does not show zero-order release. |
| 042-136 | 50 mg Carvedilol | PEO 200.000<br>Carvedilol Potassium Metabisulfite<br>BHT<br>Gentisic acid | 74,6<br>24<br>0,2<br>1<br>0,2 | The increased dosage of BHT did not produce any significant change in the level of impurities caused by PEO 200.000 compatred to 0.5% BHT The dissolution profile does not show zero-order release. |
| 042-141 | 50 mg Carvedilol | PEO 200.000<br>Carvedilol<br>Potassium Metabisulfite<br>BHT<br>Sorbitol<br>HCl | 64,765<br>24<br><br>0,2<br>1<br>10<br>0,035 | The increased dosage of BHT did not produce any significant change in the level of impurities caused by PEO 200.000 compared to 0,5% BHT The dissolution profile does not show zero-order release. |

**2.3 Inorganic antioxidants**

| Batch No. | Formulation | Matrix Ingredient | % w/w | Conclusion |
|---|---|---|---|---|
| 042-117 | 50 mg Carvedilol | PEO 200.000<br>Carvedilol<br>Potassium Metabisulfite | 75,9<br>24<br><br>0,1 1 | Diminishes degradation of PEO 200.000 and Carvedilol. The dissolution profile shows zero-order release. |
| 042-133 | 50 mg Carvedilol | PEO 200.000<br>Carvedilol<br>Potassium Metabisulfite<br>Salicylic acid<br>BHT | 72,3<br>24<br><br>0,2<br>3<br>0,5 | Diminishes degradation of PEO 200.000 and Carvedilol. The dissolution profile shows zero-order release. |
| 042-134 | 50 mg Carvedilol | PEO 200.000<br>Carvedilol<br>Potassium Metabisulfite<br>BHT | 74,8<br>24<br><br>0,2<br>1 | Diminishes degradation of PEO 200.000 and Carvedilol. The dissolution profile shows zero-order release. |

**6. Sugars**

| Batch No. | | Matrix | | | Conclusion |
|---|---|---|---|---|---|
| | Formulation | Ingredient | % w/w | | |
| 042-118 | 50 mg Carvedilol | PEO 200.000 Carvedilol Potassium Sulfite Sucrose | 75,8 24 0,1 0,1 | | The dissolution profile shows zero-order release. |
| 042-120 | 50 mg Carvedilol | PEO 200.000 Carvedilol Potassium Sulfite Sucrose | 70,9 24 0,1 5,0 | | Production cancelled, the Concentrasion of Sucrose to hight for the selected process parameters. May be produced with increased temperature |
| 042-121 | 50 mg Carvedilol | PEO 200.000 Carvedilol Potassium Sulfite Mannitol | 65,9 24 0,1 10 | | Carvedilol precipitated when standing. |
| 042-122 | 50 mg Carvedilol | PEO 200.000 Carvedilol Potassium Sulfite Sucrose BHT | 73,3 24 0,2 2 0,5 | | Concentration of Sucrose to high for the selected process parameters. May be produced with increased temperature. |
| 042-123 | 50 mg Carvedilol | PEO 200.000 Carvedilol Potassium Sulfite Sucrose BHT | 71,8 24 0,2 3,5 0,5 | | The concentration was too high and resulted in process problems. Production cancelled. |
| 042-129 | 50 mg Carvedilol | PEO 200.000 Carvedilol Potassium Sulfite Sucrose BHT | 74,8 24 0,2 0,5 0,5 | | The dissolution profile shows zero-order release. |
| 042-130 | 50 mg Carvedilol | PEO 100.000 Carvedilol Potassium Sulfite Sucrose BHT | 74,8 24 0,2 0,5 0,5 | | The dissolution profile shows zero-order release. |
| 042-137 | 50 mg Carvedilol | PEO 200.000 Carvedilol Potassium Metabisulfite BHT Mannitol | 59,8 24 0,2 1 15 | | Mannitol did not produce the expected result, because Carvedilol precipitated when standing. |

(continued)

| Batch No. | | Matrix | | Conclusion |
|---|---|---|---|---|
| | Formulation | Ingredient | % w/w | |
| 042-141 | 50 mg Carvedilol | PEO 200.000<br>Carvedilol<br>Potassium<br>Metabisulfite<br>BHT<br>Sorbitol<br>HCl | 64,765<br>24<br><br>0,2<br>1<br>10<br>0,035 | Carvedilol precipitated after standing approximately one week. |

**Increase in hydrogen bondings**

| Batch No. | | Matrix | | Conclusion |
|---|---|---|---|---|
| | Formulation | Ingredient | % w/w | |
| 042-128 | 50 mg Carvedilol | PEO 200.000<br>Carvedilol<br>Potassium<br>Metabisulfite<br>2-amino-2(hydroxymethyl)1,3 ropandiol<br>BHT | 74,8<br>24<br><br>0,2<br>0,5<br><br>0,5 | Carvedilol precipitated after standing approximately one week. Dissolution profile shows zero-order release. |
| 042-131 | 50 mg Carvedilol | PEO 200.000<br>Carvedilol<br>Potassium<br>Metabisulfite<br>Klucel<br>BHT | 74,3<br>24<br><br>0,2<br>1<br>0,5 | Dissolution profile shows zero-order release. |

| Batch No. | | Matrix | | Conclusion |
|---|---|---|---|---|
| | Formulation | Ingredient | % w/w | |
| 042-142 | 50 mg Carvedilol | PEO 200.000<br>Carvedilol<br>PVP K90 | 74<br>24<br>2 | The dissolution profile is zero-order. |
| 042-143-02-001 | 50 mg Carvedilol | PEO 200.000<br>Carvedilol<br>Sorbitol | 71<br>24<br>5 | Dissolution not zero-order. |
| 042-144 | 50 mg Carvedilol | PEO 200.000<br>Carvedilol<br>Cyclodextrin | 73<br>24<br>3 | Dissolution not zero-order. |
| 042-145 | 50 mg Carvedilol | PEO 200.000<br>Carvedilol<br>Cyclodextrin | 69<br>24<br>7 | Dissolution not zero-order.. |

| Batch No. | | Matrix | | | Conclusion |
|---|---|---|---|---|---|
| | Formulation | Ingredient | % | | |
| 042-141 | 50 mg Carvedilol | PEO 200.000 | 64,765 | | Dissolution profile does not show zero-order release, possibly due to the amount of BHT. |
| | | Carvedilol | 24 | | |
| | | Potassium Metabisulfite | 0,2 | | |
| | | BHT | 1 | | |
| | | Sorbitol | 10 | | |
| | | HCl | 0,035 | | |
| | Formulation | Ingredient | | | |
| 042-148 | 50 mg Carvedilol | PEO 200.000 | 72 | | The dissolution profile does not show zero-order release possibly due to the amount of BHT. |
| | | Carvedilol | 24,1 | | |
| | | Potassium Metabisulfite | 0,2 | | |
| | | BHT | 1 | | |
| | | $KH_2PO_4$ | 0,32 | | |
| | | HCl | 0,61 | | |
| | | PVP K90 | 1,7 | | |
| 042-153 | 50 mg Carvedilol | PEO 200.000 | 68,5 | | Test of granulation method |
| | | Carvedilol | 24 | | |
| | | Potassium Metabisulfite | 0,2 | | |
| | | BHT | 0,5 | | |
| | | $KH_2PO_4$ | 0,20 | | |
| | | $H_3PO_4$ | 4,6 | | |
| | | PVP K90 | 2,0 | | |

Examples disclosing Formulation Nos 68 to 84.

6 mm oval shaped formulations 150mm$^2$

| | |
|---|---|
| 0068<br>PEO 200.000,<br>Carv.(24,0%),<br>PM (0,2%),<br>BHT(0,5%);<br>Meta-Phosphoric acid*<br>(5,2%);<br>PVP K90 (2,0%) | Dry mixing with solid phosphoric acid.<br>pH 7,27<br>Base line Dissolution zero-order, erosion time 7 hours, not complete dissolution at hour 8, matrix on bottom of vessel<br><br>Carv/acid*: 4.6<br>Carv/HPO3: 10.9) |
| 0069<br>PEO 200.000 81.7%<br>Carv.(12,0%),<br>PM (0,2%),<br>BHT(0,5%);<br>Meta-Phosphoric acid* (3,6%)<br><br>PVP K90 (2,0%) | Dry mixing: Acid(s) and PEO mixed in mortar. Blended and crushed. All mixed. Mix kept dry.<br>pH 7,27<br>Appearance of tablets after production:<br>Transparent yellowish<br>Baseline Dissolution, zero-order, erosion time 6.5 hours, complete dissolution at hour 7<br>Dissolution in simulated fasted and fed media zero-order (Figs. 8 and 9)<br>Carv/acid*: 3.3<br>Carv/HPO3: 7.9) |

(continued)

| | |
|---|---|
| | Appearance/Dissolution after 26 days storage at 30°C/60%Rh: Transparent yellowish/Zero-order erosion time 6 hours, complete dissolution after 6.5 hours (Figure 10) |
| Not produced<br>PEO 200.000 LF83.7%<br>Carv.(12,0%),<br>PM (0,2%),<br>BHT(0,5%);<br>Meta-Phosphoric acid*<br>(3,6%) *(42,1%HO3P)* | Carvedilol/acid ratio 3.3* (*7.9 based on HO3P*)<br>Exptected values:<br>pH about 6<br>Appearance of tablets transparent yellowish<br>Dissolution, zero-order |
| 0070<br>PEO 200.000,<br>Carv.(24,0%),<br>PM (0,2%),<br>BHT(0,5%);<br>Ortho-Phosphoric acid<br>(4,4%);<br>PVP K90 (2,0%) | Dry mixing: $H_3PO_4(s)$ in PEO. Then mixed with rest. Mix kept dry.<br>pH 5.93<br>Appearance after production: White<br>Baseline Dissolution: Carvedilol.not released in buffer due to crystallization.<br>Carvedilol /acid**: 5.45 |
| Composition<br>0073<br>PEO 200.000 (LF),<br>Carv. (18,0%),<br>PM (0,2%),<br>BHT(0,5%);<br>Ortho-Phosphoric acid<br>(4,5%);<br>PVP K90 (2,0%) | Dry mixing: $H_3PO_4(s)$ in PEO. Then mixed with rest. Mix kept dry.<br>pH 3.23<br>Appearance after production: Transparent yellowish,<br>Dissolution:<br>Dissolution, zero-order, erosion time 6 hours, complete dissolution at hour 6.5<br>Carv/acid**: 4<br>Appearance: After 1-2 weeks storage white |
| 0075<br>PEO 200.000(LF)<br>Carv. (18,0%),<br>PM (0,2%),<br>BHT(0,5%);<br>Ortho-Phosphoric acid<br>(3,6%);<br>PVP K90 (2,0%) | Dry mixing: $H_3PO_4(s)$ in 40 g PEO. Rest of PEO mixed with carv. + antioxidants. Mixed all. Mix kept dry.<br>pH 5,71<br>Appearance after production: White<br>Dissolution: Cancelled due to crystallization<br>Carv/acid**: 5 |
| 0083<br>PEO 200.000(LF),<br>Carv. (12,0%),<br>PM (0,2%),<br>BHT(0,5%);<br>Ortho-Phosphoric acid (2,4%);<br>PVP K90 (2,0%) | Dry mixing: Powders mixed and $H_3PO_4$ (I) mixed in some of the powders. All mixed. Mix kept dry. pH in mixture 3.84<br>Appearance after production: White<br>Dissolution: Cancelled due to crystallization<br>Carv/acid**: 5 |
| 0084<br>PEO 200.000 (LF),<br>Carv. (12,0%),<br>PM (0,2%),<br>BHT(0,5%);<br>Meta-Phosphoric acid*<br>(3,6%); | pH 3.23 (mixture)<br>Appearance after production: Transparent yellowish,<br>Dissolution: Zero-order, erosion time 6 hours, complete dissolution at hour 6.5<br><br>Carv/acid*: 3.3<br>*Carv/HPO3: 7.9)* |

(continued)

| PVP K90 (2,0%) | |
|---|---|

*The Meha Phosphoric acid comprises 42.1 %HPO3 and 54.4%(NaPO3)$_6$
** Ortho Phosphoric acid comprises 86% H3PO4)

Items

1. A controlled release pharmaceutical composition for oral use comprising a solid dispersion of

   i) at least one therapeutically, prophylactically and/or diagnostically active substance, which at least partially is in an amorphous form,

   ii) a pharmaceutically acceptable polymer that has plasticizing properties and which has a melting point or melting interval of a temperature of at the most 200 °C, and

   iii) optionally, a stabilizing agent,

the at least one therapeutically, prophylactically and/or diagnostically active substance having a water solubility of at the most 3 mg/ml at 25 °C such as, e.g. at the most about 2 mg/ml, at the most about 1 mg/ml, and the composition being designed to release the active substance with a substantially zero order release.

2. A composition according to item 1, whrein the polymer is a polyethylene glycol and/or a polyethylene oxide having a molecular weight of at least about 20,000 in crystalline and/or amorphous form or a mixture such polymers.

3. A pharmaceutical composition according to item 1 or 2, wherein the composition is coated with a coating a coating having at least one opening exposing at the one surface of said matrix, the coating comprising

   i) a first cellulose derivative which has thermoplastic properties and which is substantially insoluble in the aqueous medium in which the composition is to be used,
   and at least one of
   ii) a second cellulose derivative which is soluble or dispersible in water,
   iii) a plasticizer, and
   iv) a filler.

4. A pharmaceutical composition according to any of items 1-3 for controlled release of the active substance into an aqueous medium by erosion of at least one surface of the composition.

5. A pharmaceutical composition according to any of the preceding items, wherein the active substance is carvedilol.

6. A composition according to item 5, wherein the active substance is selected from the group consisting of the racemate: (RS)-1-(9H-carbazol-4-yloxy)-3-[2-(2.methoxyphenoxy)-ethylaminopropan-2-ol, the two individual enantiomers: (S)-1-(9H-carbazol-4-yloxy)-3-[2-(2.methoxyphenoxy)-ethylaminopropan-2-ol and (S)-1-(9H-carbazol-4-yloxy)-3-[2-(2.methoxyphenoxy)-ethylaminopropan-2-ol, metabolites of carvedilol including desmethylcarvedilol, pharmaceutically acceptable salts, complexes, solvates and anhydrate thereof, and mixtures thereof.

7. A composition according to any of the preceding items, wherein the active substance is present in any of its crystalline, polymorphous or amorphous forms or mixtures thereof.

8. A pharmaceutical composition according to any of the preceding items, wherein the active substance at least partially is present in solid form in the dispersion.

9. A pharmaceutical composition according to any of the preceding items, wherein the active substance at least partially is present in a molecular dispersion such as, e.g., in the form of a solid or semi-solid solution.

10. A pharmaceutical composition according to item 8, wherein the active substance at least partially is present in a colloidal dispersion.

11. A pharmaceutical composition according to item 8, wherein the active substance at least partially is present in a crystalline form.

12. A pharmaceutical composition according to items 5-7, wherein carvedilol at least partially is present in amorphous form with a mean particle size of from about 0.01 $\mu$m to about 500 $\mu$m such as, e.g., from about 0.05 $\mu$m to about 500 $\mu$m, from about 0.1 $\mu$m to about 500 $\mu$m, typically from about 0.5 $\mu$m to about 300 $\mu$m, more typically from about 1 $\mu$m to about 200 $\mu$m, especially from about 1 $\mu$m to about 100 $\mu$m.

13. A composition according to any of items 2-12, wherein the stabilizing agent is a substance, which - together with the polyethylene glycol and/or polyethylene oxide - form a dispersion medium in which the active substance is contained.

14. A composition according to any of the preceding items, wherein the concentration and/or the nature of the ingredients making up the matrix composition has been adjusted in such a manner that the diffusion rate of the aqueous medium into the matrix composition corresponds to about 100% $\pm$ 30% such as, e.g. about 100% $\pm$ 25%, about 100% $\pm$20%, about 100% $\pm$15% or about 100% $\pm$10% or about 100% of the dissolution rate of the matrix composition so as to obtain a zero order release of at least about 60% w/w such as, e.g. at least about 65% w/w at least about 70% w/w, at least about 75% w/w, at least about 80% w/w, at least about 85% w/w, at least about 90% w/w, at least about 95% w/w or at least about 97 or 98% w/w of the active substance from the pharmaceutical composition when subject to an in vitro dissolution test as described herein.

15. A composition according to any of the preceding items, wherein the polymer is a substantially water soluble or crystalline polymer or a mixture of substantially water soluble and/or crystalline polymers.

16. A composition according to any of the preceding items comprising a stabilizing agent.

17. A composition according to item 16, wherein the stabilizing agent is selected from the group consisting of diffusion and dissolution adjusting agents, pH-adjusting agents, buffering agents, agents that does not increase the mobility of the ingredients in the composition, agents that prevent crystal formation and agents that have antioxidative properties.

18. A composition according to item 16 or 17, wherein the stabilizing agent is selected from the group consisting of inorganic acids, inorganic bases, inorganic salts, organic acids or bases and pharmaceutically acceptable salts thereof, saccharides, oligosaccharides, polysaccharides, and cellulose and cellulose derivatives, or mixtures thereof.

19. A composition according to item 18, wherein the organic acid is a mono-, di-, oligo, polycarboxylic acid or amino acids such as, e.g. acetic acid, ethanoic acid, succinic acid, citric acid, tartaric acid, acrylic acid, benzoic acid, malic acid, maleic acid, adipic acid, angelic acid, ascorbic acid/vitamin C, carbamic acid, cinnamic acid, citramalic acid, formic acid, fumaric acid, gallic acid, gentisic acid, glutaconic acid, glutaric acid, glyceric acid, glycolic acid, glyoxylic acid, lactic acid, levulinic acid, malonic acid, mandelic acid, oxalic acid, oxamic acid, pimelic acid, pyruvic acid, aspartic and glutamic acid, or mixtures thereof.

20. A composition according to item 19, wherein the inorganic acid is pyrophosphoric, glycerophosphoric, phosphoric such as ortho or meta phosphoric, boric acid, hydrochloric acid, or sulfuric acid, or mixtures thereof.

21. A composition according to item 18, wherein the suitable inorganic compounds include aluminium.

22. A composition according to item 18, wherein the suitable organic bases are selected from the group consisting of p-nitrophenol, succinimide, benzenesulfonamide, 2-hydroxy-2cyclohexenone, imidazole, pyrrole, diethanolamine, ethyleneamine, tris (hydroxymethyl) aminomethane, hydroxylamine and derivates of amines, sodium citrate, aniline, and hydrazine, or mixtures thereof.

23. A composition according to item 18, wherein the suitable inorganic bases are selected from the group consisting of aluminium oxide such as, e.g., aluminium oxide trihydrate, alumina, sodium hydroxide, potassium hydroxide, calcium carbonate, ammonium carbonate, ammnonium hydroxide, KOH and the like, or mixtures thereof.

24. A composition according to item 18, wherein the pharmaceutically acceptable salt of an organic acid is e.g. an alkali metal salt or an alkaline earth metal salt such as, e.g. sodium phosphate, sodium dihydrogenphosphate, disodium hydrogenphosphate etc., potassium phosphate, potassium dihydrogenphosphate, potassium hydrogenphosphate etc., calcium phosphate, dicalcium phosphate etc., sodium sulfate, potassium sulfate, calcium sulfate, sodium carbonate, sodium hydrogencarbonate, potassium carbonate, potassium hydrogencarbonate, calcium carbonate, magnesium carbonate etc., sodium acetate, potassium acetate, calcium acetate, sodium succinate, potassium succinate, calcium succinate, sodium citrate, potassium citrate, calcium citrate, sodium tartrate, potassium tartrate, calcium tartrate, zinc gluconate, zinc sulphate etc., or mixtures thereof.

25. A composition according to item 18, wherein the inorganic salt is sodium chloride, potassium chloride, calcium chloride, magnesium chloride etc., or mixtures thereof.

26. A composition according to item 18, wherein the pharmaceutically acceptable excipient is selected from glucose and other monosaccharides, ribose, arabinose, xylose, lyxose, allose, altrose, inosito, glucose, sorbitol, mannose, gulose, idose, galactose, talose, mannitol, fructose, lactose, sucrose, and other disaccharides, dextrin, dextran or other polysaccharides, amylose, xylan, cellulose and cellulose derivatives such as, e.g. microcrystalline cellulose, methyl cellulose, ethyl cellulose, ethylhydroxyethyl cellulose, ethylmethylcellulose, hydroxyethylcellulose, hydroxyethylmethyl cellulose, carboxymethyl cellulose, hydroxypropyl cellulose, hydroxymethylpropyl cellulose, hydroxypropylmethyl cellulose, amylopectin, pectin, starch, sodium starch etc.,kaolin, bentonit, acacia, alginic acid, sodium alginate, calcium alginate, gelatin, dextrose, molasses, extract of Irish moss, panwar gum, ghatti gum, mucilage of isapol husk, veegum, glycollate, magnesium stearate, calcium stearate, stearic acid, talc, titanium dioxide, silicium dioxide, clays, croscarmellose, gums, agar etc., or mixtures thereof.

27. A composition according to any of the preceding items further comprising a pharmaceutically acceptable excipient selected from the group consisting of fillers, diluents, disintegrants, glidants, pH-adjusting agents, viscosity adjusting agents, solubility increasing or decreasing agents, osmotically active agents and solvents.

28. A composition according to item 18, wherein the stabilizing agent is citric acid including solvates and anhydrate thereof.

29. A composition according to item 28, wherein citric acid is in racemic form or in any of its enantiomers or mixtures thereof.

30. A composition according to item 28 or 29, wherein the citric acid is in any of its crystalline, polymorphous and/or amorphous forms.

31. A composition according to any of items 28-30, wherein the citric acid has a water content of at the most about 15% w/w such as, e.g., at the most about 12% w/w, such as e.g., at the most about 10% w/w, such as e.g., at the most about 8% w/w, such as e.g., at the most about 5% w/w, such as e.g., at the most about 3% w/w, such as e.g., at the most about 2% w/w, such as at the most about 1 % w/w or at the most about 0.5% w/w.

32. A composition according to any of items 28-31, wherein the citric acid at least partially is present in the form of a molecular dispersion.

33. A composition according to any of items 28-31, wherein the citric acid at least partially is present in the form of a colloidal dispersion.

34. A composition according to any of items 28-31, wherein the citric acid at least partially is present in the crystalline form.

35. A composition according to item 18, wherein the stabilizing agent is a phosphoric acid or a phosphonic acid or a salt thereof.

36. A composition according to item 35, wherein the phosphoric acid is ortho or meta phoshoric acid or a mixture thereof.

37. A composition according to any items 2-36, wherein polyethylene glycol and/or polyethylene oxide are: a polyethylene glycol, a polyethylene oxide, and/or a block copolymer of ethylene oxide and propylene oxide including poly(ethylene-glycol-b-(DL-lactic acid-co-glycolic acid) - b- ethylene glycol (PEG-PLGA PEG), poly((DL-lactic acid-co-glycolic acid) - g-ethylene glycol) (PLGA-g-PEG), and polyethylene oxide - polypropylene oxide (PEO-PPO).

38. A composition according to item 37 wherein the polyethylene glycol, the polyethylene oxide and/or the block copolymer of ethylene oxide and propylene oxide has a molecular weight of from about 20,000 daltons, such as, e.g., from about 20,000 to about 700,000 daltons, from about 20,000 to about 600,000 daltons, from about 35,000 to about 500,000 daltons, from about 35,000 to about 400,000 daltons, from about 35,000 to about 300,000 daltons, from about 50,000 to about 300,000 daltons, such as, e.g. about 35,000 daltons, about 50,000 daltons, about 75,000 daltons, about 100,000 daltons, about 150,000 daltons, about 200,000 daltons, about 250,000 daltons, about 300,000 daltons or about 400,000 daltons.

39. A composition according to item 37, wherein the block copolymer of ethylene oxide and propylene oxide comprises up to about 30% w/w of the propylene oxide based block, and has a molecular weight of about 5,000 daltons, typically about 5,000 to about 30,000 daltons such as, e.g. from about 8,000 to about 15,000 daltons.

40. A composition according to any of items 2-39, wherein the polyethylene glycol and/or polyethylene oxide has a melting point of about 20-120°C such as, e.g. from about 30 to about 100°C or from about 40 to about 80°C.

41. A composition according to any of the preceding items, wherein the concentration of the active substance in the composition corresponds to a concentration of at the most the saturated concentration in component ii) at a temperature corresponding to the melting point or the lowest end point of the melting interval of component ii) optionally together with component iii).

42. A composition according to any of the preceding items, wherein component ii) is a polyethylene glycol and/or a polyethylene oxide having a molecular weight of at least about 20,000 in crystalline and/or amorphous form or a mixture such polymers.

43. A composition according to any of the preceding items, wherein component ii) is of a quality that ensures that free radicals formed, if any, do not significantly increase the degradation of the active substance in the composition.

44. A composition according to any of the preceding items further comprising one or more antioxidants that inhibits the formation of peroxides and/or inactivates any peroxides present.

55. A composition according to any of the preceding items, wherein the active substance has antioxidant properties.

56. A composition according to any of the preceding items, wherein the composition is stable with respect to physical stability.

57. A composition according to any of the preceding items, wherein the composition is stable with respect to *in vitro* dissolution of the active substance from the composition.

58. A composition according to item 57, wherein the composition is stable with respect to *in vitro* dissolution behaviour in such a manner that $t_{50\%}$, i.e. the time for 50% w/w of the active substance to dissolve in a dissolution medium, differs at the most $\pm$ 20% w/w such as, e.g., at the most $\pm$ 15% w/w, at the most $\pm$ 10% w/w, at the most $\pm$ 7.5% w/w, at the most $\pm$ 5% w/w, at the most $\pm$ 2.5% w/w, at the most $\pm$ 1.5% w/w or at the most 1% w/w when two compositions from the same batch is compared with a time difference of 2 weeks under similar storage and test conditions.

59. A composition according to any of the preceding items, wherein the composition is stable with respect to chemical stability of the active substance.

60. A composition according to item 59, wherein the concentration of the active substance in the composition decreases at the most 20% w/w such as, e.g. at the most 15% w/w, at the most 10% w/w, at the most 7.5% w/w or at the most 5% w/w when stored at room temperature for a time period of at least 3 months such as, e.g. 6 months, 12 months, 18 months or 24 months and a relative humidity of at the most 75% such as, e.g., at the most 70%, at the most 65%, at the most 60%, at the most 55%, at the most 50% or at the most 45%.

61. A composition according to any of the preceding items comprising carvedilol and one or more active substances for human or veterinary use, a vitamin or other nutritional supplement, a disinfectant, a deodorant or another substance to be administered continuously in an aqueous environment.

62. A composition according to any of items 1-40, wherein the active substance is present in the composition in a concentration of from about 0.1 to about 98% w/w such as, e.g. at the most about 90% w/w, at the most about 85% w/w, at the most about 80% w/w, at the most about 75% w/w, at the most about 70% w/w, at the most about 65% w/w or at the most about 60% w/w.

63. A composition according to item 27, wherein at least one pharmaceutically acceptable excipient and/or the stabilizing agent has a solubility of at least 1 mg/ml such as, e.g. at least about 3 mg/ml, at least about 5 mg/ml, at least about 10 mg/ml, at least about 25 mg/ml or at least about 50 mg/ml in water at ambient temperature.

64. A composition according to item 27, wherein at least one pharmaceutically acceptable excipient and/or the stabilizing agent, which has a solubility of at the most about 3 mg/ml such as, e.g., at the most about 1 mg/ml, at the most about 0.1 mg/ml, at the most about 0.05 mg/ml such as, e.g. at the most about 0.001 mg/ml in water at ambient temperature.

65. A composition according to any of the preceding items in the form of a matrix composition.

66. A composition according to item 65, wherein any exposed matrix surfaces erode at a substantially constant rate.

67. A composition according to any of items 3-66, wherein in the aqueous medium in which the composition is to be used, the coating does not completely crumble or erode before the matrix has completely eroded.

68. A composition according to any of items 3-67, wherein said first cellulose derivative is a cellulose ether which, when heated, is shapeable by molding or extrusion, including injection molding, blow molding and compression molding.

69. A composition according to item 68 in which the cellulose ether comprises at least one ethylcellulose.

70. A composition according to item 69 in which said ethylcellulose has an ethoxyl content in the range of 44.5-52.5%.

71. A composition according to item 70 in which said ethylcellulose has an ethoxyl content in the range of 45-49.5%.

72. A composition according to any of items 3-67 in which said first cellulose derivative is selected from the group consisting of cellulose acetate, cellulose propionate and cellulose nitrate.

73. A composition according to any of items 3-67 in which said second cellulose derivative is selected from the group consisting of methylcellulose, carboxymethylcellulose and salts thereof, cellulose acetate phthalate, microcrystalline cellulose, ethylhydroxyethylcellulose, ethylmethylcellulose, hydroxyethylcellylose, hydroxyethylmethylcellulose, hydroxypropylcellulose, hydroxymethylcellulose and hydroxymethylpropylcellulose.

74. A composition according to item 73 in which said salt of carboxymethylcellulose is selected from the group consisting of alkali metal and alkaline earth metal salts.

75. A composition according to any of items 3-74, in which said plasticizer is selected from the group consisting of phosphate esters; phthalate esters; amides; mineral oils; fatty acids and esters thereof with polyethylene glycol, glycerin or sugars; fatty alcohols and ethers thereof with polyethylene glycol, glycerin or sugars; vegetable oils and hydrogenated vegetable oils; nitrobenzene, carbon disulfide, β-naphtyl salicylate, phthalyl glycolate, diocyl phthalate etc.

76. A composition according to item 75 in which said fatty alcohol is selected from the group consisting of cetostearyl alcohol, cetyl alcohol, stearyl alcohol, oleyl alcohol and myristyl alcohol.

77. A composition according to any of items 3-76 in which said plasticizer is a non-ionic surfactant.

78. A composition according to any of items 1-77, wherein the solid dispersion does not contain polyethylene glycol 2000 monostearate or polyethylene glycol 400 monostearate.

79. A composition according to any of the preceding items, wherein the composition comprises

a) a third cellulose derivative which has thermoplastic properties and which is substantially insoluble in the aqueous medium in which the composition is to be used,
and at least one of
b) a fourth cellulose derivative which is soluble or dispersible in water,
c) a second plasticizer, and
d) a second filler.

80. A according to item 79, wherein said third derivatives is of a type as defined in any one of items 68-71.

81. A composition according to item 79, wherein said fourth cellulose derivative is of a type as defined in any one of items 73 or 74.

82. A composition according to item 79, wherein said second plasticizer is of a type defined in any one of items 75-77.

83. A composition according to item 79, wherein said filler is a filler, a diluent, a binder, a lubricant a disintegrant or a water-soluble anti-oxidant, a lipid soluble antioxidant and/or a preservative.

84. A composition according to any of the preceding items, wherein the polyethylene glycol and/or polyethylene oxide have a molecular weight of at least 100,000 daltons and at the most 400,000 daltons.

85. A composition according to any of the preceding items comprising PEO 200,000 and a phosphoric and/or citric acid and/or succinic acid as a stabilizing agent.

86. A composition according to any of the preceding items, wherein the release of carvedilol from the composition is zero order and about 50% w/w carvedilol is released from the composition within 5-6 hours from start of release as measured by the dissolution test described herein.

87. A composition according to any of items 1-85, wherein the release of carvedilol from the composition is zero order and about 50% w/w carvedilol is released from the composition within 4-5 hours from start of release as measured by the dissolution test described herein.

88. A composition according to any of items 1-85, wherein the release of carvedilol from the composition is zero order and about 50% w/w carvedilol is released from the composition within 3-4 hours from start of release as measured by the dissolution test described herein.

89. A composition according to any of items 1- 85, wherein the release of carvedilol from the composition is zero order and about 50% w/w carvedilol is released from the composition within 2-3 hours from start of release as measured by the dissolution test described herein.

90. A composition according to any of the preceding items, wherein the release of carvedilol from the composition is substantially delayed for 0.25 to 4 hours, e.g. for 0.5 to 3 hours, such as from 1 to 2 hours before the zero order release starts as measured by the dissolution test described herein.

91. A method of treating hypertension, angina pectoris and/or congestive heart failure, which method comprises administering carved in a composition according to any of items 1-90.

92. A method for preparing a composition according to any of items 1-90, the method comprises injection moulding of a melted or semi-solid mixture of the individual components making up the composition into a suitable form, application of a coating by means of injection moulding and cooling the thus prepared coated composition to solidify the composition.

93. A method according to item 92, wherein the method is a substantially single continuous process.

94. A method according to item 92, wherein the cooling is performed under controlled conditions to a temperature of from about 0 °C to about 20 °C such as, e.g. from about 5 °C to about 18 °C, from about 10 °C to about 16 °C such as, e.g. about 10 °C, about 12 °C, about 14 °C, about 15 °C or about 16 °C.

95. A method according to item 92 comprising a step of heating while the polymer and the active substance is in physical contact with each other.

96. A package comprising a selection of pharmaceutical compositions according to any of items 1-90, the selection comprising compositions having different content of carvedilol.

97. A package according to item 96, wherein the selection comprises compositions wherein the difference in carvedilol content is at 2 fold or more such as 3, 4, 5, 6, 7, 8, 9, 10, 11, 12, 13, 14, 15 or 16 fold.

98. A package according to item 96, wherein the selection comprises compositions having a content selected from 6.25, 12.5, 25, 37.5 and 50 mg carvedilol.

99. A package according to item 96, wherein the selection comprises at least two compositions such as 2, 3, 4, 5, 6, 7 or 8 compositions.

100. A composition according to any of items 1-90 containing carvedilol as active substance in a concentration of at the most about 23% w/w such as, e.g., at the most about 22% w/w, at the most about 21 % w/w or at the most about 20% w/w.

101. A composition according to item 100 containing carvedilol as active substance and PEO 200,000 as component ii) and wherein the concentration of carvedilol in PEO 200,000 is at the most about 22% w/w, at the most about 21 % w/w or at the most about 20% w/w.

## Claims

1. A controlled release pharmaceutical composition for oral use comprising a solid dispersion of

    i) carvedilol, which at least partially is in an amorphous form,
    ii) a pharmaceutically acceptable polymer that has plasticizing properties and which has a melting point or melting interval of a temperature of at the most 200 °C,

    and the composition being designed to release carvedilol with a substantially zero order release.

2. A composition according to claim 1, wherein the composition is stable with respect to chemical stability of carvedilol.

3. A composition according to any of the preceding claims, wherein the composition is stable with respect to physical stability.

4. A composition according to any of the preceding claims, wherein the composition is stable with respect to *in vitro* dissolution of carvedilol from the composition.

5. A composition according to any of the preceding claims further comprising a stabilizing agent selected from the group consisting of inorganic acids, inorganic bases, inorganic salts, organic acids or bases and pharmaceutically acceptable salts thereof, saccharides, oligosaccharides, polysaccharides, and cellulose and cellulose derivatives, or mixtures thereof.

6. A composition according to claim 5, wherein the stabilizing agent is a phosphoric acid or a phosphonic acid or a salt thereof.

7. A composition according to claim 6, wherein the phosphoric acid is ortho or meta phoshoric acid or a mixture thereof.

8. A composition according to any of the preceding claims, wherein the polymer is a polyethylene glycol and/or a polyethylene oxide having a molecular weight of at least about 20,000 in crystalline and/or amorphous form or a mixture such polymers.

9. A composition according to any of the preceding claims containing carvedilol in a concentration of at the most about 23% w/w such as, e.g., at the most about 22% w/w, at the most about 21 % w/w or at the most about 20% w/w.

**10.** A composition according to claim 9 containing PEO 200,000 as component ii) and wherein the concentration of carvedilol in PEO 200,000 is at the most about 22% w/w, at the most about 21% w/w or at the most about 20% w/w.

**11.** A pharmaceutical composition according to any of the preceding claims, wherein the composition is coated with a coating a coating having at least one opening exposing at the one surface of said matrix, the coating comprising

> i) a first cellulose derivative which has thermoplastic properties and which is substantially insoluble in the aqueous medium in which the composition is to be used,
> and at least one of
> ii) a second cellulose derivative which is soluble or dispersible in water,
> iii) a plasticizer, and
> iv) a filler.

**12.** A pharmaceutical composition according to any of the preceding claims for controlled release of carvedilol into an aqueous medium by erosion of at least one surface of the composition.

**13.** A composition according to any of the preceding claims, wherein carvedilol is selected from the group consisting of the racemate: (RS)-1-(9H-carbazol-4-yloxy)-3-[2-(2.methoxyphenoxy)-ethylaminopropan-2-ol, the two individual enantiomers: (S)-1-(9H-carbazol-4-yloxy)-3-[2-(2.methoxyphenoxy)-ethylaminopropan-2-ol and (R)-1-(9H-carbazol-4-yloxy)-3-[2-(2.methoxyphenoxy)-ethylaminopropan-2-ol, metabolites of carvedilol including desmethylcarvedilol, pharmaceutically acceptable salts, complexes, solvates and anhydrate thereof, and mixtures thereof.

**14.** A composition according to any of the preceding claims, wherein carvedilol is present in any of its crystalline, polymorphous or amorphous forms or mixtures thereof.

**15.** A composition according to any of the preceding claims, wherein the polymer is a substantially water soluble or crystalline polymer or a mixture of substantially water soluble and/or crystalline polymers.

**16.** A composition according to any claims 8-15, wherein polyethylene glycol and/or polyethylene oxide are: a polyethylene glycol, a polyethylene oxide, and/or a block copolymer of ethylene oxide and propylene oxide including poly (ethylene-glycol-b-(DL-lactic acid-co-glycolic acid) - b- ethylene glycol (PEG-PLGA PEG), poly((DL-lactic acid-co-glycolic acid) - g-ethylene glycol) (PLGA-g-PEG), and polyethylene oxide - polypropylene oxide (PEO-PPO).

**17.** A composition according to any of the preceding claims, wherein component ii) is of a quality that ensures that free radicals formed, if any, do not significantly increase the degradation of carvedilol in the composition.

**18.** A composition according to any of the preceding claims further comprising one or more antioxidants that inhibits the formation of peroxides and/or inactivates any peroxides present.

**19.** A composition according to claim 18, wherein the antioxidant is betacarotene, butylated hydroxyanisole, butylated hydroxytoluene, potassium metabisulfite, sodium metabisulfite, tocopherol, tocopherol hemisuccinate, TPGS or other tocopherol derivatives.

**20.** A composition according to any of the preceding claims comprising carvedilol and one or more active substances for human or veterinary use, a vitamin or other nutritional supplement, a disinfectant, a deodorant or another substance to be administered continuously in an aqueous environment.

**21.** A composition according to any of the preceding claims in the form of a matrix composition.

**22.** A composition according to claim 21, wherein any exposed matrix surfaces erodes at a substantially constant rate.

**23.** A composition according to any of claims 11-22, wherein in the aqueous medium in which the composition is to be used, the coating does not completely crumble or erode before the matrix has completely eroded.

**24.** A composition according to any of claims 11-23, wherein said first cellulose derivative is a cellulose ether which, when heated, is shapeable by molding or extrusion, including injection molding, blow molding and compression molding.

**25.** A composition according to claim 24 in which the cellulose ether comprises at least one ethylcellulose.

**26.** A composition according to any of claims 11-25, in which said plasticizer is selected from the group consisting of phosphate esters; phthalate esters; amides; mineral oils; fatty acids and esters thereof with polyethylene glycol, glycerin or sugars; fatty alcohols and ethers thereof with polyethylene glycol, glycerin or sugars; vegetable oils and hydrogenated vegetable oils; nitrobenzene, carbon disulfide, β-naphtyl salicylate, phthalyl glycolate, diocyl phthalate etc.

**27.** A composition according to any of the preceding claims, wherein the release of carvedilol from the composition is zero order and about 50% w/w carvedilol is released from the composition within 5-6 hours from start of release as measured by the dissolution test described herein.

**28.** A composition according to any of the preceding claims, which upon administration to a human fulfils at least one of the following criteria:

i) has an AUC (0-36 h) between 285 and 421 h*ng/ml,
ii) has a relative AUC compared to an immediate release composition between 70 and 97%,
iii) has a $C_{max}$ between 26.7 and 41.4 ng/ml,
iv) has a C (12 h) between 7.1 and 11.8 ng/ml,
v) has a C (24 h) between 2.9 and 5.0 ng/ml, and
vi) has a $t_{max}$ between 3 and 4 hours.

**29.** A method for preparing a composition according to any of claims 1-28, the method comprises injection moulding of a melted or semi-solid mixture of the individual components making up the composition into a suitable form, application of a coating by means of injection moulding and cooling the thus prepared coated composition to solidify the composition.

**30.** A method for preparing a composition according to any of claims 1-81, the method comprises extrusion of a melted or semi-solid mixture of the individual components making up the composition into a suitable form, application of a coating by means of co-extrusion and cooling the thus prepared coated composition to solidify the composition.

**31.** A method according to claim 29 or 30, wherein the method is a substantially single continuous process.

**32.** A method according to claim 29 or 30, wherein the cooling is performed under controlled conditions to a temperature of from about 0 °C to about 20 °C such as, e.g. from about 5 °C to about 18 °C, from about 10 °C to about 16 °C such as, e.g. about 10 °C, about 12 °C, about 14 °C, about 15 °C or about 16 °C.

**33.** A method according to claim 29 or 30 comprising a step of heating while the polymer and the active substance is in physical contact with each other.

**34.** A package comprising a selection of pharmaceutical compositions according to any of claims 1-28, the selection comprising compositions having different content of carvedilol.

**35.** A package according to claim 34, wherein the selection comprises compositions wherein the difference in carvedilol content is 2 fold or more such as 3, 4, 5, 6, 7, 8, 9, 10, 11, 12, 13, 14, 15 or 16 fold.

**36.** A package according to claim 34, wherein the selection comprises compositions having contents selected from 3.125, 6.25, 12.5, 25, 37.5 and 50 mg carvedilol.

**37.** A method for treating hypertension, angina pectoris and/or congestive heart failure, which method comprises administering carvedilol in a composition as claimed in any of claims 1-28.

Fig. 1

Carvedilol 50mg IR vs Egalet 9, 7½ and 6mm, Round
10 subjects

Fig. 2

Fig. 3

Fig. 4

**Fig. 5**

Fig. 6

Fig. 7

Fig. 8

Fig. 9

# Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0893440 A
- EP 0406315 B
- EP 0493513 B
- EP 0740310 B
- WO 9951208 A
- WO 9522962 A
- EP 0746310 B